(19) Europäisches Patentamt
European Patent Office
Office européen des brevets

(11) **EP 3 060 685 B1**

(12) **EUROPEAN PATENT SPECIFICATION**

(45) Date of publication and mention
of the grant of the patent:
**01.05.2019 Bulletin 2019/18**

(21) Application number: **14856518.7**

(22) Date of filing: **22.10.2014**

(51) Int Cl.:
*C12Q 1/6883* (2018.01)     *A61K 39/00* (2006.01)

(86) International application number:
**PCT/US2014/061759**

(87) International publication number:
**WO 2015/061441 (30.04.2015 Gazette 2015/17)**

(54) **METHOD OF PREDICTING THE RESPONSE OF AN ASTHMA PATIENT TO THERAPY**

METHODE ZUR VORHERSAGE DER REAKTION EINES ASTHMAPATIENTEN AUF THERAPIE

MÉTHODE DE PRÉDICTION DE LA RÉPONSE D'UN PATIENT ASTHMATIQUE AU TRAITEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.10.2013 US 201361894831 P**

(43) Date of publication of application:
**31.08.2016 Bulletin 2016/35**

(73) Proprietor: **F. Hoffmann-La Roche AG
4070 Basel (CH)**

(72) Inventors:
• **ABBAS, Alexander R.**
**South San Francisco**
**California 94080 (US)**
• **ARRON, Joseph R.**
**South San Francisco**
**California 94080 (US)**
• **CHOY, David F.**
**South San Francisco**
**California 94080 (US)**
• **JIA, Guiquan**
**South San Francisco**
**California 94080 (US)**
• **LEWIN-KOH, Nicholas J. I.**
**South San Francisco**
**California 94080 (US)**
• **MORSHEAD, Katrina B.**
**South San Francisco**
**California 94080 (US)**

(74) Representative: **Heiroth, Ulrike Hildegard
F. Hoffmann-La Roche AG
Patent Department
Grenzacherstrasse 124
4070 Basel (CH)**

(56) References cited:
**WO-A1-2013/148232       WO-A2-2005/093092
WO-A2-2005/116088       WO-A2-2008/086043
WO-A2-2008/091814       US-A1- 2007 077 585
US-A1- 2007 077 585      US-A1- 2012 156 194
US-A1- 2012 156 194**

• **Jonathan Corren ET AL: "Lebrikizumab
Treatment in Adults with Asthma", The New
England Journal of Medicine, 22 September 2011
(2011-09-22), pages 1088-1098, XP055189397,
United States DOI: 10.1056/NEJMoa1106469
Retrieved from the Internet:
URL:http://www.ncbi.nlm.nih.gov/pubmed/218
12663**
• **I Affymetrix: "A GeneChip Human Genome
Arrays", , 1 January 2013 (2013-01-01),
XP055373173, Retrieved from the Internet:
URL:http://www.affymetrix.com/support/tech
nical/datasheets/human_datasheet.pdf
[retrieved on 2017-05-16]**
• **A Randomized Trial ET AL: "Omalizumab in
Severe Allergic Asthma Inadequately Controlled
With Standard Therapy", , 3 May 2011
(2011-05-03), XP055373293, Retrieved from the
Internet:
URL:http://annals.org/pdfaccess.ashx?url=/
data/journals/aim/20231/0000605-201105030-
00002.pdf [retrieved on 2017-05-16]**

• PEI-SONG GAO ET AL: "Polymorphisms in the sialic acid-binding immunoglobulin-like lectin-8 (Siglec-8) gene are associated with susceptibility to asthma", EUROPEAN JOURNAL OF HUMAN GENETICS., vol. 18, no. 6, 20 January 2010 (2010-01-20), pages 713-719, XP055404271, CH ISSN: 1018-4813, DOI: 10.1038/ejhg.2009.239

**Description**

**FIELD**

Methods of diagnosing and treating disorders related to excess eosinophil numbers or activity, including but not limited to asthma, are disclosed. Also disclosed are methods of selecting or identifying patients for treatment with certain therapeutic agents that are TH2 pathway inhibitors.

**BACKGROUND**

Asthma is a complex disease with increasing worldwide incidence. Among other events, eosinophilic inflammation has been reported in the airways of asthma patients. The pathophysiology of the disease is characterized by variable airflow obstruction, airway inflammation, mucus hypersecretion, and subepithelial fibrosis. Clinically, patients may present with cough, wheezing, and shortness of breath. While many patients are adequately treated with currently available therapies, some patients with asthma have persistent disease despite the use of current therapies.

A plethora of drugs are on the market or in development for treating asthma. Targets for asthma therapy include cytokines such as IL-13, IL-17, IL-5, and IL-4 as well as targets associated with allergy such as IgE. Exemplary therapeutic molecules on the market and therapeutic candidates in development for the treatment of asthma include, but are not limited to, omalizumab (XOLAIR®) (targeting soluble IgE) (*see*, *e.g.*, Chang et al., J Allergy Clin Immunol. 117 (6): 1203-12 (2006); Winchester et al., N. Engl. J. Med. 355 (12): 1281-2 (2006); Brodlie et al., Arch Dis Child. 97 (7): 604-9 (2012); Bousquet et al., Chest 125 (4): 1378-86 (2004); Schulman, ES, Am J Respir Crit Care Med. 164 (8 Pt 2): S6-11 (2001); Chang et al., Adv Immunol. 93: 63-119 (2007)), lebrikizumab (targeting IL-13) (*see*, *e.g.*, Corren et al. (2011) N Engl J Med 365: 1088-98; Scheerens et al. (2012) Am J Respir Crit Care Med 185: A3960; Jia et al. (2012) J Allergy Clin Immunol 130: 647-654 e10; WO 2012/083132), mepolizumab (targeting IL-5) (*see*, *e.g.*, Haldar et al., N Engl J Med. 2009 Mar 5;360(10):973-84; Nair et al., N Engl J Med. 2009 Mar 5;360(10):985-93; Pavord et al., The Lancet 380 (9842): 651-659, doi:10.1016/S0140-6736(12)60988-X), and quilizumab (targeting membrane-bound IgE) (*see, e.g.*, US patent pub. no. 2013/0243750).

Although human asthma is commonly regarded as an allergic disorder characterized by type 2 cytokine expression and eosinophilic inflammation in the airways, it is clearly heterogeneous with respect to airway inflammation. Genomic approaches have identified heterogeneous gene expression patterns in asthmatic airways corresponding to the degree of type 2 cytokine expression and eosinophilic inflammation. These gene expression patterns have led to the identification of candidate biomarkers of eosinophilic airway inflammation that do not require bronchoscopy or sputum induction. Candidate biologic therapies targeting mediators of type 2 airway inflammation have progressed through clinical studies in patients with moderate-severe asthma in recent years. Serum periostin, fractional exhaled nitric oxide ($FE_{NO}$), and blood eosinophil counts are among those biomarkers that have emerged as potential predictive and pharmacodynamics biomarkers that may enrich for clinical benefit in clinical studies of biologic therapies targeting IL-13, IL-5, and IgE. Arron et al., 2013, DOI: 10.1513/AnnalsATS.201303-047AW. In US2007077585, US2012156194, WO2008086043 and Corren, J et al, The New England Journal Of Medicine, 22 September 2011, pages 1088-1098 studies for predicting the response of a patient suffering from asthma or a respiratory disorder to a therapy comprising a Th2 cell inhibitor are disclosed. Affymetrix: "A GeneChip Human Genome Arrays", 1 January 2013, XP055373173 discloses the Affymetrix Human Genome U1333Plus 2.0 array used herein, and comprising a probe to measure the expression of CSF1. WO2005116088 and Gao, P-S et al, European Journal of Human Genetics 18(6):713-719 (2010) disclose a link between SIGLEC8 and asthma.

Although such biomarkers as discussed above have demonstrated potential for identifying asthma patients that may be more likely to respond to particular therapeutic treatments, to date none have been validated and approved for such use by regulatory authorities. In addition, the previously identified biomarkers may have certain practical limitations and confounding factors associated with their use such as a need for a particular device to measure the biomarker, significant intrapatient or interpatient variability, or biomarker levels that may vary during development (e.g., pediatric levels compared to adult levels) or may be associated with additional disease states beyond asthma. Also, no clinically validated diagnostic markers, e.g., biomarkers, have been identified that enable clinicians or others to accurately define pathophysiological aspects of asthma, clinical activity, response to therapy, prognosis, or risk of developing the disease. Accordingly, as asthma patients seek treatment, there is at present considerable trial and error involved in the search for therapeutic agent(s) effective for a particular patient. Such trial and error often involves considerable risk and discomfort the the patient in order to find the most effective therapy.

Thus, there is a continuing need to identify new biomarkers that are effective for determining which asthma patients, and patients suffering with other atopic or eosinophilic disorders such as, for example but not limited to, atopic dermatitis, allergic rhinitis, nasal polyposis, eosiniophilic esophagitis, hypereosinophilic syndrome, will respond to which treatment and for incorporating such determinations into more effective treatment regimens for asthma and other eosinophilic-disorder patients. In addition, statistically and biologically significant and reproducible information regarding associations of such biomarkers with disease state could be utilized as an integral component in efforts to identify specific subsets of patients who would be expected to significantly benefit from treatment with a particular therapeutic agent, for example, where the therapeutic agent is or has been shown in clinical studies to be of therapeutic benefit in such specific patient subpopulation.

## SUMMARY

The invention is defined by the appended claims. Described are also therapeutic agents for inhibiting the TH2 pathway and methods of using the same. Further described are methods for diagnosing disease for use in treating the disease optionally with the TH2 pathway inhibitor.

The methods of treatment and diagnosis as disclosed herein can be applied to patients suffering from asthma, eosinophilic disorder, respiratory disorders, IL-13 mediated disorder and/or IgE-mediated disorder, or symptoms related to those disorders. Patients suffering from asthma-like symptoms, include patients that have not been diagnosed with asthma may be treated according to the methods disclosed herein.

According to one example, a patient treated according to the methods disclosed herein suffers from asthma, an eosinophilic disorder, a respiratory disorder, an IL-13 mediated disorder and/or an IgE-mediated disorder, or symptoms related to those disorders, and does not have cancer or a neoplasm. According to another example, the patient treated according to the methods disclosed herein is suffering from asthma, eosinophilic disorder, respiratory disorders, IL-13 mediated disorder and/or IgE-mediated disorder, or symptoms related to those disorders, and is 2 years old or older, 12 years old or older, 18 years old or older, 19 years old or older, between 2 and 18 years old, between 2 and 17 years old, between 12-17 years old, between 12 and 18 years old, between 2 and 75 years old, between 12 and 75 years old, or between 18 and 75 years old.

In some examples, an Eosinophilic Inflammation Diagnostic Assay (EIDA) is disclosed.

In some examples, methods of identifying an asthma patient or a respiratory disorder patient who is likely to be responsive to treatment with a TH2 pathway inhibitor are disclosed. In some examples, the method comprises determining whether the patient is Eosinophilic Inflammation Positive (EIP) using an Eosinophilic Inflammation Diagnostic Assay (EIDA), wherein the EIP status indicates that the patient is likely to be responsive to treatment with the TH2 pathway inhibitor.

In some examples, methods of identifying an asthma patient or a respiratory disorder patient who is likely to suffer from severe exacerbations are disclosed. In some examples, the method comprises determining whether the patient is Eosinophilic Inflammation Positive (EIP) using an Eosinophilic Inflammation Diagnostic Assay (EIDA), wherein the EIP status indicates that the patient is likely to suffer from an increase in severe exacerbations. In some examples, the methods comprise obtaining a biological sample from the patient, measuring the mRNA level of at least one, at least two, or at least three markers in the sample selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2, comparing the mRNA level detected in the sample to a reference level, and predicting that the patient is likely to suffer from severe exacerbations when the mRNA level measured in the sample is elevated compared to the reference level. In some examples, the methods comprise (a) measuring the mRNA level of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a biological sample from the patient; (b) comparing the mRNA level measured in (a) to a reference level; and (c) identifying the patient as more likely to suffer from severe exacerbations when the mRNA level measured in (a) is above the reference level. In some examples, the measuring the mRNA levels comprises amplification. In some examples, the measuring the mRNA levels comprises quantitative PCR. In some examples, the measuring the mRNA levels comprises amplifying the mRNA and detecting the amplified product, thereby measuring the level of the mRNA. In some examples, the reference level is the median level of the respective marker in a reference population. In some examples, the at least one, at least two, or at least three markers are selected from CSF1, MEIS2, CCL23, HSD3B7, SORD, CACNG6, MGAT3, SLC47A1, and ABTB2. In some examples, the at least one, at least two, or at least three markers are selected from MEIS2, LGALS12, IDOI, ALOX15, SIGLEC8, CCL23, PYROXD2, HSD3B7, CACNG6, and GPR44. In some examples, the at least one, at least two, or at least three markers are selected from CCL23, IDOI, HSD3B7, and CACNG6. In some examples, the at least one, at least two, or three markers are selected from CCL23, IDOI, and CACNG6. In some examples, the at least one, at least two, or three markers are selected from HSD3B7, SIGLEC8, and GPR44. In some examples, the at least one, at least two, at least three, or four markers are selected from SIGLEC8, CCL23, CACNG6, and GPR44.

In some examples, methods of identifying an asthma patient or a respiratory disorder patient who is less likely to be responsive to treatment with a TH2 pathway inhibitor are disclosed. In some examples, the method comprises determining whether the patient is Eosinophilic Inflammation Negative (EIN) using an Eosinophilic Inflammation Diagnostic Assay (EIDA), wherein the EIN status indicates that the patient is less likely to be responsive to treatment with the TH2 pathway inhibitor.

In some examples, methods of monitoring an asthma patient being treated with a TH2 Pathway inhibitor are disclosed. In some examples, the method comprises determining whether the patient is Eosinophilic Inflammation Positive (EIP) or Eosinophilic Inflammation Negative (EIN) using an Eosinophilic Inflammation Diagnostic Assay (EIDA). In some examples, the method further comprises determining a treatment regimen for the TH2 pathway inhibitor. In some such examples, the determination of EIP indicates continuing therapy with the TH2 pathway inhibitor and the determination of EIN indicates discontinuing therapy with the TH2 pathway inhibitor.

In any of the examples described herein, the EIDA may comprise the steps of a) determining the level of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a sample obtained from the patient; and b) comparing the levels of the at least one, at least two, or at least three markers determined in step a) to a reference level. In some examples, the EIDA further comprises c) stratifying said patient into the category of responder or non-responder based on the comparison obtained in step b). In some examples, a method further comprises selecting a therapy comprising a TH2 pathway inhibitor if the patient is a responder. In some of the above examples, the EIDA comprises determining the level of at least one, at least two, at least three, or four markers selected from SIGLEC8, CCL23, CACNG6, and GPR44.

In any of the examples described herein, the EIDA may comprise the steps of a) determining the level of at least one, at least two, or at least three markers selected from CSF1, MEIS2, CCL23, HSD3B7, SORD, CACNG6, MGAT3, SLC47A1, and ABTB2 in a sample obtained from the patient; and b) comparing the levels of the at least one, at least two, or at least three markers determined in step a) to a reference level. In some examples, the EIDA further comprises c) stratifying said patient into the category of responder or non-responder based on the comparison obtained in step b). In some examples, a method further comprises selecting a therapy comprising a TH2 pathway inhibitor if the patient is a responder.

In any of the examples described herein, the EIDA may comprise the steps of a) determining the level of at least one, at least two, or at least three markers selected from MEIS2, LGALS12, IDOI, ALOX15, SIGLEC8, CCL23, PYROXD2, HSD3B7, CACNG6, and GPR44 in a sample obtained from the patient; and b) comparing the levels of the at least one, at least two, or at least three markers determined in step a) to a reference level. In some examples, the EIDA further comprises c) stratifying said patient into the category of responder or non-responder based on the comparison obtained in step b). In some examples, a method further comprises selecting a therapy comprising a TH2 pathway inhibitor if the patient is a responder. In some of the above examples, the EIDA comprises determining the level of at least one, at least two, at least three, or four markers selected from SIGLEC8, CCL23, CACNG6, and GPR44.

In any of the examples described herein, the EIDA may comprise the steps of a) determining the level of at least one, at least two, or at least three markers selected from CCL23, IDOI, HSD3B7, and CACNG6 in a sample obtained from the patient; and b) comparing the levels of the at least one, at least two, or at least three markers determined in step a) to a reference level. In some examples, the EIDA further comprises c) stratifying said patient into the category of responder or non-responder based on the comparison obtained in step b). In some examples, a method further comprises selecting a therapy comprising a TH2 pathway inhibitor if the patient is a responder.

In any of the examples described herein, the EIDA may comprise the steps of a) determining the level of at least one, at least two, or three markers selected from CCL23, IDOI, and CACNG6 in a sample obtained from the patient; and b) comparing the levels of the at least one, at least two, or at least three markers determined in step a) to a reference level. In some examples, the EIDA further comprises c) stratifying said patient into the category of responder or non-responder based on the comparison obtained in step b). In some examples, a method further comprises selecting a therapy comprising a TH2 pathway inhibitor if the patient is a responder.

In any of the examples described herein, the EIDA may comprise the steps of a) determining the level of at least one, at least two, or three markers selected from HSD3B7, SIGLEC8, and GPR44 in a sample obtained from the patient; and b) comparing the levels of the at least one, at least two, or at least three markers determined in step a) to a reference level. In some examples, the EIDA further comprises c) stratifying said patient into the category of responder or non-responder based on the comparison obtained in step b). In some examples, a method further comprises selecting a therapy comprising a TH2 pathway inhibitor if the patient is a responder.

In some examples, methods of predicting the response of a patient suffering from asthma or a respiratory disorder to a therapy comprising a TH2 pathway inhibitor are disclosed. In some examples, the method comprises obtaining a biological sample from the patient and measuring the mRNA level of at least one, at least two, or at least three markers in the sample selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2. In some examples, the method comprises comparing the mRNA level detected in the sample to a reference level. In some examples, the method comprises predicting that the patient will respond to the therapy when the mRNA level measured in the sample is elevated compared to the reference level and predicting that the patient will not respond to the therapy when the mRNA level measured in the sample is reduced compared to the reference level. In some of the above examples, the mRNA level of at least one, at least two, at least three, or four markers selected from SIGLEC8, CCL23, CACNG6, and GPR44 is measured.

In some examples, methods of predicting responsiveness of an asthma patient or a respiratory disorder patient to a TH2 pathway inhibitor treatment are disclosed. In some examples, the method comprises measuring the mRNA level of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a biological sample from the patient. In some examples, an elevated mRNA level compared to a reference level identifies the patient as one who is likely to respond to the TH2 pathway inhibitor treatment. In some of the above examples, the mRNA level of at least one, at least two, at least three, or four markers selected from SIGLEC8, CCL23, CACNG6, and GPR44 is measured.

In some examples, methods of identifying a patient suffering from asthma or a respiratory disorder as likely to respond to a therapy comprising a TH2 pathway inhibitor are disclosed. In some examples, the method comprises measuring the mRNA level of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a biological sample from the patient. In some examples, that method further comprises comparing the measured mRNA level to a reference level. In some examples, the method comprises identifying the patient as more likely to respond to the therapy comprising the TH2 pathway inhibitor when the measured mRNA level is above the reference level. In one embodiment, a method of identifying a patient suffering from asthma as likely to respond to a therapy comprising lebrikizumab is provided, the method comprising: (a) measuring the mRNA level of marker CSF1 in a biological sample from the patient; (b) comparing the mRNA level measured in (a) to a reference level; and (c) identifying the patient as more likely to respond to the therapy comprising lebrikizumab when the mRNA level measured in (a) is above the reference level. In some of the above examples, the mRNA level of at least one, at least two, at least three, or four markers selected from SIGLEC8, CCL23, CACNG6, and GPR44 is measured.

In some examples, methods of treating patients having asthma or a respiratory disorder are disclosed. In some examples, the method comprises measuring the mRNA level of at least one, at least two, or at least three markers selected from CSF1, MEIS2, LGALS12, IDO1, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a biological sample from the patient. In some examples, the method comprises comparing the measured mRNA level to a reference level. In some examples, the method comprises identifying the patient as more likely to respond a therapy comprising a TH2 pathway inhibitor when the measured mRNA level is above the reference level. In some examples, the method comprises administering the therapy when the measured mRNA level is above the reference level, thereby treating the asthma or respiratory disorder. In some of the above examples, the mRNA level of at least one, at least two, at least three, or four markers selected from SIGLEC8, CCL23, CACNG6, and GPR44 is measured.

In some examples, a method of treating asthma or a respiratory disorder in a patient comprises administering to the patient a therapeutically effective amount of a TH2 pathway inhibitor, wherein a biological sample obtained from the patient has been determined to have elevated mRNA levels of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2. In one embodiment, Lebrikizumab for use in the treatment of asthma in a patient is provided, wherein a biological sample obtained from the patient has been determined to have elevated mRNA levels of marker CSF1, compared to the mRNA levels of the respective markers in a reference population. In some of the above examples, elevated mRNA levels of at least one, at least two, at least three, or four markers selected from SIGLEC8, CCL23, CACNG6, and GPR44 has been determined.

In some examples, a method of treating asthma or a respiratory disorder in a patient comprises administering to the patient a therapeutically effective amount of a TH2 pathway inhibitor, wherein the patient has been selected for treatment based on elevated mRNA levels in biological sample obtained from the patient of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2. In some of the above examples, elevated mRNA levels of at least one, at least two, at least three, or four markers selected from SIGLEC8, CCL23, CACNG6, and GPR44 has been determined.

In any of the methods described herein, the at least one, at least two, or at least three markers may be selected from CSF1, MEIS2, CCL23, HSD3B7, SORD, CACNG6, MGAT3, SLC47A1, and ABTB2. In any of the methods described herein, the at least one, at least two, or at least three markers may be selected from MEIS2, LGALS12, IDOI, ALOX15, SIGLEC8, CCL23, PYROXD2, HSD3B7, CACNG6, and GPR44. In any of the methods described herein, the at least one, at least two, or at least three markers may be selected from CCL23, IDO1, HSD3B7, and CACNG6. In any of the methods described herein, the at least one, at least two, or three markers may be selected from CCL23, IDOI, and CACNG6. In any of the methods described herein, the at least one, at least two, or three markers may be selected from HSD3B7, SIGLEC8, and GPR44. In any of the methods described herein, the at least one, at least two, or at least three markers or four markers may be selected from SIGLEC8, CCL23, CACNG6, and GPR44.

In any of the examples described herein, determining the levels of at least one marker may comprise amplification. In any of the examples described herein, determining the levels of at least one marker may comprise RT-PCR. In any of the examples described herein, determining the levels of at least one marker may comprise quantitative PCR. In any of the examples described herein, measuring the mRNA levels may comprise amplifying the mRNA and detecting the amplified product, thereby measuring the level of the mRNA. In one embodiment, a method of identifying a patient suffering from asthma as likely to respond to a therapy comprising lebrikizumab is provided, the method comprising: (a) measuring the mRNA level of marker CSF1 in a biological sample from the patient; (b) comparing the mRNA level measured in (a) to a reference level; and (c) identifying the patient as more likely to respond to the therapy comprising lebrikizumab when the mRNA level measured in (a) is above the reference level, wherein measuring the mRNA levels comprises amplifying the mRNA and detecting the amplified product, thereby measuring the level of the mRNA.

In any of the examples described herein, thet reference level may be the median, mean, or average level of the respective marker in a reference population. In any of the examples described herein, the reference level may be the median level of the respective marker in a reference population. In one embodiment, a method of identifying a patient suffering from asthma as likely to respond to a therapy comprising lebrikizumab is provided, the method comprising: (a) measuring the mRNA level of marker CSF1 in a biological sample from the patient; (b) comparing the mRNA level measured in (a) to a reference level; and (c) identifying the patient as more likely to respond to the therapy comprising lebrikizumab when the mRNA level measured in (a) is above the reference level, wherein measuring the mRNA levels optionally comprises amplifying the mRNA and detecting the amplified product, thereby measuring the level of the mRNA, wherein the reference level is the median level of the respective marker in a reference population. In any of the examples described herein, thet reference level may be the mean level of the respective marker in a reference population. In any of the examples described herein, thet reference level may be the average level of the respective marker in a reference population. Nonlimiting exemplary reference populations include patients with asthma, patients with moderate to severe asthma, healthy individuals, and a group including healthy individuals and patients with asthma. In some examples, a reference population comprises patients with moderate to severe asthma. Further nonlimiting exemplary reference populations include patients with an eosinophilic disorder (including the eosinophilic disorders described herein), such as patients with atopic dermatitis, patients with allergic rhinitis, patients with nasal polyposis, patients with eosinophilic esophagitis, patients with hyper-eosinophilic syndrome, etc.

In some examples, if the level of at least one marker is above the reference level, the patient is stratified into the category of responder. In some examples, if the level of at least one markeris above the reference level, the patient is Eosinophilic Inflammation Positive (EIP).

In some examples, the biological sample is selected from blood, serum, plasma, and peripheral blood mononucleocytes (PBMCs). In some examples, the biological sample is PBMCs. In some examples, the biological sample is obtained from an asthma patient. In certain examples, the patient according to the methods described above is suffering from moderate to severe asthma. In certain examples, the asthma or respiratory disorder is uncontrolled on a corticosteroid. In certain examples, the corticosteroid is an inhaled corticosteroid. In certain examples, the inhaled corticosteroid is Qvar®, Pulmicort®, Symbicort®, Aerobid®, Flovent®, Flonase®, Advair® or Azmacort®. In one example, the patient is also being treated with a second controller. In certain examples, the second controller is a long acting bronchial dilator (LABD). In certain examples, the LABD is a long-acting beta-2 agonist (LABA), leukotriene receptor antagonist (LTRA), long-acting muscarinic antagonist (LAMA), theophylline, or oral corticosteroids (OCS). In certain examples, the LABD is Symbicort®, Advair®, Brovana®, Foradil®, Perforomist™ or Serevent®.

In any of the examples described herein, the patient may be 0-17 years old, 2-17 years old, 2-6 years old, 6-11 years old, 8-17 years old, 12-17 years old, 2 years old or older, 6 years old or older, or 12 years old or older. In some examples, the patient is 18 years or older. In any of the examples described herein, the patient may be a human.

In any of the examples described herein, the TH2 pathway inhibitor may inhibit the target ITK, BTK , IL-9 (e.g., MEDI-528), IL-5 (e.g., Mepolizumab, CAS No. 196078-29-2; resilizumab), IL-13 (e.g., IMA-026, IMA-638 (also referred to as, anrukinzumab, INN No. 910649-32-0; QAX-576; IL4/IL13 trap), tralokinumab (also referred to as CAT-354, CAS No. 1044515-88-9); AER-001, ABT-308 (also referred to as humanized 13C5.5 antibody), IL-4 (e.g., AER-001, IL4/IL13 trap), OX40L, TSLP, IL-25, IL-33 and IgE (e.g., XOLAIR, QGE-031; MEDI-4212; quilizumab); and receptors such as: IL-9 receptor, IL-5 receptor (e.g., MEDI-563 (benralizumab, CAS No. 1044511-01-4), IL-4receptor alpha (e.g., AMG-317, AIR-645, dupilumab), IL-13receptoralpha1 (e.g., R-1671) and IL-13receptoralpha2, OX40, TSLP-R, IL-7Ralpha (a co-receptor for TSLP), IL17RB (receptor for IL-25), ST2 (receptor for IL-33), CCR3, CCR4, CRTH2 (e.g., AMG-853, AP768, AP-761, MLN6095, ACT129968), FcepsilonRI, FcepsilonRII/CD23 (receptors for IgE), Flap (e.g., GSK2190915), Syk kinase (R-343, PF3526299); CCR4 (AMG-761), TLR9 (QAX-935), or is a multi-cytokine inhibitor of CCR3, IL5, IL3, GM-CSF (e.g., TPI ASM8).

In any of the examples described herein, the TH2 pathway inhibitor may be an anti-IL13/IL4 pathway inhibitor or an anti IgE binding agent. In any of the examples described herein, the TH2 pathway inhibitor may be an anti-IL-13 antibody. In certain examples, the anti-IL-13 antibody is an antibody comprising a VH comprising a sequence selected from SEQ ID NOs: 9, 19, and 21, and VL comprising a sequence selected from SEQ ID NO: 10, 20, and 22, an anti-IL13 antibody comprising HVRH1, HVRH2, HVRH3, HVRL1, HVRL2, and HVRL3, the respective HVRs having the amino acid sequence of SEQ ID NO.: 11, SEQ ID NO.: 12, SEQ ID NO.: 13, SEQ ID NO.: 14, SEQ ID NO.: 15, and SEQ ID NO.: 16 or lebrikizumab.

In some examples, the patient is administered a flat dose of 37.5 mg, or 125 mg or 250 mg every four weeks. In some examples, the anti-IL-13 antibody is administered subcutaneously. In some examples, the anti-IL-13 antibody is administered using a prefilled syringe or autoinjector device.

In certain examples, the anti-IL-13 antibody is a bispecific antibody. In certain examples, the anti-IL-13 antibody is a bispecific antibody that also binds IL-4.

In any of the examples described herein, the TH2 pathway inhibitor may be an anti-IgE antibody. In certain examples, the anti-IgE antibody is (i) the XOLAIR® antibody, (ii) anti-M1' antibody comprising a variable heavy chain and a variable light chain, wherein the variable heavy chain is SEQ ID NO:1 and the variable light chain is SEQ ID NO:2 or (iii) an anti-M1' antibody comprising a variable heavy chain and a variable light chain, wherein the variable heavy chain further comprises an HVR-H1, HVR-H2 and HVR-H3, and the variable light chain further comprises and HVR-L1, HVR, L2 and HVR-L3 and: (a) the HVR-H1 has the sequence of SEQ ID NO: 3 [GFTFSDYGIA]; (b) the HVR-H2 has the sequence of SEQ ID NO: 4 [AFISDLAYTIYYADTVTG]; (c) the HVR-H3 has the sequence of SEQ ID NO: 5 [ARDNWDAMDY]; (d) the HVR-L1 has the sequence of SEQ ID NO: 6 [RSSQSLVHNNANTYLH]; (e) the HVR-L2 has the sequence of SEQ ID NO: 7 [KVSNRFS]; (f) the HVR-L3 has the sequence of SEQ ID NO: 8 [SQNTLVPWT]. In some examples, the anti-IgE antibody is an anti-M1' antibody.

In some examples, the anti-M1' antibody is administered subcutaneously at a flat dose of 150 mg once every 12 weeks, 300 mg once every 4 weeks or 450 mg once every 12 weeks. In some examples, the anti-M1' antibody is administered subcutaneously at a flat dose of 150 mg once every 12 weeks. In some examples, the anti-M1' antibody is administered subcutaneously at a flat dose of 450 mg once every 12 weeks. In some examples, an additional dose of the anti-M1' antibody is administered subcutaneously four weeks after administration of an initial dose.

In one example, a patient treated with a TH2 pathway inhibitor is also treated with one, two, three or more therapeutic agents. In one example, the patient is an asthma patient. According to one example, the patient is treated with the TH2 pathway inhibitor and one, two, three or more therapeutic agents, wherein at least one therapeutic agent, other than the TH2 inhibitor, is a corticosteroid, a leukotriene antagonist, a LABA, a corticosteroid/LABA combination composition, a theophylline, cromolyn sodium, nedocromil sodium, omalizumab, a LAMA, a MABA, a 5-Lipoxygenase Activating Protein (FLAP) inhibitor, or an enzyme PDE-4 inhibitor. According to one aspect of the description, a TH2 pathway inhibitor is administered to an asthma patient diagnosed as EIP status, wherein the diagnosis comprises the use of an EID assay (alone or in combination with other assays) to determine the EIP status. In one further example, the asthma patient is uncontrolled on a corticosteroid prior to the treatment. In another example, the asthma patient is also being treated with a second controller. In one example, the second controller is a corticosteroid, a LABA or a leukotriene antagonist. In a further example, the asthma patient is suffering from moderate to severe asthma. Thus, in one example, the patient to be treated with the TH2 pathway inhibitor is a moderate to severe asthma patient who is uncontrolled on a corticosteroid prior to treatment with the TH2 pathway inhibitor, and then is treated with the TH2 pathway inhibitor and one, two, three or more controllers. In one example, at least one of the controllers is a corticosteroid. In a further example, such patient is treated with a TH2 pathway inhibitor, a corticosteroid and another controller. In another example, the patient is suffering from mild asthma but is not being treated with a corticosteroid. It should be understood that the therapeutic agents may have different treatment cycles as compared with the TH2 inhibitor and, consequently can be administered at different times compared to the TH2 inhibitor as a part of the patient's treatment. Therefore, according to one example, a method of treatment may comprise the steps of administering to a patient a TH2 pathway inhibitory and optionally, administering at least one, two or three additional therapeutic agents. In one example, the TH2 pathway inhibitor is present in a composition with another therapeutic agent. In another example, the TH2 pathway inhibitor is not present in a composition with another therapeutic agent.

According to another example, a method for treating asthma is described comprising administering an anti-IL-13 antibody comprising a VH comprising a sequence selected from SEQ ID NOs: 9, 19, and 21, and VL comprising a sequence selected from SEQ ID NO: 10, 20, and 22; an anti-IL13 antibody comprising HVRH1, HVRH2, HVRH3, HVRL1, HVRL2, and HVRL3, wherein the respective HVRs have the amino acid sequence of SEQ ID NO.: 11, SEQ ID NO.: 12, SEQ ID NO.: 13, SEQ ID NO.: 14, SEQ ID NO.: 15, and SEQ ID NO.: 16; or lebrikizumab; as a flat dose. In one example an anti-IL13 antibody comprising a VH comprising a sequence selected from SEQ ID NOs: 9, 19, and 21 and VL comprising a sequence selected from SEQ ID NO: 10, 20, and 22 is administered as a flat dose (i.e., not weight dependent) of between 125-1000 mg, or a flat dose of 37.5 mg, or a flat dose of 125 mg, or a flat dose of 250 mg, or a flat dose of 500 mg, by subcutaneous injection or by intravenous injection, at a frequency of time selected from: every 2 weeks, every 3 weeks, and every 4 weeks. In one example an anti-IL13 antibody comprising HVRH1, HVRH2, HVRH3, HVRL1, HVRL2, and HVRL3, the respective HVRs having the amino acid sequence of SEQ ID NO.: 11, SEQ ID NO.: 12, SEQ ID NO.: 13, SEQ ID NO.: 14, SEQ ID NO.: 15, and SEQ ID NO.: 16 is administered as a flat dose (i.e., not weight dependent) of between 125-1000 mg, or a flat dose of 37.5 mg, or a flat dose of 125 mg, or a flat dose of 250 mg, or a flat dose of 500 mg, by subcutaneous injection or by intravenous injection, at a frequency of time selected from: every 2 weeks, every 3 weeks, and every 4 weeks. In one example, the anti-IL13 antibody is lebrikizumab, which is administered as a flat dose (i.e., not weight dependent) of between 125-1000 mg, or a flat dose of 37.5 mg, or a flat dose of 125 mg, or a flat dose of 250 mg, or a flat dose of 500 mg, by subcutaneous injection or by intravenous injection, at a frequency of time selected from: every 2 weeks, every 3 weeks, and every 4 weeks. In some examples, the patient is diagnosed with EIP using an EID Assay described herein.

According to another example, an antibody comprising VH comprising a sequence selected from SEQ ID NOs: 9, 19, and 21, and VL comprising a sequence selected from SEQ ID NO: 10, 20, and 22 is administered to treat asthma in a therapeutically effective amount sufficient to reduce the rate of exacerbations of the patient over time or improve $FEV_1$. In yet another example, a method for treating asthma is described comprising administering an anti-IL-13 antibody comprising a VH comprising a sequence selected from SEQ ID NOs: 9, 19, and 21, and VL comprising a sequence selected from SEQ ID NO: 10, 20, and 22 or an anti-IL13 antibody comprising HVRH1, HVRH2, HVRH3, HVRL1, HVRL2, and HVRL3, the respective HVRs having the amino acid sequence of SEQ ID NO.: 11, SEQ ID NO.: 12, SEQ ID NO.: 13, SEQ ID NO.: 14, SEQ ID NO.: 15, and SEQ ID NO.: 16 as a flat dose (i.e., not weight dependent) of 37.5 mg, or a flat dose of 125 mg, or a flat dose of 250 mg. In certain examples, the dose is administered by subcutaneous injection once every 4 weeks for a period of time. In certain examples, the period of time is 6 months, one year, two years, five years, ten years, 15 years, 20 years, or the lifetime of the patient. In certain examples, the asthma is severe asthma and the patient is inadequately controlled or uncontrolled on inhaled corticosteroids plus a second controller medication. In some examples, the patient is diagnosed with EIP status using an EID Assay to determine EIP status and the patient is selected for treatment with an anti-IL13 antibody as described above. In another example, the method comprises treating an asthma patient with an anti-IL13 antibody as described above where the patient was previously diagnosed with EIP status using an EID Assay described herein to determine EIP status. In one example, the asthma patient is age 18 or older. In one example, the asthma patient is age 12 to 17 and the anti-IL13 is administered in as a flat dose of 250 mg or a flat dose of 125 mg. In one example, the asthma patient is age 6 to 11 and the anti-IL13 antibody is administered in as a flat dose of 125 mg or a flat dose of 62.5 mg.

The present application discloses a therapeutic agent that is a TH2 pathway inhibitor for use in treating asthma or a respiratory disorder in a patient, wherein the patient is EIP. In some examples, the target for inhibition in the TH2 pathway is selected from: IL-9, IL-5, IL-13, IL-4, OX40L, TSLP, IL-25, IL-33 and IgE; and receptors such as: IL-9 receptor, IL-5 receptor, IL-4receptor alpha, IL-13receptoralpha1 and IL-13receptoralpha2, OX40, TSLP-R, IL-7Ralpha (a co-receptor for TSLP), IL17RB (receptor for IL-25), ST2 (receptor for IL-33), CCR3, CCR4, CRTH2, FcepsilonRI and FcepsilonRII/CD23 (receptors for IgE). In one example, the patient to be treated is suffering from mild to severe asthma, optionally moderate to severe asthma, and whose asthma is uncontrolled on a corticosteroid. In some examples, the patient to be treated in addition to having elevated levels of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2, or at least one, at least two, or at least three markers selected from CSF1, MEIS2, CCL23, HSD3B7, SORD, CACNG6, MGAT3, SLC47A1, and ABTB2, or at least one, at least two, or at least three markers selected from MEIS2, LGALS12, IDOI, ALOX15, SIGLEC8, CCL23, PYROXD2, HSD3B7, CACNG6, and GPR44, or at least one, at least two, or at least three markers selected from CCL23, IDOI, HSD3B7, and CACNG6, or at least one, at least two, or all three markers selected from CCL23, IDOI, and CACNG6, or at least one, at least two, or all three of the markers selected from HSD3B7, SIGLEC8, and GPR44, or at least one, at least two, at least three, or all four markers selected from SIGLEC8, CCL23, CACNG6, and GPR44, has a $FE_{NO}$ level greater than 21 ppb, or greater than 35 ppb.

In another aspect, uses of a kit for measuring the levels of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a sample obtained from an asthma patient for stratifying/classifying asthma patients into likely responders and non-responders for therapeutic treatment with a TH2 pathway inhibitor. In certain examples, the use comprises the steps of: (a) determining the levels of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a sample obtained from an asthma patient; (b) comparing the levels of the one or more markers determined in step (a) to a reference level; and (c) stratifying said patient into the category of responder or non-responder based on the comparison obtained in step (b).

In another aspect, uses of a kit for measuring the levels of at least one, at least two, or at least three markers selected from CSF1, MEIS2, CCL23, HSD3B7, SORD, CACNG6, MGAT3, SLC47A1, and ABTB2 in a sample obtained from an asthma patient for stratifying/classifying asthma patients into likely responders and non-responders for therapeutic treatment with a TH2 pathway inhibitor. In certain examples, the use comprises the steps of: (a) determining the levels of at least one, at least two, or at least three markers selected from CSF1, MEIS2, CCL23, HSD3B7, SORD, CACNG6, MGAT3, SLC47A1, and ABTB2 in a sample obtained from an asthma patient; (b) comparing the levels of the one or more markers determined in step (a) to a reference level; and (c) stratifying said patient into the category of responder or non-responder based on the comparison obtained in step (b).

In another aspect, uses of a kit for measuring the levels of at least one, at least two, or at least three markers selected from MEIS2, LGALS12, IDOI, ALOX15, SIGLEC8, CCL23, PYROXD2, HSD3B7, CACNG6, and GPR44 in a sample obtained from an asthma patient for stratifying/classifying asthma patients into likely responders and non-responders for therapeutic treatment with a TH2 pathway inhibitor. In certain examples, the use comprises the steps of: (a) determining the levels of at least one, at least two, or at least three markers selected from MEIS2, LGALS12, IDOI, ALOX15, SIGLEC8, CCL23, PYROXD2, HSD3B7, CACNG6, and GPR44 in a sample obtained from an asthma patient; (b) comparing the levels of the one or more markers determined in step (a) to a reference level; and (c) stratifying said patient into the category of responder or non-responder based on the comparison obtained in step (b).

In another aspect, uses of a kit for measuring the levels of at least one, at least two, or at least three markers selected from CCL23, IDOI, HSD3B7, and CACNG6, or uses of a kit fro measuring the levels of at least one, at least two, at least three, or all four markers selected from SIGLEC8, CCL23, CACNG6, and GPR44 in a sample obtained from an asthma patient for stratifying/classifying asthma patients into likely responders and non-responders for therapeutic treatment with a TH2 pathway inhibitor. In certain examples, the use comprises the steps of: (a) determining the levels of at least one, at least two, or at least three markers selected from CCL23, IDOI, HSD3B7, and CACNG6, or determining the levels of at least one, at least two, at least three, or all four markers selected from SIGLEC8, CCL23, CACNG6, and GPR44 in a sample obtained from an asthma patient; (b) comparing the levels of the one or more markers determined in step (a) to a reference level; and (c) stratifying said patient into the category of responder or non-responder based on the comparison obtained in step (b).

In another aspect, uses of a kit for measuring the levels of at least one, at least two, or all three markers selected from CCL23, IDOI, and CACNG6, or uses of a kit for measuring the levels of at least one, at least two, at least three, or all four markers selected from SIGLEC8, CCL23, CACNG6, and GPR44 in a sample obtained from an asthma patient for stratifying/classifying asthma patients into likely responders and non-responders for therapeutic treatment with a TH2 pathway inhibitor. In certain examples, the use comprises the steps of: (a) determining the levels of at least one, at least two, or all three markers selected from CCL23, IDOI, and CACNG6, or determining the levels of at least one, at least two, at least three, or all four markers selected from SIGLEC8, CCL23, CACNG6, and GPR44 in a sample obtained from an asthma patient; (b) comparing the levels of the one or more markers determined in step (a) to a reference level; and (c) stratifying said patient into the category of responder or non-responder based on the comparison obtained in step (b).

In another aspect, uses of a kit for measuring the levels of at least one, at least two, or all three markers selected from HSD3B7, SIGLEC8, and GPR44 in a sample obtained from an asthma patient for stratifying/classifying asthma patients into likely responders and non-responders for therapeutic treatment with a TH2 pathway inhibitor. In certain examples, the use comprises the steps of: (a) determining the levels of at least one, at least two, or all three markers selected from HSD3B7, SIGLEC8, and GPR44 in a sample obtained from an asthma patient; (b) comparing the levels of the one or more markers determined in step (a) to a reference level; and (c) stratifying said patient into the category of responder or non-responder based on the comparison obtained in step (b).

In any of the examples described herein, determining the levels of at least one marker may comprise amplification. In any of the examples described herein, determining the levels of at least one marker may comprise RT-PCR. In any of the examples described herein, determining the levels of at least one marker may comprise quantitative PCR. In any of the examples described herein, measuring the mRNA levels may comprise amplifying the mRNA and detecting the amplified product, thereby measuring the level of the mRNA.

In some examples, a reference level of a marker is the median level of the marker in a reference population. In any of the examples described herein, the reference level may be the mean level of the respective marker in a reference population. In some examples, a reference level of a marker is the average level of the marker in a reference population. Nonlimiting exemplary reference populations include patients with asthma, patients with moderate to severe asthma, healthy individuals, and a group including healthy individuals and patients with asthma. In some examples, a reference population comprises patients with moderate to severe asthma. Further nonlimiting exemplary reference populations include patients with an eosinophilic disorder (including the eosinophilic disorders described herein), such as patients with atopic dermatitis, patients with allergic rhinitis, patients with nasal polyposis, patients with eosinophilic esophagitis, patients with hyper-eosinophilic syndrome, etc.

In some examples, if the level of at least one marker is above the reference level, the patient is stratified into the category of responder. In some examples, if the level of at least one marker is above the reference level, the patient is Eosinophilic Inflammation Positive (EIP).

In some examples, the biological sample is selected from blood, serum, plasma, and peripheral blood mononucleocytes (PBMCs). In some examples, the biological sample is PBMCs. In some examples, the biological sample is obtained from an asthma patient. In certain examples, the patient according to the uses described in the paragraph above is suffering from moderate to severe asthma. In certain examples, the asthma or respiratory disorder is uncontrolled on a corticosteroid. In certain examples, the corticosteroid is an inhaled corticosteroid. In certain examples, the inhaled corticosteroid is Qvar®, Pulmicort®, Symbicort®, Aerobid®, Flovent®, Flonase®, Advair® or Azmacort®. In one example, the patient is also being treated with a second controller. In certain examples, the second controller is a long acting bronchial dilator (LABD). In certain examples, the LABD is a long-acting beta-2 agonist (LABA), leukotriene receptor antagonist (LTRA), long-acting muscarinic antagonist (LAMA), theophylline, or oral corticosteroids (OCS). In certain examples, the LABD is Symbicort®, Advair®, Brovana®, Foradil®, Perforomist™ or Serevent®.

In certain examples, the TH2 pathway inhibitor according to the uses above inhibits the target ITK, BTK , IL-9 (e.g., MEDI-528), IL-5 (e.g., Mepolizumab, CAS No. 196078-29-2; resilizumab), IL-13 (e.g., IMA-026, IMA-638 (also referred to as, anrukinzumab, INN No. 910649-32-0; QAX-576; IL4/IL13 trap), tralokinumab (also referred to as CAT-354, CAS No. 1044515-88-9); AER-001, ABT-308 (also referred to as humanized 13C5.5 antibody), IL-4 (e.g., AER-001, IL4/IL13 trap), OX40L, TSLP, IL-25, IL-33 and IgE (e.g., XOLAIR, QGE-031; MEDI-4212; quilizumab); and receptors such as: IL-9 receptor, IL-5 receptor (e.g., MEDI-563 (benralizumab, CAS No. 1044511-01-4), IL-4receptor alpha (e.g., AMG-317, AIR-645, dupilumab), IL-13receptoralpha1 (e.g., R-1671) and IL-13receptoralpha2, OX40, TSLP-R, IL-7Ralpha (a co-receptor for TSLP), IL17RB (receptor for IL-25), ST2 (receptor for IL-33), CCR3, CCR4, CRTH2 (e.g., AMG-853, AP768, AP-761, MLN6095, ACT129968), FcepsilonRI, FcepsilonRII/CD23 (receptors for IgE), Flap (e.g., GSK2190915), Syk kinase (R-343, PF3526299); CCR4 (AMG-761), TLR9 (QAX-935), or is a multi-cytokine inhibitor of CCR3, IL5, IL3, GM-CSF (e.g., TPI ASM8).

In any of the examples described herein, the TH2 pathway inhibitor may be an anti-IL13/IL4 pathway inhibitor or an anti IgE binding agent. In any of the examples described herein, the TH2 pathway inhibitor may be an anti-IL-13 antibody. In certain examples, the anti-IL-13 antibody is an antibody comprising a VH comprising a sequence selected from SEQ ID NOs: 9, 19, and 21, and VL comprising a sequence selected from SEQ ID NO: 10, 20, and 22, an anti-IL13 antibody comprising HVRH1, HVRH2, HVRH3, HVRL1, HVRL2, and HVRL3, the respective HVRs having the amino acid sequence of SEQ ID NO.: 11, SEQ ID NO.: 12, SEQ ID NO.: 13, SEQ ID NO.: 14, SEQ ID NO.: 15, and SEQ ID NO.: 16 or lebrikizumab.

In certain examples, the anti-IL-13 antibody is a bispecific antibody. In certain examples, the anti-IL-13 antibody is a bispecific antibody that also binds IL-4.

In any of the examples described herein, the TH2 pathway inhibitor may be an anti-IgE antibody. In certain examples, the anti-IgE antibody is (i) the XOLAIR® antibody, (ii) anti-M1' antibody comprising a variable heavy chain and a variable light chain, wherein the variable heavy chain is SEQ ID NO:1 and the variable light chain is SEQ ID NO:2 or (iii) an anti-M1' antibody comprising a variable heavy chain and a variable light chain, wherein the variable heavy chain further comprises an HVR-H1, HVR-H2 and HVR-H3, and the variable light chain further comprises and HVR-L1, HVR, L2 and HVR-L3 and: (a) the HVR-H1 has the sequence of SEQ ID NO: 3 [GFTFSDYGIA]; (b) the HVR-H2 has the sequence of SEQ ID NO: 4 [AFISDLAYTIYYADTVTG]; (c) the HVR-H3 has the sequence of SEQ ID NO: 5 [ARDNWDAMDY]; (d) the HVR-L1 has the sequence of SEQ ID NO: 6 [RSSQSLVHNNANTYLH]; (e) the HVR-L2 has the sequence of SEQ ID NO: 7 [KVSNRFS]; (f) the HVR-L3 has the sequence of SEQ ID NO: 8 [SQNTLVPWT]. In some examples, the anti-IgE antibody is an anti-M1' antibody.

In any of the examples described herein, the patient may be 0-17 years old, 2-17 years old, 2-6 years old, 6-11 years old, 8-17 years old, 12-17 years old, 2 years old or older, 6 years old or older, or 12 years old or older. In some examples, the patient is 18 years or older. In any of the examples described herein, the patient may be a human.

In yet another aspect, kits for measuring the levels of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a biological sample obtained from an asthma patient or a patient suffering from a respiratory disorder are disclosed. In some examples, the kit comprises instructions for (i) measuring the mRNA levels of the at least one, at least two, or at least three markers, (ii) comparing the levels of the at least one, at least two, or at least three markers to a reference level, and (iii) stratifying said patient into the category of responder or non-responder based on the comparison. In some examples, the kit comprises at least one, at least two, or at least three first nucleic acid molecules that hybridize to at least one, at least two, or at least three second nucleic acid molecules, wherein the at least one, at least two, or at least three second nucleic acid molecules encode at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 or a portion thereof. In certain examples, the kit comprises a package insert containing information describing the uses disclosed above.

In yet another aspect, kits for measuring the levels of at least one, at least two, or at least three markers selected from CSF1, MEIS2, CCL23, HSD3B7, SORD, CACNG6, MGAT3, SLC47A1, and ABTB2 in a biological sample obtained from an asthma patient or a patient suffering from a respiratory disorder are disclosed. In some examples, the kit comprises instructions for (i) measuring the mRNA levels of the at least one, at least two, or at least three markers, (ii) comparing the levels of the at least one, at least two, or at least three markers to a reference level, and (iii) stratifying said patient into the category of responder or non-responder based on the comparison. In some examples, the kit comprises at least one, at least two, or at least three first nucleic acid molecules that hybridize to at least one, at least two, or at least three second nucleic acid molecules, wherein the at least one, at least two, or at least three second nucleic acid molecules encode at least one, at least two, or at least three markers selected from CSF1, MEIS2, CCL23, HSD3B7, SORD, CACNG6, MGAT3, SLC47A1, and ABTB2 or a portion thereof. In certain examples, the kit comprises a package insert containing information describing the uses disclosed above.

In yet another aspect, kits for measuring the levels of at least one, at least two, or at least three markers selected from MEIS2, LGALS12, IDOI, ALOX15, SIGLEC8, CCL23, PYROXD2, HSD3B7, CACNG6, and GPR44 in a biological sample obtained from an asthma patient or a patient suffering from a respiratory disorder are disclosed. In some examples, the kit comprises instructions for (i) measuring the mRNA levels of the at least one, at least two, or at least three markers, (ii) comparing the levels of the at least one, at least two, or at least three markers to a reference level, and (iii) stratifying said patient into the category of responder or non-responder based on the comparison. In some examples, the kit comprises at least one, at least two, or at least three first nucleic acid molecules that hybridize to at least one, at least two, or at least three second nucleic acid molecules, wherein the at least one, at least two, or at least three second nucleic acid molecules encode at least one, at least two, or at least three markers selected from MEIS2, LGALS12, IDOI, ALOX15, SIGLEC8, CCL23, PYROXD2, HSD3B7, CACNG6, and GPR44 or a portion thereof. In certain examples, the kit comprises a package insert containing information describing the uses disclosed above.

In yet another aspect, kits for measuring the levels of at least one, at least two, or at least three markers selected from CCL23, IDOI, HSD3B7, and CACNG6, or kits for measuring the leels of at least one, at least two, at least three, or all four markers selected from SIGLEC8, CCL23, CACNG6, and GPR44 in a biological sample obtained from an asthma patient or a patient suffering from a respiratory disorder are disclosed. In some examples, the kit comprises instructions for (i) measuring the mRNA levels of the at least one, at least two, or at least three markers, (ii) comparing the levels of the at least one, at least two, or at least three markers to a reference level, and (iii) stratifying said patient into the category of responder or non-responder based on the comparison. In some examples, the kit comprises at least one, at least two, or at least three first nucleic acid molecules that hybridize to at least one, at least two, or at least three second nucleic acid molecules, wherein the at least one, at least two, or at least three second nucleic acid molecules encode at least one, at least two, or at least three markers selected from CCL23, IDOI, HSD3B7, and CACNG6 or a portion thereof, or at least one, at least two, or at least three selected from SIGLEC8, CCL23, CACNG6, and GPR44 or a portion thereof. In certain examples, the kit comprises a package insert containing information describing the uses disclosed above.

In yet another aspect, kits for measuring the levels of at least one, at least two, or all three markers selected from CCL23, IDOI, and CACNG6 in a biological sample obtained from an asthma patient or a patient suffering from a respiratory disorder are disclosed. In some examples, the kit comprises instructions for (i) measuring the mRNA levels of the at least one, at least two, or at least three markers, (ii) comparing the levels of the at least one, at least two, or at least three markers to a reference level, and (iii) stratifying said patient into the category of responder or non-responder based on the comparison. In some examples, the kit comprises at least one, at least two, or at least three first nucleic acid molecules that hybridize to at least one, at least two, or at least three second nucleic acid molecules, wherein the at least one, at least two, or at least three second nucleic acid molecules encode at least one, at least two, or all three markers selected from CCL23, IDOI, and CACNG6 or a portion thereof. In certain examples, the kit comprises a package insert containing information describing the uses disclosed above.

In yet another aspect, kits for measuring the levels of at least one, at least two, or all three markers selected from HSD3B7, SIGLEC8, and GPR44 in a biological sample obtained from an asthma patient or a patient suffering from a respiratory disorder are disclosed. In some examples, the kit comprises instructions for (i) measuring the mRNA levels of the at least one, at least two, or at least three markers, (ii) comparing the levels of the at least one, at least two, or at least three markers to a reference level, and (iii) stratifying said patient into the category of responder or non-responder based on the comparison. In some examples, the kit comprises at least one, at least two, or at least three first nucleic acid molecules that hybridize to at least one, at least two, or at least three second nucleic acid molecules, wherein the at least one, at least two, or at least three second nucleic acid molecules encode at least one, at least two, or all three markers selected from HSD3B7, SIGLEC8, and GPR44 or a portion thereof. In certain examples, the kit comprises a package insert containing information describing the uses disclosed above.

In still yet another aspect, kits for diagnosing an asthma subtype in a patient are disclosed, the kits comprising: (1) determining the levels of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a serum sample obtained from the patient; and (2) instructions for measuring the levels of the at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in the serum sample, wherein the elevated expression levels of any one, combination or all of said markers is indicative of the asthma subtype.

In still yet another aspect, kits for diagnosing an asthma subtype in a patient are disclosed, the kits comprising: (1) determining the levels of at least one, at least two, or at least three markers selected from CSF1, MEIS2, CCL23, HSD3B7, SORD, CACNG6, MGAT3, SLC47A1, and ABTB2 in a serum sample obtained from the patient; and (2) instructions for measuring the levels of the at least one, at least two, or at least three markers selected from CSF1, MEIS2, CCL23, HSD3B7, SORD, CACNG6, MGAT3, SLC47A1, and ABTB2 in the serum sample, wherein the elevated expression levels of any one, combination or all of said markers is indicative of the asthma subtype.

In still yet another aspect, kits for diagnosing an asthma subtype in a patient are disclosed, the kits comprising: (1) determining the levels of at least one, at least two, or at least three markers selected from MEIS2, LGALS12, IDOI, ALOX15, SIGLEC8, CCL23, PYROXD2, HSD3B7, CACNG6, and GPR44 in a serum sample obtained from the patient; and (2) instructions for measuring the levels of the at least one, at least two, or at least three markers selected from MEIS2, LGALS12, IDOI, ALOX15, SIGLEC8, CCL23, PYROXD2, HSD3B7, CACNG6, and GPR44 in the serum sample, wherein the elevated expression levels of any one, combination or all of said markers is indicative of the asthma subtype.

In still yet another aspect, kits for diagnosing an asthma subtype in a patient are disclosed, the kits comprising: (1) determining the levels of at least one, at least two, or at least three markers selected from CCL23, IDOI, HSD3B7, and CACNG6 in a serum sample obtained from the patient; and (2) instructions for measuring the levels of the at least one, at least two, or at least three markers selected from CCL23, IDOI, HSD3B7, and CACNG6 in the serum sample, wherein the elevated expression levels of any one, combination or all of said markers is indicative of the asthma subtype.

In still yet another aspect, kits for diagnosing an asthma subtype in a patient are disclosed, the kits comprising: (1) determining the levels of at least one, at least two, or all three markers selected from CCL23, IDOI, and CACNG6 in a serum sample obtained from the patient; and (2) instructions for measuring the levels of the at least one, at least two, or all three markers selected from CCL23, IDOI, and CACNG6 in the serum sample, wherein the elevated expression levels of any one, combination or all of said markers is indicative of the asthma subtype.

In still yet another aspect, kits for diagnosing an asthma subtype in a patient are disclosed, the kits comprising: (1) determining the levels of at least one, at least two, or all three markers selected from HSD3B7, SIGLEC8, and GPR44 in a serum sample obtained from the patient; and (2) instructions for measuring the levels of the at least one, at least two, or all three markers selected from HSD3B7, SIGLEC8, and GPR44 in the serum sample, wherein the elevated expression levels of any one, combination or all of said markers is indicative of the asthma subtype.

In still yet another aspect, kits for diagnosing an asthma subtype in a patient are disclosed, the kits comprising: (1) determining the levels of at least one, at least two, at least three, or all four markers selected from SIGLEC8, CCL23, CACNG6, and GPR44 in a serum sample obtained from the patient; and (2) instructions for measuring the levels of the at least one, at least two, at least three, or all four markers selected from SIGLEC8, CCL23, CACNG6, and GPR44 in the serum sample, wherein the elevated expression levels of any one, combination or all of said markers is indicative of the asthma subtype.

In some examples, the kit further comprises a package insert for determining whether an asthma patient or respiratory disorder patient is EIP or EIN. In some examples, the kit further comprises a package insert for determining whether an asthma patient is likely to respond to a TH2 pathway inhibitor. In some examples, the kit further comprises a package insert containing information describing any of the uses disclosed above. In some examples, the kit further comprises an empty container to hold a biological sample. In some examples, the kit comprises reagents for determining the levels of the one or more markers. In some examples, the reagents for determining the levels of the one or more markers include, but are not limited to, one or more first nucleic acid molecules that hybridize to one or more second nucleic acid molecules that encode one or more markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2.

In still another aspect, methods of treating of a patient suffering from asthma or a respiratory disease comprising administering a TH2 pathway inhibitor to the patient diagnosed as EIP are disclosed. In certain examples, the methods comprise the step of diagnosing the patient as EIP using an EID Assay. In certain examples, the methods further comprise the step of retreating the patient with the TH2 pathway inhibitor if the patient is determined to be EIP. In certain examples, serum, whole blood, PBMCs, or plasma from the patient is used to determine whether the patient is EIP.

## BRIEF DESCRIPTION OF THE DRAWINGS

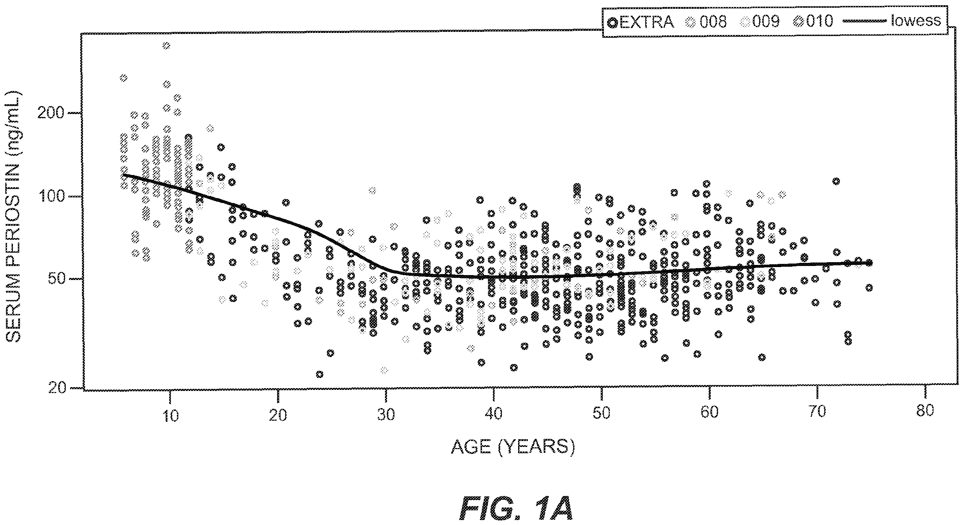

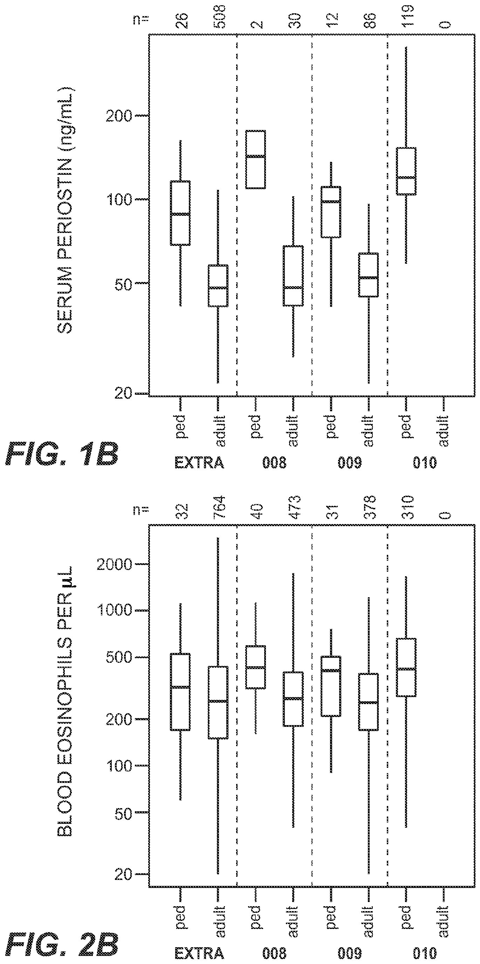

**Figure 1.** Serum periostin is elevated in asthma patients under the age of 18 years but is not related to age in adult asthma patients. (A) Serum periostin level vs. age in 783 asthma patients from omalizumab studies 008, 009, 010, and EXTRA, 159 of whom were under the age of 18 years. (B) Range and distribution of serum periostin levels by study. Thick horizontal black line denotes median, box and whiskers denote interquartile and total ranges, respectively.

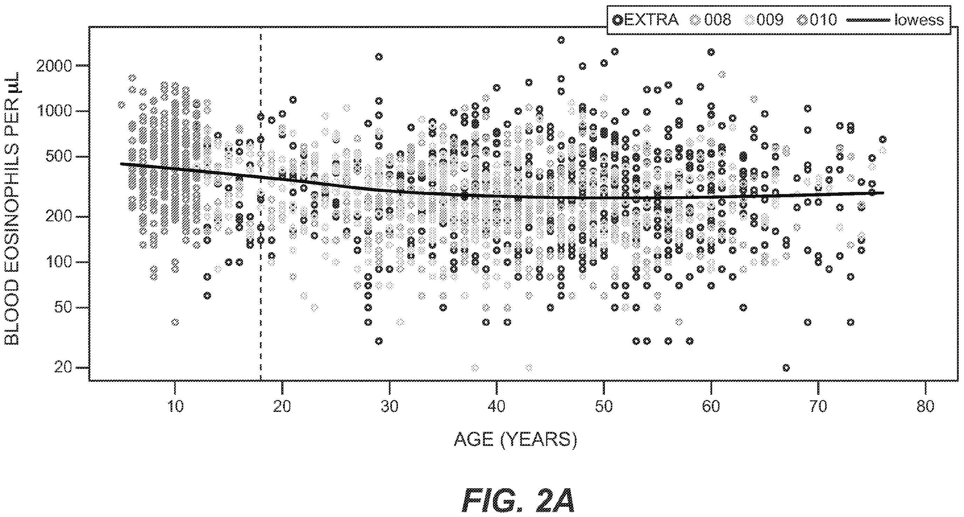

**Figure 2.** Relationship between blood eosinophils and age is continuous across pediatric and adult asthma patients. (A) Blood eosinophil counts vs. age in 2028 asthma patients from omalizumab studies 008, 009, 010, and EXTRA, 413 of whom were under the age of 18 years. (B) Range and distribution of blood eosinophil counts by study. Thick horizontal black line denotes median, box and whiskers denote interquartile and total ranges, respectively.

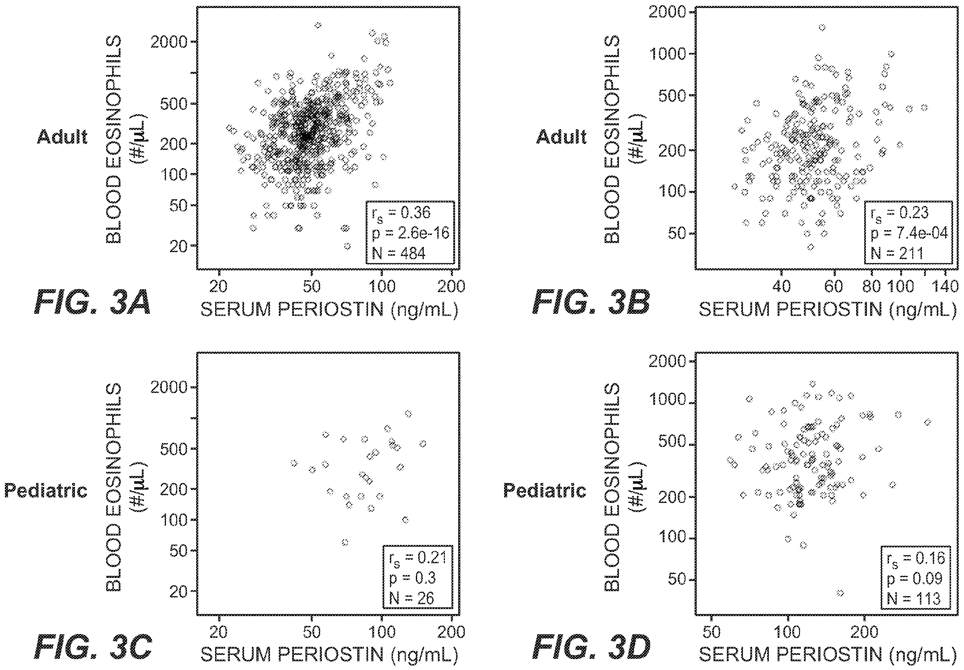

**Figure 3.** Serum periostin levels and blood eosinophil counts are positively correlated in adult but not pediatric asthma patients. (A) adults in the EXTRA study; (B) adults in the MILLY study; (C) patients age 12-17 in EXTRA; (D) patients age 6-12 in study 010. rS, Spearman's rank-order correlation coefficient.

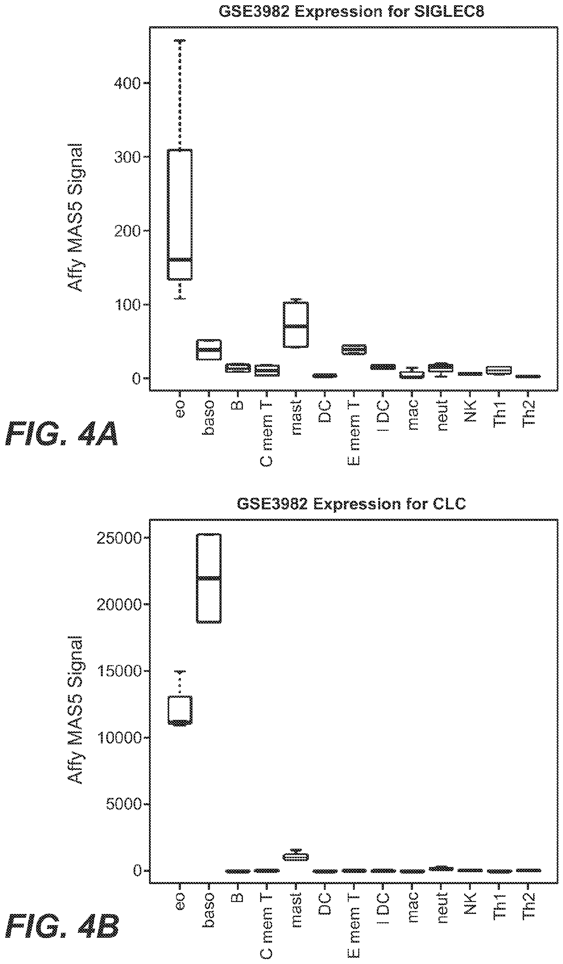

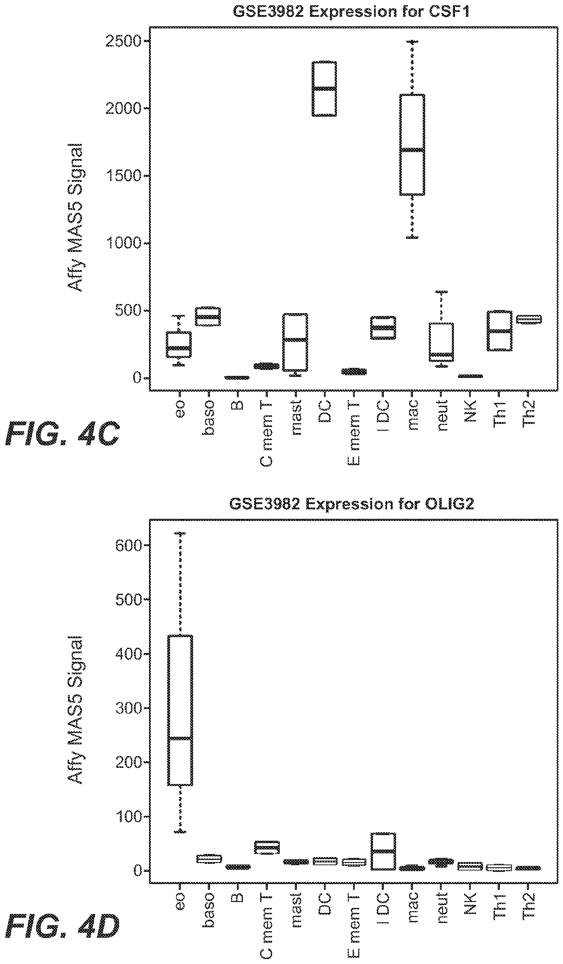

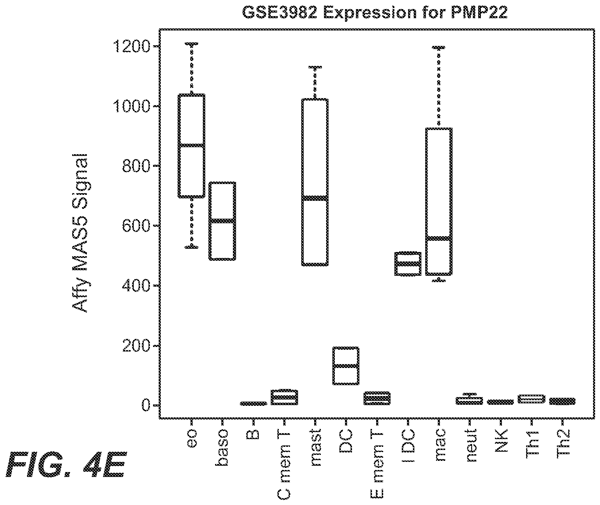

**Figure 4.** Different cellular distribution of eosinophil-related genes in peripheral blood. Selected genes correlated with blood eosinophil counts in EXTRA are variably expressed in isolated peripheral blood leukocyte populations in GSE3982 (Liu et al. (2006) J Allergy Clin Immunol 118: 496-503). (A) SIGLEC8 expression is mainly restricted to eosinophils. (B) CLC expression is restricted to eosinophils and basophils. (C) CSF1 expression is distributed across multiple peripheral blood leukocyte types including eosinophils, basophils, mast cells, neutrophils, dendritic cells, macrophages, and T lymphocytes. (D) OLIG2, described as a transcription factor expressed in oligodendrocyte cells in the central nervous system, is expressed at elevated levels in eosinophils. (E) PMP22, which encodes peripheral myelin protein expressed in Schwann cells in the peripheral nervous system, is expressed in multiple myeloid lineage cell types.

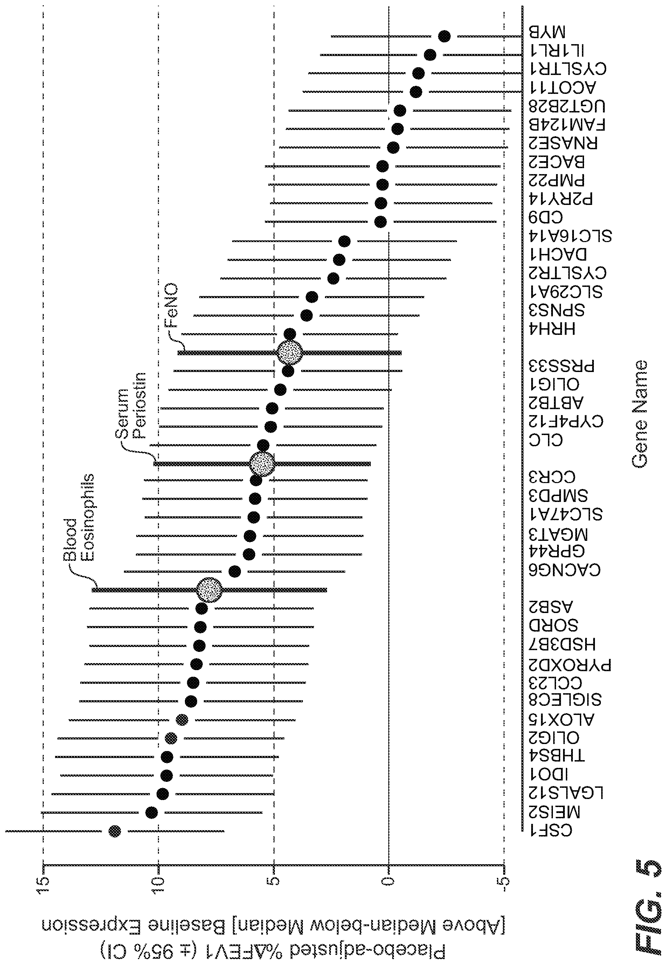

**Figure 5.** Expression of peripheral blood genes related to eosinophils identifies moderate-severe asthma patients with increased clinical benefit from lebrikizumab. Blood gene expression was successfully measured at day 0 prior to the first dose of lebrikizumab or placebo in 200 patients in the MILLY study (Corren et al. (2011) N Engl J Med 365: 1088-98) and patients were divided according to the median level of each transcript indicated on the x-axis. The difference in percent change from baseline in mean $FEV_1$ between lebrikizumab and placebo-treated patients after 12 weeks of treatment in patients with gene expression above vs. below the median level for each gene is shown. Dots represent mean placebo adjusted change in $FEV_1$ and whiskers represent 95% confidence intervals. Thin black lines with solid circles, selected individual genes; Thick black lines with stippled circles, non-gene expression biomarkers serum periostin, blood eosinophil counts, and FeNO in the same population of patients for whom gene expression data were available are indicated for illustrative purposes.

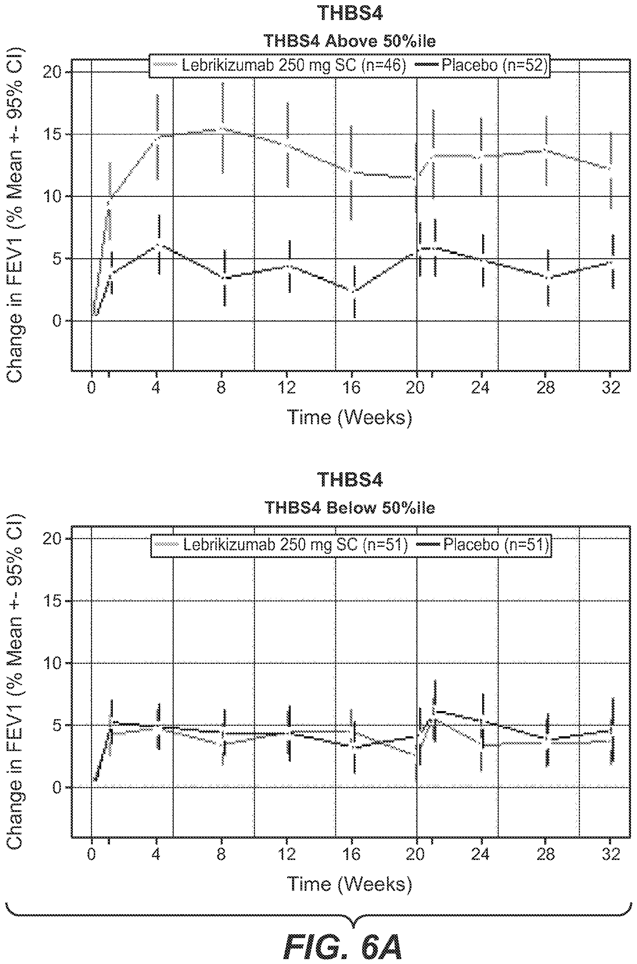

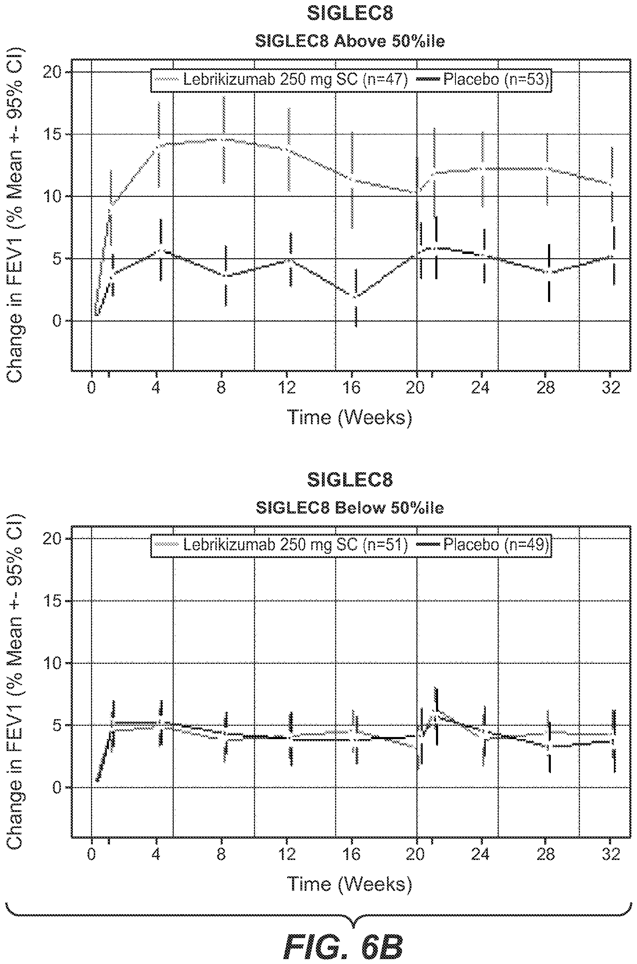

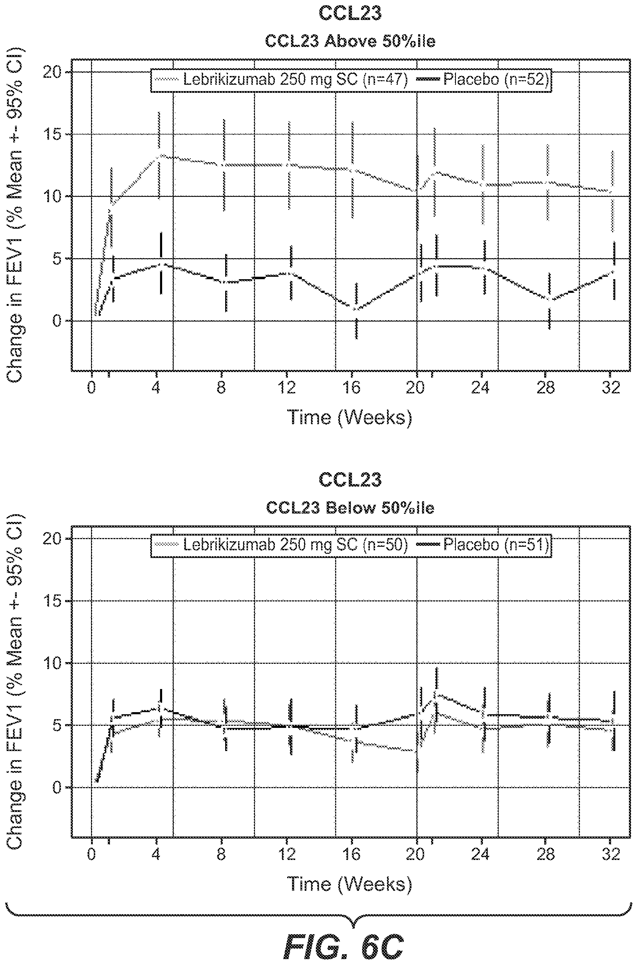

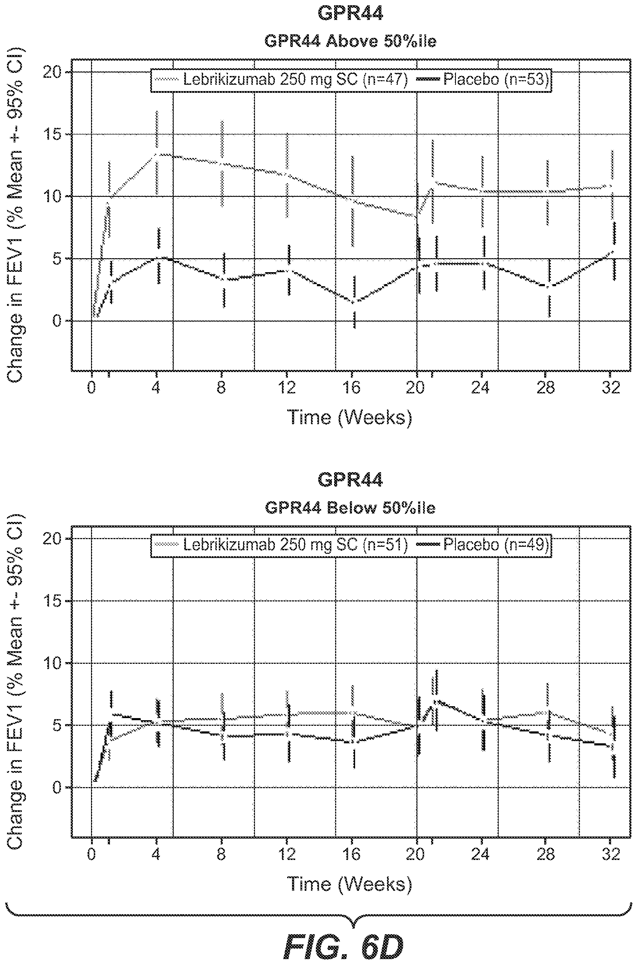

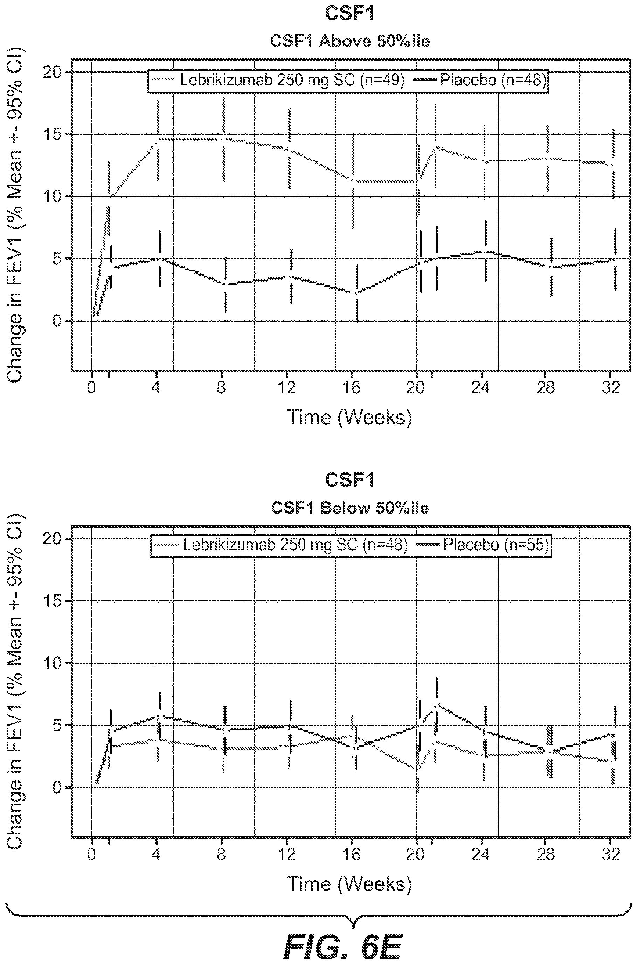

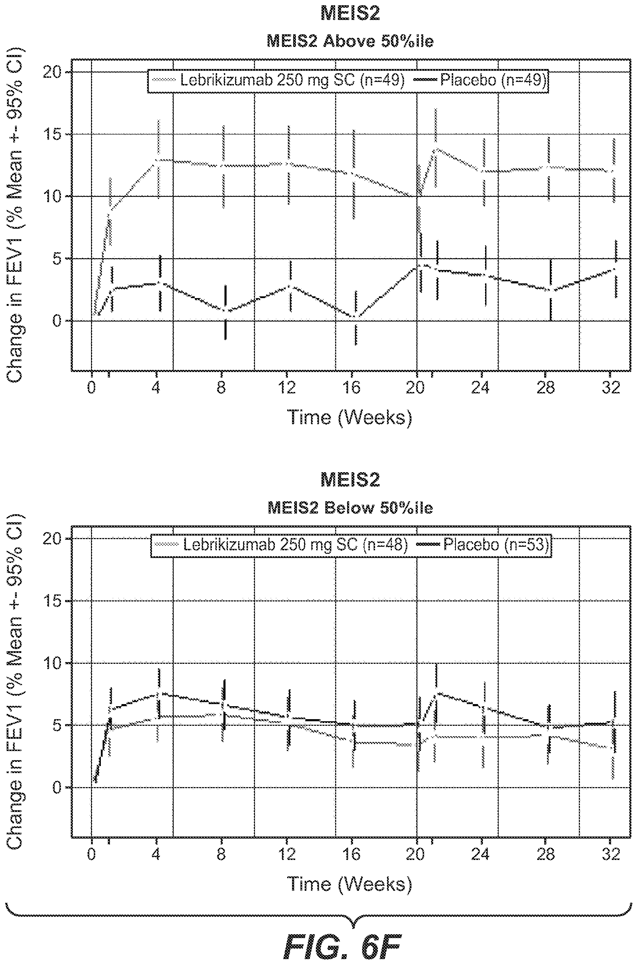

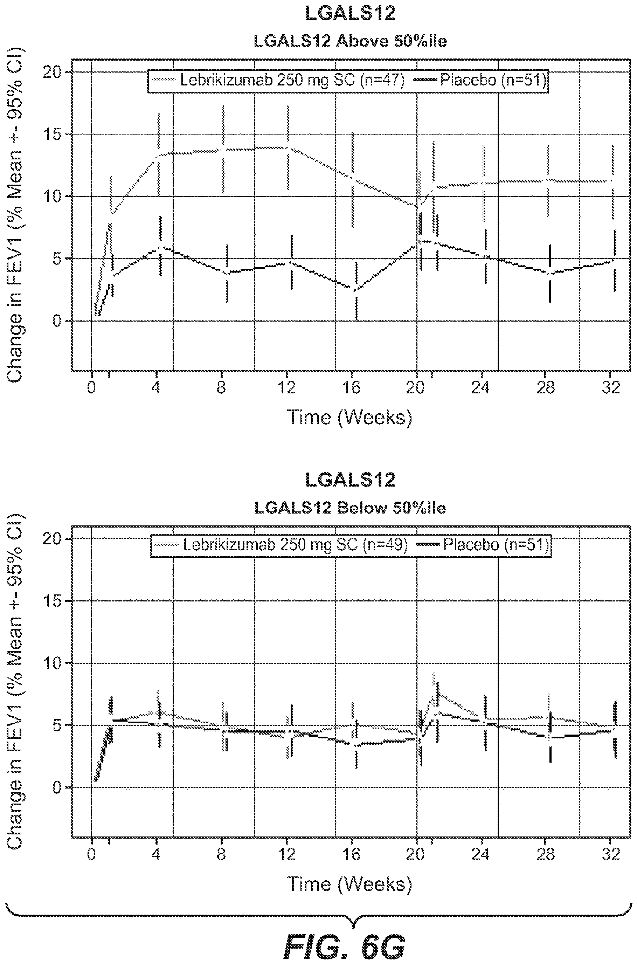

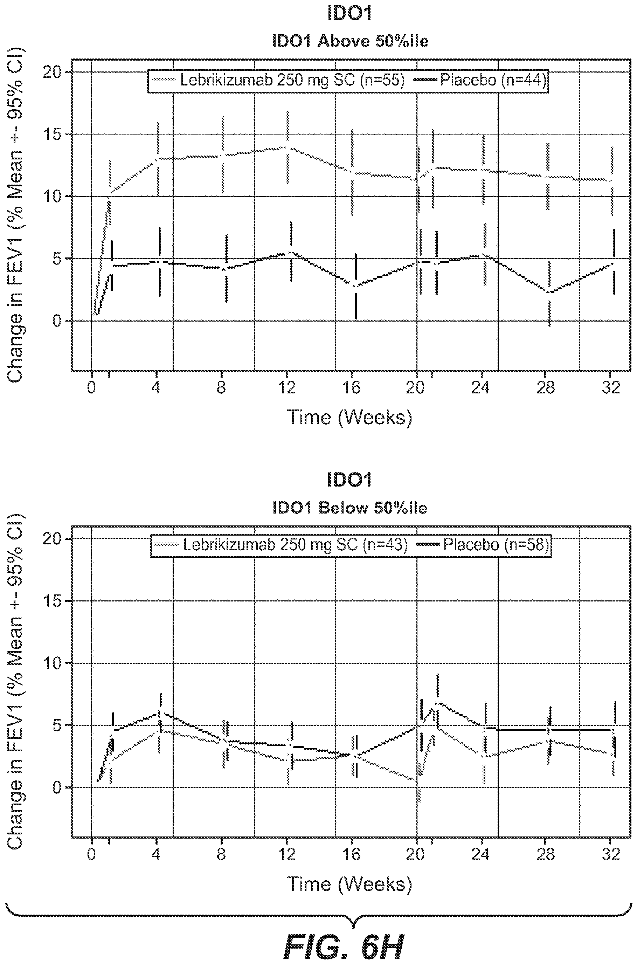

**Figure 6.** Consistent enhancement of clinical response in patients with gene expression levels above vs. below the median over a 32-week period. Selected genes shown: (A) THBS4; (B) SIGLEC8; (C) CCL23; (D) GPR44; (E) CSF1; (F) MEIS2; (G) LGALS12; (H) IDOI. Percent change from baseline in $FEV_1$ in lebrikizumab-treated patients shown in grey, placebo-treated patients shown in black. Patients with gene expression levels above the median for the entire population shown in upper panels; below the median shown in lower panels. In cases where there was missing data, last observation carried forward (LOCF) was used as per Corren et al. (2011) N Engl J Med 365: 1088-98.

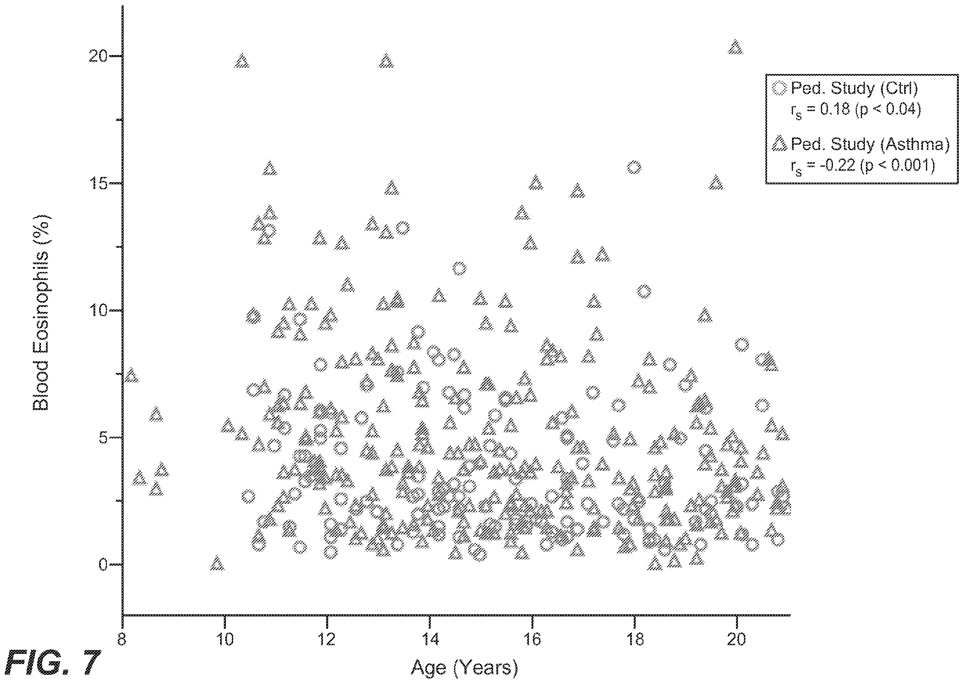

**Figure 7.** Relationship between blood eosinophil percentage and age in the GALA II pediatric cohort (Ped. Study). Asthmatic patients shown in triangles; healthy controls in circles. rS, Spearman's rank-order correlation coefficient.

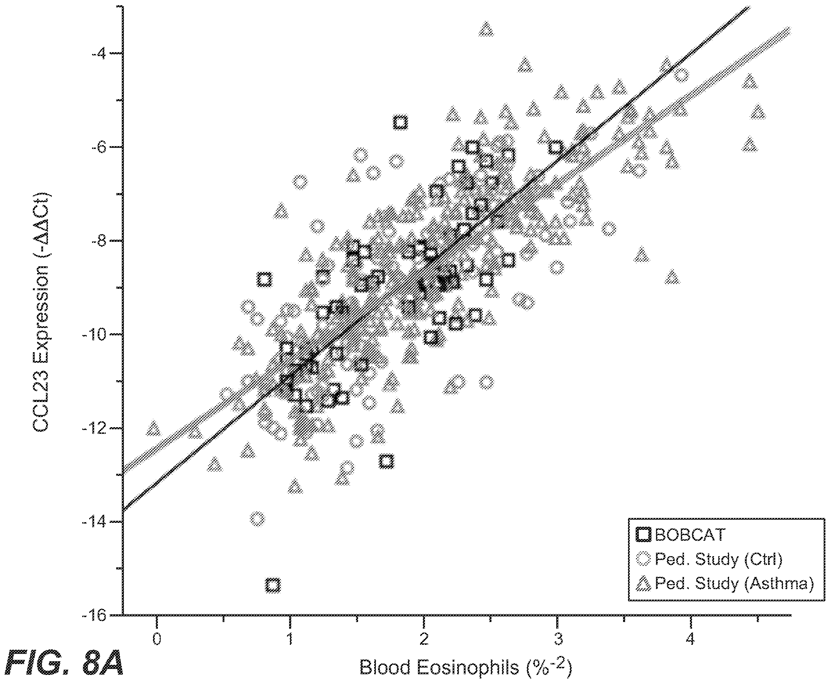

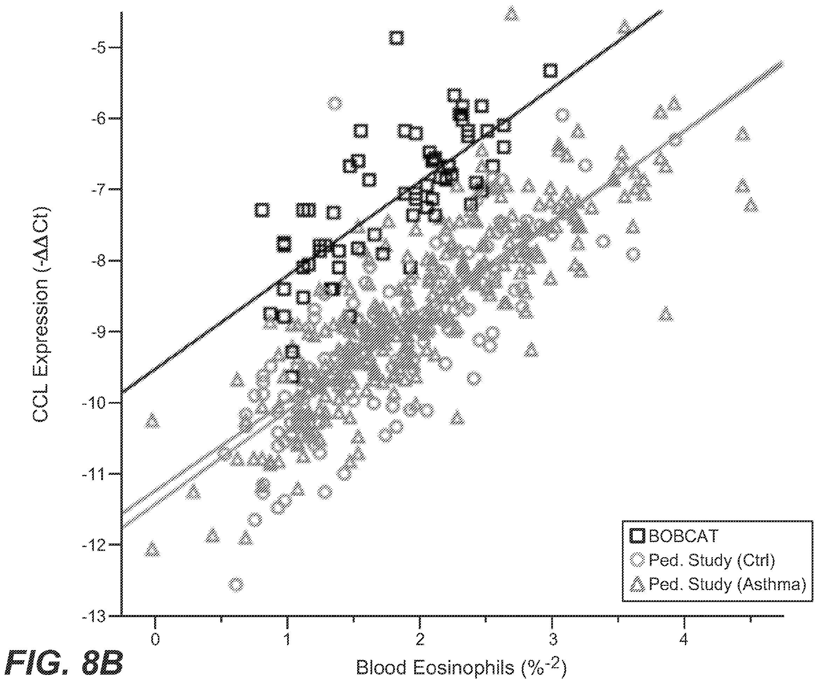

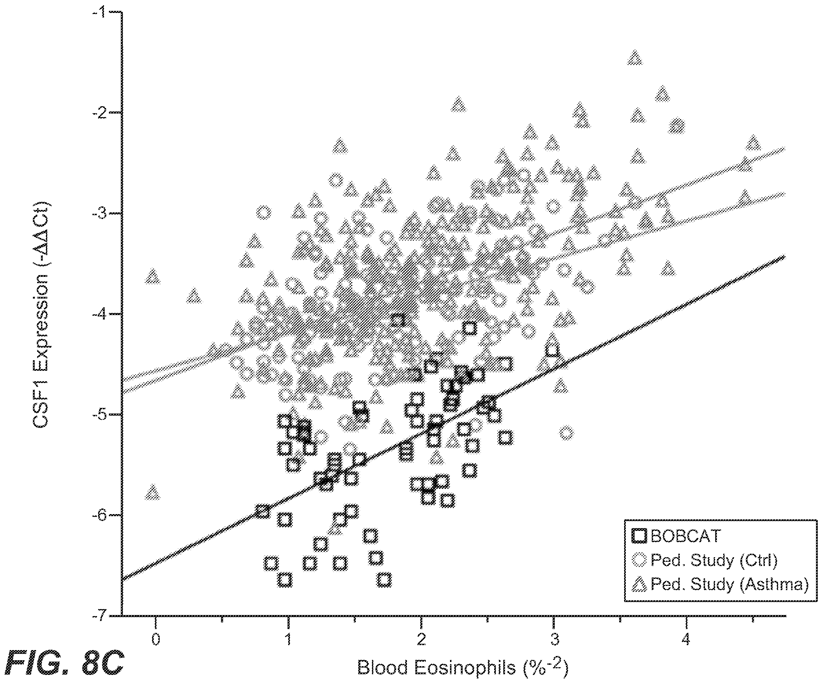

**Figure 8.** Examples of match and mismatch between gene expression and blood eosinophil percentage in adult and pediatric subjects. Expression of peripheral blood levels of eosinophil-related transcripts (-ΔΔCt) plotted as a function of the square root of blood eosinophil percentage. Adult moderate-severe asthmatic subjects (BOBCAT) shown in squares; pediatric asthmatic subjects (GALA [Ped. Study]) shown in triangles; pediatric healthy control subjects (GALA [Ped. Study]) shown in circles. (A) CCL23 expression exhibits a consistent relationship to blood eosinophilia regardless of age or diagnosis; (B) CCL expression is comparably related to blood eosinophilia but at different levels in adults and pediatric subjects; (C) CSF1 expression varies in both correlation coefficient and scaling between adult and pediatric subjects.

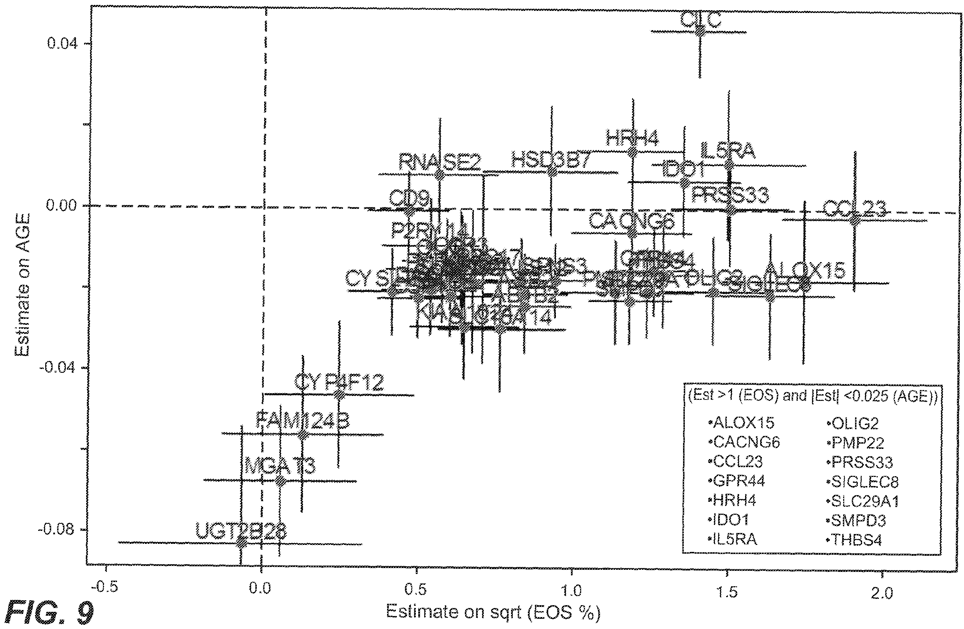

**Figure 9.** Model of effects of age and blood eosinophil percentage on gene expression in MILLY, BOBCAT, and GALA II cohorts. A linear regression model was constructed: Gene expression = (blood eosinophil percentage)-2 + Age + Age*(blood eosinophil percentage)-2 + batch and the estimates for blood eosinophil percentage and age are plotted with 95% confidence intervals for each transcript. Genes with absolute value estimates > 1 for eosinophil percentage and < 0.025 for age are listed as the transcripts maximizing relationships to eosinophil percentage independent of age.

## DETAILED DESCRIPTION

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

## CERTAIN DEFINITIONS

For purposes of interpreting this specification, the following definitions will apply and whenever appropriate, terms used in the singular will also include the plural and vice versa.

As used in this specification and the appended claims, the singular forms "a," "an" and "the" include plural referents unless the context clearly dictates otherwise. Thus, for example, reference to "a protein" or an "antibody" includes a plurality of proteins or antibodies, respectively; reference to "a cell" includes mixtures of cells, and the like.

As used herein, "Eosinophilic Inflammation Diagnostic Assay," abbreviated "EIDA" or "EID Assay" is an assay that diagnoses a patient having eosinophilic inflammation in the body or TH2 pathway inflammation in the body by measuring levels of at least one eosinophilic inflammation marker in a biological sample from a patient, wherein at least one, at least two, or at least three of the markers is selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2. In some examples, an EID Assay comprises measuring levels of at least one eosinophilic inflammation marker in a biological sample from a patient, wherein at least one, at least two, or at least three of the markers is selected from CSF1, MEIS2, CCL23, HSD3B7, SORD, CACNG6, MGAT3, SLC47A1, and ABTB2. In some examples, an EID Assay comprises measuring levels of at least one eosinophilic inflammation marker in a biological sample from a patient, wherein at least one, at least two, or at least three of the markers is selected from MEIS2, LGALS12, IDOI, ALOX15, SIGLEC8, CCL23, PYROXD2, HSD3B7, CACNG6, and GPR44. In some examples, an EID Assay comprises measuring levels of at least one eosinophilic inflammation marker in a biological sample from a patient, wherein at least one, at least two, or at least three of the markers is selected from CCL23, IDOI, HSD3B7, and CACNG6. In some examples, an EID Assay comprises measuring levels of at least one eosinophilic inflammation marker in a biological sample from a patient, wherein at least one, at least two, or all three of the markers is selected from CCL23, IDOI, and CACNG6. In some examples, an EID Assay comprises measuring levels of at least one eosinophilic inflammation marker in a biological sample from a patient, wherein at least one, at least two, or all three of the markers is selected from HSD3B7, SIGLEC8, and GPR44. In some examples, an EID Assay comprises measuring levels of at least one eosinophilic inflammation marker in a biological sample from a patient, wherein at least one, at least two, at least three, or all four of the markers is selected from SIGLEC8, CCL23, CACNG6, and GPR44. In some examples, mRNA levels are measured. In some examples, two or more assays can be conducted to make a diagnosis of eosinophilic inflammation in a patient. In one example, the EID Assay comprises measuring levels of at least one eosinophilic inflammation marker as described above, in combination with a $FE_{NO}$ assay.

Eosinophilic Inflammation Positive (EIP) Patient or Status: refers to a patient who, if a biological sample from that patient had been tested for levels of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 would have a level of one or more of the selected markers that is above the reference level of the respective marker. In some examples, the biological sample is RNA obtained from blood, e.g., whole blood or a cellular fraction of blood, such as PBMC. In some examples, the biological sample is serum or plasma. In some examples, a patient is EIP if a biological sample from that patient had been tested for levels of at least one, at least two, or at least three markers selected from CSF1, MEIS2, CCL23, HSD3B7, SORD, CACNG6, MGAT3, SLC47A1, and ABTB2 would have a level of one or more of the markers that is above the reference level of the respective marker. In some examples, a patient is EIP if a biological sample from that patient had been tested for levels of at least one, at least two, or at least three markers selected from MEIS2, LGALS12, IDOI, ALOX15, SIGLEC8, CCL23, PYROXD2, HSD3B7, CACNG6, and GPR44 would have a level of one or more of the markers that is above the reference level of the respective marker. In some examples, a patient is EIP if a biological sample from that patient had been tested for levels of at least one, at least two, or at least three markers selected from CCL23, IDO1, HSD3B7, and CACNG6 would have a level of one or more of the markers that is above the reference level of the respective marker. In some examples, a patient is EIP if a biological sample from that patient had been tested for levels of at least one, at least two, or all three markers selected from CCL23, IDOI, and CACNG6 would have a level of one or more of the markers that is above the reference level of the respective marker. In some examples, a patient is EIP if a biological sample from that patient had been tested for levels of at least one, at least two, or all three markers selected from HSD3B7, SIGLEC8, and GPR44 would have a level of one or more of the markers that is above the reference level of the respective marker. In some examples, a patient is EIP if a biological sample from that patient had been tested for levels of at least one, at least two, at least three, or all four markers selected from SIGLEC8, CCL23, CACNG6, and GPR44 would have a level of one or more of the markers that is above the reference level of the respective marker. In some examples, the reference level is the median level in a reference population. In some examples, a reference level of a marker is the mean level of the marker in a reference population. In some examples, a reference level of a marker is the average level of the marker in a reference population. Nonlimiting exemplary reference populations include patients with asthma, patients with moderate to severe asthma, healthy individuals, and a group including healthy individuals and patients with asthma. In some examples, a reference population comprises patients with moderate to severe asthma. Further nonlimiting exemplary reference populations include patients with an eosinophilic disorder (including the eosinophilic disorders described herein), such as patients with atopic dermatitis, patients with allergic rhinitis, patients with nasal polyposis, patients with eosinophilic esophagitis, patients with hyper-eosinophilic syndrome, etc.

In any of the examples described herein, the level of an mRNA that encodes the marker may be determined. Various methods of determining an mRNA level in a biological sample are known in the art and/or are described herein. In some examples, the biological sample is RNA obtained from blood, e.g., whole blood or a cellular fraction of blood, such as PBMC. In some examples, the biological sample is serum or plasma. In some examples, detection of the level of an mRNA comprises reverse transcription polymerase chain reaction (RT-PCR). In some examples, detection of the level of an mRNA comprises quantitative PCR (qPCR).

Eosinophilic Inflammation Negative (EIN) Patient or Status refers to a patient who, if a biological sample from that patient had been tested for levels of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 would have a level of each of the selected markers that is at or below the reference level of the respective marker. In some examples, the biological sample is RNA obtained from blood, e.g., whole blood or a cellular fraction of blood, such as PBMC. In some examples, the biological sample is serum or plasma. In some examples, a patient is EIN if a biological sample from that patient had been tested for levels of at least one, at least two, or at least three markers selected from CSF1, MEIS2, CCL23, HSD3B7, SORD, CACNG6, MGAT3, SLC47A1, and ABTB2 would have a level of each of the selected markers that is at or below the reference level of the respective marker. In some examples, a patient is EIN if a biological sample from that patient had been tested for levels of at least one, at least two, or at least three markers selected from MEIS2, LGALS12, IDOI, ALOX15, SIGLEC8, CCL23, PYROXD2, HSD3B7, CACNG6, and GPR44 would have a level of each of the selected markers that is at or below the reference level of the respective marker. In some examples, a patient is EIN if a biological sample from that patient had been tested for levels of at least one, at least two, or at least three markers selected from CCL23, IDOI, HSD3B7, and CACNG6 would have a level of each of the selected markers that is at or below the reference level of the respective marker. In some examples, a patient is EIN if a biological sample from that patient had been tested for levels of at least one, at least two, or all three markers selected from CCL23, IDOI, and CACNG6 would have a level of each of the selected markers that is at or below the reference level of the respective marker. In some examples, a patient is EIN if a biological sample from that patient had been tested for levels of at least one, at least two, or all three markers selected from HSD3B7, SIGLEC8, and GPR44 would have a level of each of the selected markers that is at or below the reference level of the respective marker. In some examples, a patient is EIN if a biological sample from that patient had been tested for levels of at least one, at least two, at least three or all four markers selected from SIGLEC8, CCL23, CACNG6, and GPR44 would have a level of each of the selected markers that is at or below the reference level of the respective marker. In some examples, the reference level is the median level in a reference population. In some examples, a reference level of a marker is the mean level of the marker in a reference population. In some examples, a reference level of a marker is the average level of the marker in a reference population. Nonlimiting exemplary reference populations include patients with asthma, patients with moderate to severe asthma, healthy individuals, and a group including healthy individuals and patients with asthma. In some examples, a reference population comprises patients with moderate to severe asthma. Further nonlimiting exemplary reference populations include patients with an eosinophilic disorder (including the eosinophilic disorders described herein), such as patients with atopic dermatitis, patients with allergic rhinitis, patients with nasal polyposis, patients with eosinophilic esophagitis, patients with hyper-eosinophilic syndrome, etc.

It should be understood that the EIN Status represents the state of the patient, and is not dependent on the type of assay used to determine the status. Thus, other Eosinophilic Inflammation Diagnostic EID) Assays can be used or developed to be used to test for Eosinophilic Inflammation Negative status.

In certain examples, the term "at the reference level" refers to a level of the biomarker in the sample from the individual or patient that is essentially identical to the reference level or to a level that differs from the reference level by up to 1%, up to 2%, up to 3%, up to 4%, up to 5%. In some examples, the reference level is the median level of the biomarker in a reference population. In some examples, a reference level of a marker is the mean level of the marker in a reference population. In some examples, a reference level of a marker is the average level of the marker in a reference population. Nonlimiting exemplary reference populations include patients with asthma, patients with moderate to severe asthma, healthy individuals, and a group including healthy individuals and patients with asthma. In some examples, a reference population comprises patients with moderate to severe asthma. Further nonlimiting exemplary reference populations include patients with an eosinophilic disorder (including the eosinophilic disorders described herein), such as patients with atopic dermatitis, patients with allergic rhinitis, patients with nasal polyposis, patients with eosinophilic esophagitis, patients with hyper-eosinophilic syndrome, etc.

In certain examples, the term "above the reference level" refers to a level of the biomarker in the sample from the individual or patient above the reference level by at least 5%, 10%, 20%, 25%, 30%, 40%, 50%, 60%, 70%, 80%, 85%, 90%, 95%, 100% or greater, determined by the methods described herein, as compared to the reference level. In some examples, the reference level is the median level in a reference population. In some examples, a reference level of a marker is the mean level of the marker in a reference population. In some examples, a reference level of a marker is the average level of the marker in a reference population. Nonlimiting exemplary reference populations include patients with asthma, patients with moderate to severe asthma, healthy individuals, and a group including healthy individuals and patients with asthma. In some examples, a reference population comprises patients with moderate to severe asthma. Further nonlimiting exemplary reference populations include patients with an eosinophilic disorder (including the eosinophilic disorders described herein), such as patients with atopic dermatitis, patients with allergic rhinitis, patients with nasal polyposis, patients with eosinophilic esophagitis, patients with hyper-eosinophilic syndrome, etc.

In certain examples, the term "below the reference level" refers to a level of the biomarker in the sample from the individual or patient below the reference level by at least 5%, 10%, 20%, 25%, 30%, 40%, 50%, 60%, 70%, 80%, 85%, 90%, 95%, 100% or greater, determined by the methods described herein, as compared to the reference level. In some examples, the reference level is the median level in a reference population. In some examples, a reference level of a marker is the mean level of the marker in a reference population. In some examples, a reference level of a marker is the average level of the marker in a reference population. Nonlimiting exemplary reference populations include patients with asthma, patients with moderate to severe asthma, healthy individuals, and a group including healthy individuals and patients with asthma. In some examples, a reference population comprises patients with moderate to severe asthma. Further nonlimiting exemplary reference populations include patients with an eosinophilic disorder (including the eosinophilic disorders described herein), such as patients with atopic dermatitis, patients with allergic rhinitis, patients with nasal polyposis, patients with eosinophilic esophagitis, patients with hyper-eosinophilic syndrome, etc.

The terms "marker" and "biomarker" are used interchangeably to refer to a molecule, including a gene, protein, carbohydrate structure, or glycolipid, metabolite, mRNA, miRNA, protein, DNA (cDNA or genomic DNA), DNA copy number, or an epigenetic change, e.g., increased, decreased, or altered DNA methylation (e.g., cytosine methylation, or CpG methylation, non-CpG methylations); histone modification (e.g., (de)acetylation, (de) methylation,(de) phosphorylation, ubiquitination, SUMOylation, ADP-ribosylation); altered nucleosome positioning, the expression or presence of which in or on a mammalian tissue or cell can be detected by standard methods (or methods disclosed herein) and which may be predictive, diagnostic and/or prognostic for a mammalian cell's or tissue's sensitivity to treatment regimes based on TH2 pathway inhibition using, for example, a TH2 pathway inhibitor described herein. A biomarker may also be a biological or clinical attribute that can be measured in a biological sample obtained from a subject, such as for example but not limited to, blood cell count, e.g., blood eosinophil count, $FEV_1$ or FeNO. In certain examples, the level of such a biomarker is determined to be higher or lower than that observed for a reference population. In certain examples, a blood eosinophil count is 200/$\mu$l, or 250/$\mu$l, or 300/$\mu$l, or 400/$\mu$l.

The term "comparing" refers to comparing the level of the biomarker in the sample from the individual or patient with the reference level of the biomarker specified elsewhere in this description. It is to be understood that comparing usually refers to a comparison of corresponding parameters or values, e.g., an absolute amount is compared to an absolute reference amount while a concentration is compared to a reference concentration or an intensity signal obtained from the biomarker in a sample is compared to the same type of intensity signal obtained from a reference sample. The comparison may be carried out manually or computer assisted. Thus, the comparison may be carried out by a computing device (e.g., of a system disclosed herein). The value of the measured or detected level of the biomarker in the sample from the individual or patient and the reference level can be, e.g., compared to each other and the said comparison can be automatically carried out by a computer program executing an algorithm for the comparison. The computer program carrying out the said evaluation will provide the desired assessment in a suitable output format. For a computer assisted comparison, the value of the determined amount may be compared to values corresponding to suitable references which are stored in a database by a computer program. The computer program may further evaluate the result of the comparison, i.e. automatically provide the desired assessment in a suitable output format. For a computer assisted comparison, the value of the determined amount may be compared to values corresponding to suitable references which are stored in a database by a computer program. The computer program may further evaluate the result of the comparison, i.e. automatically provides the desired assessment in a suitable output format.

The term "detecting" a biomarker refers to methods of detecting the presence or quantity of the biomarker in the sample employing appropriate methods of detection described elsewhere herein.

The term "measuring" the level of a biomarker refers to the quantification of the biomarker, e.g. to determining the level of the biomarker in the sample, employing appropriate methods of detection described elsewhere herein.

The term "monitoring the efficacy of a therapy" is used to indicate that a sample is obtained at least once, including serially, from a patient before and/or under therapy and that one or more biomarkers are is measured therein to obtain an indication whether the therapy is efficient or not.

In the monitoring of the efficacy of a therapy the levels of one or more biomarkers are measured and in some examples compared to a reference level for the biomarkers, or, in some examples, are compared to the level of the biomarkers in a sample obtained from the same patient at an earlier point in time. In some examples, the current levels of one or more biomarker are compared to the levels of the biomarkers in a sample obtained from the same patient before start of a therapy in said patient.

In some examples, a level of at least one, at least two, or at least three markers selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 that is above the reference level of the respective marker indicates that the patient is more likely to respond to the therapy. In some examples, a level of at least one, at least two, or at least three markers selected from CSF1, MEIS2, CCL23, HSD3B7, SORD, CACNG6, MGAT3, SLC47A1, and ABTB2 that is above the reference level of the respective marker indicates that the patient is more likely to respond to the therapy. In some examples, a level of at least one, at least two, or at least three markers selected from MEIS2, LGALS12, IDOI, ALOX15, SIGLEC8, CCL23, PYROXD2, HSD3B7, CACNG6, and GPR44 that is above the reference level of the respective marker indicates that the patient is more likely to respond to the therapy. In some examples, a level of at least one, at least two, or at least three markers selected from CCL23, IDOI, HSD3B7, and CACNG6 that is above the reference level of the respective marker indicates that the patient is more likely to respond to the therapy. In some examples, a level of at least one, at least two, or all three markers selected from CCL23, IDOI, and CACNG6 that is above the reference level of the respective marker indicates that the patient is more likely to respond to the therapy. In some examples, a level of at least one, at least two, or all three markers selected from HSD3B7, SIGLEC8, and GPR44 that is above the reference level of the respective marker indicates that the patient is more likely to respond to the therapy. In some examples, a level of at least one, at least two, at least three, or all four markers selected from SIGLEC8, CCL23, CACNG6, and GPR44 that is above the reference level of the respective marker indicates that the patient is more likely to respond to the therapy. In some examples, the reference level is the median level in a reference population. In some examples, a reference level of a marker is the mean level of the marker in a reference population. In some examples, a reference level of a marker is the average level of the marker in a reference population. Nonlimiting exemplary reference populations include patients with asthma, patients with moderate to severe asthma, healthy individuals, and a group including healthy individuals and patients with asthma. In some examples, a reference population comprises patients with moderate to severe asthma. Further nonlimiting exemplary reference populations include patients with an eosinophilic disorder (including the eosinophilic disorders described herein), such as patients with atopic dermatitis, patients with allergic rhinitis, patients with nasal polyposis, patients with eosinophilic esophagitis, patients with hyper-eosinophilic syndrome, etc.

The phrase "recommending a treatment" refers to using the information or data generated relating to the level or presence of one or more biomarkers described herein in a sample of a patient to identify the patient as suitably treated or not suitably treated with a TH2 pathway inhibitor. The phrase "recommending a treatment" may refer to using the information or data generated for proposing or selecting a therapy comprising a TH2 pathway inhibitor for a patient identified or selected as more or less likely to respond to the therapy comprising a TH2 pathway inhibitor. The information or data used or generated may be in any form, written, oral or electronic. In some examples, using the information or data generated includes communicating, presenting, reporting, storing, sending, transferring, supplying, transmitting, dispensing, or combinations thereof. In some examples, communicating, presenting, reporting, storing, sending, transferring, supplying, transmitting, dispensing, or combinations thereof are performed by a computing device, analyzer unit or combination thereof. In some further examples, communicating, presenting, reporting, storing, sending, transferring, supplying, transmitting, dispensing, or combinations thereof are performed by a laboratory or medical professional. In some examples, the information or data includes a comparison of the levels of one or more markers described herein to a reference level. In some examples, the information or data includes an indication that the patient is suitably treated or not suitably treated with a therapy comprising a TH2 pathway inhibitor, including, in some instances, an indication that the patient is suitably treated or not suitably treated with a therapy comprising a particular TH2 pathway inhibitor, such as an anti-IL13 antibody or an anti-M1' antibody.

The phrase "selecting a patient" or "identifying a patient" refers to using the information or data generated relating to the levels of one or more markers described herein in a sample of a patient to identify or select the patient as more likely to benefit or less likely to benefit from a therapy comprising a TH2 pathway inhibitor. The information or data used or generated may be in any form, written, oral or electronic. In some examples, using the information or data generated includes communicating, presenting, reporting, storing, sending, transferring, supplying, transmitting, dispensing, or combinations thereof. In some examples, communicating, presenting, reporting, storing, sending, transferring, supplying, transmitting, dispensing, or combinations thereof are performed by a computing device, analyzer unit or combination thereof. In some further examples, communicating, presenting, reporting, storing, sending, transferring, supplying, transmitting, dispensing, or combinations thereof are performed by a laboratory or medical professional. In some examples, the information or data includes a comparison of the levels of one or more markers described herein to a reference level. In some examples, the information or data includes an indication that the patient is suitably treated or not suitably treated with a therapy comprising a TH2 pathway inhibitor, including, in some instances, an indication that the patient is suitably treated or not suitably treated with a therapy comprising a particular TH2 pathway inhibitor, such as an anti-IL13 antibody or an anti-M1' antibody.

The phrase "selecting a therapy" refers to using the information or data generated relating to the level or presence of one or more markers described herein in a sample of a patient to identify or selecting a therapy for a patient.

In some example the therapy may comprise a TH2 pathway inhibitor. The phrase "recommending a treatment" also may refer to using the information or data generated for proposing or selecting a therapy comprising a TH2 pathway inhibitor for a patient identified or selected as more or less likely to respond to the therapy comprising a TH2 pathway inhibitor. The information or data used or generated may be in any form, written, oral or electronic. In some examples, using the information or data generated includes communicating, presenting, reporting, storing, sending, transferring, supplying, transmitting, dispensing, or combinations thereof. In some examples, communicating, presenting, reporting, storing, sending, transferring, supplying, transmitting, dispensing, or combinations thereof are performed by a computing device, analyzer unit or combination thereof. In some further examples, communicating, presenting, reporting, storing, sending, transferring, supplying, transmitting, dispensing, or combinations thereof are performed by a laboratory or medical professional. In some examples, the information or data includes an indication that the patient is suitably treated or not suitably treated with a therapy comprising a TH2 pathway inhibitor, including, in some instances, an indication that the patient is suitably treated or not suitably treated with a therapy comprising a particular TH2 pathway inhibitor, such as an anti-IL13 antibody or an anti-M1' antibody.

The term "biological sample" includes, but is not limited to, blood, serum, plasma, peripheral blood mononuclear cells (PBMCs), sputum, tissue biopsies (e.g., lung samples), and nasal samples including nasal swabs or nasal polyps. The sample may be taken before treatment, during treatment or post-treatment. The sample may be taken from a patient who is suspected of having, or is diagnosed as having asthma or a respiratory disorder, and hence is likely in need of treatment or from a normal individual who is not suspected of having any disorder. In some examples, RNA is extracted from a biological sample described herein prior to detecting or measuring the mRNA level of a marker.

The term "amplifying" a marker or biomarker refers to the amplification of the marker employing appropriate methods of marker amplification known in the art and/or described elsewhere herein.

FENO assay refers to an assay that measures $FE_{NO}$ (fractional exhaled nitric oxide) levels. Such levels can be evaluated using, e.g., a hand-held portable device, NIOX MINO® (Aerocrine, Solna, Sweden), in accordance with guidelines published by the American Thoracic Society (ATS) in 2005. $FE_{NO}$ may be noted in other similar ways, e.g., FeNO or FENO, and it should be understood that all such similar variations have the same meaning.

Age of Patients to be tested or treated according to the methods disclosed herein include: all ages. In some examples, the ages are 18+ years old. In some examples, the ages are 12+ years old. In some examples, the ages are 2+ years old. In some examples, the ages are 2-18 years old, 12-18 years old, 18-75 year olds, 12-75 year olds or 2-75 year olds.

Asthma is a complex disorder characterized by variable and recurring symptoms, reversible airflow obstruction (e.g., by bronchodilator) and bronchial hyperresponsiveness which may or may not be associated with underlying inflammation. Examples of asthma include aspirin sensitive/exacerbated asthma, atopic asthma, severe asthma, mild asthma, moderate to severe asthma, corticosteroid naive asthma, chronic asthma, corticosteroid resistant asthma, corticosteroid refractory asthma, newly diagnosed and untreated asthma, asthma due to smoking, asthma uncontrolled on corticosteroids and other asthmas as mentioned in J Allergy Clin Immunol (2010) 126(5):926-938.

Eosinophilic Disorder means: a disorder associated with excess eosinophil numbers in which atypical symptoms may manifest due to the levels or activity of eosinophils locally or systemically in the body. Disorders associated with excess eosinophil numbers or activity include but are not limited to, asthma (including aspirin sensitive asthma), atopic asthma, atopic dermatitis, allergic rhinitis (including seasonal allergic rhinitis), non-allergic rhinitis, asthma, severe asthma, chronic eosinophilic pneumonia, allergic bronchopulmonary aspergillosis, coeliac disease, Churg-Strauss syndrome (periarteritis nodosa plus atopy), eosinophilic myalgia syndrome, hypereosinophilic syndrome, oedematous reactions including episodic angiodema, helminth infections, where eosinophils may have a protective role, onchocercal dermatitis and Eosinophil- Associated Gastrointestinal Disorders, including but not limited to, eosinophilic esophagitis, eosinophilic gastritis, eosinophilic gastroenteritis, eosinophilic enteritis and eosinophilic colitis, nasal micropolyposis and polyposis, aspirin intolerance, asthma and obstructive sleep apnoea. Eosinophil-derived secretory products have also been associated with the promotion of angiogenesis and connective tissue formation in tumors and the fibrotic responses seen in conditions such as chronic asthma, Crohn's disease, scleroderma and endomyocardial fibrosis (Munitz A, Levi-Schaffer F. Allergy 2004; 59: 268-75, Adamko et al. Allergy 2005; 60: 13-22, Oldhoff, et al. Allergy 2005; 60: 693-6). Other examples include cancer (e.g., glioblastoma (such as glioblastoma multiforme), non-Hodgkin's lymphoma (NHL)), atopic dermatitis, allergic rhinitis, asthma, fibrosis, inflammatory bowel disease, pulmonary fibrosis (including idiopathic pulmonary fibrosis (IPF) and pulmonary fibrosis secondary to sclerosis), COPD, hepatic fibrosis.

IL-13 mediated disorder means a disorder associated with excess IL-13 levels or activity in which atypical symptoms may manifest due to the levels or activity of IL-13 locally and/or systemically in the body. Examples of IL-13 mediated disorders include: cancers (e.g., non-Hodgkin's lymphoma, glioblastoma), atopic dermatitis, allergic rhinitis, asthma, fibrosis, inflammatory bowel disease (e.g., Crohn's disease), lung inflammatory disorders (e.g., pulmonary fibrosis such as IPF), COPD, hepatic fibrosis.

IL-4 mediated disorder means: a disorder associated with excess IL4 levels or activity in which atypical symptoms may manifest due to the levels or activity of IL4 locally and/or systemically in the body. Examples of IL4 mediated disorders include: cancers (e.g., non-Hodgkin's lymphoma, glioblastoma), atopic dermatitis, allergic rhinitis, asthma, fibrosis, inflammatory bowel disease (e.g., Crohn's disease), lung inflammatory disorders (e.g., pulmonary fibrosis such as IPF), COPD, hepatic fibrosis.

IL-5 mediated disorder means: a disorder associated with excess IL5 levels or activity in which atypical symptoms may manifest due to the levels or activity of IL5 locally and/or systemically in the body. Examples of IL5 mediated disorders include: cancers (e.g., non-Hodgkin's lymphoma, glioblastoma), atopic dermatitis, allergic rhinitis, asthma, fibrosis, inflammatory bowel disease (e.g., Crohn's disease), lung inflammatory disorders (e.g., pulmonary fibrosis such as IPF), COPD, hepatic fibrosis.

IL-9 mediated disorder means: a disorder associated with excess IL9 levels or activity in which atypical symptoms may manifest due to the levels or activity of IL9 locally and/or systemically in the body. Examples of IL9 mediated disorders include: cancers (e.g., non-Hodgkin's lymphoma, glioblastoma), atopic dermatitis, allergic rhinitis, asthma, fibrosis, inflammatory bowel disease (e.g., Crohn's disease), lung inflammatory disorders (e.g., pulmonary fibrosis such as IPF), COPD, hepatic fibrosis.

TSLP mediated disorder means: a disorder associated with excess TSLP levels or activity in which atypical symptoms may manifest due to the levels or activity of TSLP locally and/or systemically in the body. Examples of TSLP mediated disorders include: cancers (e.g., non-Hodgkin's lymphoma, glioblastoma), atopic dermatitis, allergic rhinitis, asthma, fibrosis, inflammatory bowel disease (e.g., Crohn's disease), lung inflammatory disorders (e.g., pulmonary fibrosis such as IPF), COPD, hepatic fibrosis.

IgE-mediated disorder means: a disorder associated with excess IgE levels or activity in which atypical symptoms may manifest due to levels of IgE locally and/or systemically in the body. Such disorders include, asthma, atopic dermatitis, allergic rhinitis, fibrosis (e.g., pulmonary fibrosis, such as IPF).

Asthma-Like Symptom includes a symptom selected from the group consisting of shortness of breath, cough (changes in sputum production and/or sputum quality and/or cough frequency), wheezing, chest tightness, bronchioconstriction and nocturnal awakenings ascribed to one of the symptoms above or a combination of these symptoms (Juniper et al (2000) Am. J. Respir. Crit. Care Med., 162(4), 1330-1334.).

The term "respiratory disorder" include, but is not limited to asthma (e.g., allergic and non-allergic asthma (e.g., due to infection, e.g., with respiratory syncytial virus (RSV), e.g., in younger children)); bronchitis (e.g., chronic bronchitis); chronic obstructive pulmonary disease (COPD) (e.g., emphysema (e.g., cigarette-induced emphysema); conditions involving airway inflammation, eosinophilia, fibrosis and excess mucus production, e.g., cystic fibrosis, pulmonary fibrosis, and allergic rhinitis. Examples of diseases that can be characterized by airway inflammation, excessive airway secretion, and airway obstruction include asthma, chronic bronchitis, bronchiectasis, and cystic fibrosis.

Exacerbations (commonly referred to as asthma attacks or acute asthma) are episodes of new or progressive increase in shortness of breath, cough (changes in sputum production and/or sputum quality and/or cough frequency), wheezing, chest tightness, nocturnal awakenings ascribed to one of the symptoms above or a combination of these symptoms. Exacerbations are often characterized by decreases in expiratory airflow (PEF or $FEV_1$). However, PEF variability does not usually increase during an exacerbation, although it may do so leading up to or during the recovery from an exacerbation. The severity of exacerbations ranges from mild to life-threatening and can be evaluated based on both symptoms and lung function. Severe asthma exacerbations as described herein include exacerbations that result in any one or combination of the following hospitalization for asthma treatment, high corticosteroid use (e.g., quadrupling the total daily corticosteroid dose or a total daily dose of greater or equal to 500 micrograms of FP or equivalent for three consecutive days or more), or oral/parenteral corticosteroid use.

A TH2 pathway inhibitor is an agent that inhibits the TH2 pathway.

Examples of a TH2 pathway inhibitor include inhibitors of the activity of any one of the targets selected from the group consisting of: ITK, BTK , IL-9 (e.g., MEDI-528), IL-5 (e.g., Mepolizumab, CAS No. 196078-29-2; resilizumab), IL-13 (e.g., IMA-026, IMA-638 (also referred to as, anrukinzumab, INN No. 910649-32-0; QAX-576; IL4/IL13 trap), tralokinumab (also referred to as CAT-354, CAS No. 1044515-88-9); AER-001, ABT-308 (also referred to as humanized 13C5.5 antibody), IL-4 (e.g., AER-001, IL4/IL13 trap), OX40L, TSLP, IL-25, IL-33, soluble IgE (e.g., XOLAIR, QGE-031; MEDI-4212) and membrane-bound IgE (quilizumab); and receptors such as: IL-9 receptor, IL-5 receptor (e.g., MEDI-563 (benralizumab, CAS No. 1044511-01-4), IL-4receptor alpha (e.g., AMG-317, AIR-645, dupilumab), IL-13receptoralpha1 (e.g., R-1671) and IL-13receptoralpha2, OX40, TSLP-R, IL-7Ralpha (a co-receptor for TSLP), IL17RB (receptor for IL-25), ST2 (receptor for IL-33), CCR3, CCR4, CRTH2 (e.g., AMG-853, AP768, AP-761, MLN6095, ACT129968), FcepsilonRI, FcepsilonRII/CD23 (receptors for IgE), Flap (e.g., GSK2190915), Syk kinase (R-343, PF3526299); CCR4 (AMG-761), TLR9 (QAX-935) and multi-cytokine inhibitor of CCR3, IL5, IL3, GM-CSF (e.g., TPI ASM8). Examples of inhibitors of the aforementioned targets are disclosed in, for example, WO2008/086395; WO2006/085938; US 7,615,213; US 7,501,121; WO2006/085938; WO 2007/080174; US 7,807,788; WO2005007699; WO2007036745; WO2009/009775; WO2007/082068; WO2010/073119; WO2007/045477; WO2008/134724; US2009/0047277; and WO2008/127,271).

A therapeutic agent as disclosed herein includes an agent that can bind to the target identified herein above, such as a polypeptide(s) (e.g., an antibody, an immunoadhesin or a peptibody), an aptamer or a small molecule that can bind to a protein or a nucleic acid molecule that can bind to a nucleic acid molecule encoding a target identified herein (i.e., siRNA).

"An anti-IL13/IL4 pathway inhibitor" refers to a therapeutic agent that inhibits IL-13 and/or IL-4 signaling. Examples of an anti-IL13/IL4 pathway inhibitors includes inhibitors of the interaction of IL13 and/or IL4 with its receptor(s), such inhibitors include, but are not limited to, anti-IL13 binding agents, anti-IL4 binding agents, anti-IL3/IL4 bispecific binding agents, anti-IL4receptoralpha binding agents, anti-IL13receptoralpha1 binding agents and anti-IL13 receptoralpha2 binding agents. Single domain antibodies that can bind IL13, IL4, (including bispecific antibody with a single domain binding IL13 and a single domain binding IL4), IL-13Ralpha1, IL-13Ralpha2 or IL-4Ralpha are specifically included as inhibitors. It should be understood that molecules that can bind more than one target are included.

"Anti-IL4 binding agents" refers to agent that binds to human IL-4. Such binding agents can include a small molecule, an aptamer or a polypeptide. Such polypeptide can include, but is not limited to, a polypeptide(s) selected from the group consisting of an immunoadhesin, an antibody, a peptibody and a peptide. According to one example, the binding agent binds to a human IL-4 sequence with an affinity between 1 uM - 1 pM. Specific examples of anti-IL4 binding agents can include soluble IL4Receptor alpha (e.g., extracellular domain of IL4Receptor fused to a human Fc region), anti-IL4 antibody, and soluble IL13receptoralpha1 (e.g., extracellular domain of IL13receptoralpha1 fused to a human Fc region).

"Anti-IL4receptoralpha binding agents" refers to an agent that binds to human IL4 receptoralpha. Such binding agents can include a small molecule, an aptamer or a polypeptide. Such polypeptide can include, but is not limited to, a polypeptide(s) selected from the group consisting of an immunoadhesin, an antibody, a peptibody and a peptide. According to one example, the binding agent binds to a human IL-4 receptor alpha sequence with an affinity between 1 uM - 1 pM. Specific examples of anti-IL4 receptoralpha binding agents can include anti-IL4 receptor alpha antibodies.

"Anti-IL13 binding agent" refers to agent that binds to human IL13. Such binding agents can include a small molecule, aptamer or a polypeptide. Such polypeptide can include, but is not limited to, a polypeptide(s) selected from the group consisting of an immunoadhesin, an antibody, a peptibody and a peptide. According to one example, the binding agent binds to a human IL-13 sequence with an affinity between 1 uM - 1 pM. Specific examples of anti-IL13 binding agents can include anti-IL13 antibodies, soluble IL13receptoralpha2 fused to a human Fc, soluble IL4receptoralpha fused to a human Fc, soluble IL13 receptoralpha fused to a human Fc. According to one example, the anti-IL13 antibody comprises (1) a HVRH1 comprising the amino acid sequence SEQ ID NO 11, (2) HVRH2 comprising the amino acid sequence SEQ ID NO:12, (3) HVRH3 comprising the amino acid sequence SEQ ID NO:13, (4) HVRL1 comprising the amino acid sequence SEQ ID NO:14, (5) HVRL2 comprising the amino acid sequence SEQ ID NO:15, and (6) HVRL3 comprising the amino acid sequence SEQ ID NO:16. In another example, the anti-IL-13 antibody comprises a VH comprising a sequence selected from SEQ ID NOs: 9, 19, and 21, and VL comprising a sequence selected from SEQ ID NO: 10, 20, and 22. According to one example, the antibody is an IgG1 antibody. According to another example, the antibody is an IgG4 antibody. According to one example, the IgG4 antibody comprises a S228P mutation in its constant domain.

Anti-IL13receptoralpha1 binding agents" refers to an agent that specifically binds to human IL13 receptoralpha1. Such binding agents can include a small molecule, aptamer or a polypeptide. Such polypeptide can include, but is not limited to, a polypeptide(s) selected from the group consisting of an immunoadhesin, an antibody, a peptibody and a peptide. According to one example, the binding agent binds to a human IL-13 receptor alpha1 sequence with an affinity between 1 uM - 1 pM. Specific examples of anti-IL13 receptoralpha1 binding agents can include anti-IL13 receptor alpha1 antibodies.

"Anti-IL13receptoralpha2 binding agents" refers to an agent that specifically binds to human IL13 receptoralpha2. Such binding agents can include a small molecule, an aptamer or a polypeptide. Such polypeptide can include, but is not limited to, a polypeptide(s) selected from the group consisting of an immunoadhesin, an antibody, a peptibody and a peptide. According to one example, the binding agent binds to a human IL-13 receptor alpha2 sequence with an affinity between 1 $\mu$M - 1 pM. Specific examples of anti-IL13 receptoralpha2 binding agents can include anti-IL13 receptor alpha2 antibodies.

"Anti IgE binding agents" refers to an agent that specifically binds to human IgE. Such binding agents can include a small molecule, an aptamer or a polypeptide. Such polypeptide can include, but is not limited to, a polypeptide(s) selected from the group consisting of an immunoadhesin, an antibody, a peptibody and a peptide. According to one example, the anti-IgE antibody comprises a VL sequence comprising the amino acid sequence of SEQ ID NO:17 and a VH sequence comprising the amino acid sequence SEQ ID NO:18.

"Anti-M1' binding agents" refers to an agent that specifically binds to the membrane proximal M1' region of surface expressed IgE on B cells. Such binding agents can include a small molecule, an aptamer or a polypeptide. Such polypeptide can include, but is not limited to, a polypeptide(s) selected from the group consisting of an immunoadhesin, an antibody, a peptibody and a peptide. According to one example, the anti-IgE antibody comprises an antibody described in WO2008/116149 or a variant thereof. According to another example, the anti-M1' antibody comprises a variable heavy chain and a variable light chain, wherein the variable heavy chain is SEQ ID NO:1 and the variable light chain is SEQ ID NO:2. According to another example, An anti-IgE/Ml' antibody comprising a variable heavy chain and a variable light chain, wherein the variable heavy chain further comprises an HVR-H1, HVR-H2 and HVR-H3, and the variable light chain further comprises and HVR-L1, HVR, L2 and HVR-L3 and: (a) the HVR-H1 is residues 26-35 of SEQ ID NO:1, [GFTFSDYGIA; SEQ ID NO:3]; (b) the HVR-H2 is residues 49-66 of SEQ ID NO:1, [AFISDLAYTIYYADTVTG; SEQ ID NO:4]; (c) the HVR-H3 is residues 97-106 of SEQ ID NO:1, [ARDNWDAMDY; SEQ ID NO:5]; (d) the HVR-L1 is residues 24-39 of SEQ ID NO:2, [RSSQSLVHNNANTYLH; SEQ ID NO:6]; (e) the HVR-L2 is residues 55-61 of SEQ ID NO:2, [KVSNRFS; SEQ ID NO:7]; (f) the HVR-L3 is residues 94-102 of SEQ ID NO:2. [SQNTLVPWT; SEQ ID NO:8].

The term "small molecule" refers to an organic molecule having a molecular weight between 50 Daltons to 2500 Daltons.

The term "antibody" is used in the broadest sense and specifically covers, for example, monoclonal antibodies, polyclonal antibodies, antibodies with polyepitopic specificity, single chain antibodies, multi-specific antibodies and fragments of antibodies. Such antibodies can be chimeric, humanized, human and synthetic. Such antibodies and methods of generating them are described in more detail below.

The term "uncontrolled" or "uncontrollable" refers to the inadequacy of a treatment regimen to minimize a symptom of a disease. As used herein, the term "uncontrolled" and "inadequately controlled" can be used interchangeably and are meant to refer to the same state. The control status of a patient can be determined by the attending physician based on a number of factors including the patient's clinical history, responsiveness to treatment and level of current treatment prescribed. For example, a physician may consider factors such as $FEV_1$ <75% predicted or personal best, frequency of need for a SABA in the past 2-4 weeks (e.g., greater than or equal two doses/week), nocturnal awakenings/symptoms in the past 2-4 weeks (e.g., less than or equal to 2 nights/week), limitations on activity in the past 2-4 weeks, daytime symptoms in the past 2-4 weeks

The term "therapeutic agent" refers to any agent that is used to treat a disease.

The term "controller" or "preventor" refers to any therapeutic agent that is used to control asthma inflammation. Examples of controllers include corticosteroids, leukotriene receptor antagonists (e.g., inhibit the synthesis or activity of leukotrienes such as montelukast, zileuton, pranlukast, zafirlukast), LABAs, corticosteroid/LABA combination compositions , theophylline (including aminophylline), cromolyn sodium, nedocromil sodium, omalizumab, LAMAs, MABA (e.g, bifunctional muscarinic antagonist-beta2 Agonist), 5-Lipoxygenase Activating Protein (FLAP) inhibitors, and enzyme PDE-4 inhibitor (e.g., roflumilast). A "second controller" typically refers to a controller that is not the same as the first controller.

The term "corticosteroid sparing" or "CS" means the decrease in frequency and/or amount, or the elimination of, corticosteroid used to treat a disease in a patient taking corticosteroids for the treatment of the disease due to the administration of another therapeutic agent. A "CS agent" refers to a therapeutic agent that can cause CS in a patient taking a corticosteroid.

The term "corticosteroid" includes, but is not limited to fluticasone (including fluticasone propionate (FP)), beclometasone, budesonide, ciclesonide, mometasone, flunisolide, betamethasone and triamcinolone. "Inhalable corticosteroid" means a corticosteroid that is suitable for delivery by inhalation. Exemplary inhalable corticosteroids are fluticasone, beclomethasone dipropionate, budenoside, mometasone furoate, ciclesonide, flunisolide, triamcinolone acetonide and any other corticosteroid currently available or becoming available in the future. Examples of corticosteroids that can be inhaled and are combined with a long-acting beta2-agonist include, but are not limited to: budesonide/formoterol and fluticasone/salmeterol.

Examples of corticosteroid/LABA combination drugs include fluticasone furoate/vilanterol trifenatate and indacaterol/mometasone.

The term "LABA" means long-acting beta-2 agonist, which agonist includes, for example, salmeterol, formoterol, bambuterol, albuterol, indacaterol, arformoterol and clenbuterol.

The term "LAMA" means long-acting muscarinic antagonist, which agonists include: tiotropium.

Examples of LABA/LAMA combinations include, but are not limited to: olodaterol tiotropium (Boehringer Ingelheim's) and indacaterol glycopyrronium (Novartis)

The term "SABA" means short-acting beta-2 agonists, which agonists include, but are not limited to, salbutamol, levosalbutamol, fenoterol, terbutaline, pirbuterol, procaterol, bitolterol, rimiterol, carbuterol, tulobuterol and reproterol

Leukotriene receptor antagonists (sometimes referred to as a leukast) (LTRA) are drugs that inhibit leukotrienes. Examples of leukotriene inhibitors include montelukast, zileuton, pranlukast, and zafirlukast.

The term "$FEV_1$" refers to the volume of air exhaled in the first second of a forced expiration. It is a measure of airway obstruction. Provocative concentration of methacholine required to induce a 20% decline in $FEV_1$ (PC20) is a measure of airway hyperresponsiveness. $FEV_1$ may be noted in other similar ways, e.g., $FEV_1$, and it should be understood that all such similar variations have the same meaning.

The term "relative change in $FEV_1$" = ($FEV_1$ at week 12 of treatment - $FEV_1$ prior to start of treatment) divided by $FEV_1$

The term "mild asthma" refers to a patient generally experiencing symptoms or exacerbations less than two times a week, nocturnal symptoms less than two times a month, and is asymptomatic between exacerbations. Mild, intermittent asthma is often treated as needed with the following: inhaled bronchodilators (short-acting inhaled beta2-agonists); avoidance of known triggers; annual influenza vaccination; pneumococcal vaccination every 6 to 10 years, and in some cases, an inhaled beta2-agonist, cromolyn, or nedocromil prior to exposure to identified triggers. If the patient has an increasing need for short-acting beta2-agonist (e.g., uses short-acting beta2-agonist more than three to four times in 1 day for an acute exacerbation or uses more than one canister a month for symptoms), the patient may require a stepup in therapy.

The term "moderate asthma" generally refers to asthma in which the patient experiences exacerbations more than two times a week and the exacerbations affect sleep and activity; the patient has nighttime awakenings due to asthma more than two times a month; the patient has chronic asthma symptoms that require short-acting inhaled beta2-agonist daily or every other day; and the patient's pretreatment baseline PEF or $FEV_1$ is 60 to 80 percent predicted and PEF variability is 20 to 30 percent.

The term "severe asthma" generally refers to asthma in which the patient has almost continuous symptoms, frequent exacerbations, frequent nighttime awakenings due to the asthma, limited activities, PEF or $FEV_1$ baseline less than 60 percent predicted, and PEF variability of 20 to 30 percent.

Examples of rescue medications include albuterol, ventolin and others.

"Resistant" refers to a disease that demonstrates little or no clinically significant improvement after treatment with a therapeutic agent. For example, asthma which requires treatment with high dose ICS (e.g., quadrupling the total daily corticosteroid dose or a total daily dose of greater or equal to 500 micrograms of FP (or equivalent) for at least three consecutive days or more, or systemic corticosteroid for a two week trial to establish if asthma remains uncontrolled or $FEV_1$ does not improve is often considered severe refractory asthma.

A therapeutic agent as disclosed herein can be administered by any suitable means, including parenteral, subcutaneous, intraperitoneal, intrapulmonary, and intranasal. Parenteral infusions include intramuscular, intravenous, intraarterial, intraperitoneal, or subcutaneous administration. In one example, the therapeutic agent is inhaled. According to another example, the dosing is given by injections, e.g., intravenous or subcutaneous injections. In yet another example, the therapeutic agent is administered using a syringe (e.g., prefilled or not) or an autoinjector.

For the prevention or treatment of disease, the appropriate dosage of a therapeutic agent may depend on the type of disease to be treated, the severity and course of the disease, whether the therapeutic agent is administered for preventive or therapeutic purposes, previous therapy, the patient's clinical history and response to the therapeutic agent, and the discretion of the attending physician. The therapeutic agent is suitably administered to the patient at one time or over a series of treatments. The therapeutic agent composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

Dosing for lebrikizumab, for eosinophilic diseases (including asthma) and for treating other diseases using TH2 therapies: lebrikizumab can be administered 0.1 mg/kg to 100 mg/kg of the patient's body weight. In one example, the dosage administered to a patient is between 0.1 mg/kg and 20 mg/kg of the patient's body weight. In another example, the dose is 1 mg/kg to 10 mg/kg of the patient's body weight.

In an alternative example, lebrikizumab can be administered as a flat dose. In one example lebrikizumab is administered as a flat dose (i.e., not weight dependent) of between 125-1000 mg, or a flat dose of 37.5 mg, or a flat dose of 125 mg, or a flat dose of 250 mg, or a flat dose of 500 mg, by subcutaneous injection or by intravenous injection, at a frequency of time selected from the group consisting of: every 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 1month, 2 months, 3month or 4 months. In another example, if the patient is overweight, lebrikizumab can be administered, e.g., 125-250 mg at a frequency of 3 times per month. In one example, the lebrikizumab is administered as a flat dose of 125 mg, 250 mg or 500 mg every 4 weeks. In another example, the lebrikizumab is administered in a patient >40 kg as a flat dose of 37.5 mg, 125 mg, 250 mg or 500 mg every 4 weeks.

In one example, the patient is 18 years of age or older. In one example, the asthma patient is age 12 to 17 and lebrikizumab is administered in as a flat dose of 250 mg or a flat dose of 125 mg. In one example, the asthma patient is age 6 to 11 and lebrikizumab is administered in as a flat dose of 125 mg.

Dosing for quilizumab, for eosinophilic diseases (including asthma) and for treating other diseases using TH2 therapies: quilizumab can be administered 0.003 mg/kg to 100 mg/kg of the patient's body weight. In one example, the dosage administered by intravenous or subcutaneous administration to a patient is between 0.003 mg/kg and 5 mg/kg of the patient's body weight.

In an alternative example, quilizumab can be administered as a flat dose. In one example quilizumab is administered in as a 150-450 mg flat dose (i.e., not weight dependent), by subcutaneous injection or by intravenous injection, at a frequency of time selected from the group consisting of: every 4 weeks or every 12 weeks (e.g., about once every three months or once every quarter). In one example, quilizumab is administered subcutaneously at a dose of 300 mg every 4 weeks. In one example, quilizumab is administered subcutaneously at a dose of 450 mg once every 12 weeks (i.e., about once every three months or once every quarter). In certain examples, an additional subcutaneous dose of 450 mg is administered once at week 4. In one example, quilizumab is administered subcutaneously at a dose of 150 mg once every 12 weeks (i.e., once every quarter). In certain examples, an additional subcutaneous dose of 150 mg is administered once at week 4.

"Patient response" or "response" (and grammatical variations thereof) can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (i.e., reduction, slowing down or complete stopping) of disease cell infiltration into adjacent peripheral organs and/or tissues; (5) inhibition (i.e. reduction, slowing down or complete stopping) of disease spread; (6) decrease of auto-immune response, which may, but does not have to, result in the regression or ablation of the disease lesion; (7) relief, to some extent, of one or more symptoms associated with the disorder; (8) increase in the length of disease-free presentation following treatment; and/or (9) decreased mortality at a given point of time following treatment.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen binding arm). The affinity of a molecule X for its partner Y can generally be represented by the dissociation constant (Kd). Affinity can be measured by common methods known in the art, including those described herein. Specific illustrative and exemplary examples for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-target antibody" and "an antibody that binds to target" refer to an antibody that is capable of binding the target with sufficient affinity such that the antibody is useful as a diagnostic and/or therapeutic agent in targeting the target. In one example, the extent of binding of an anti-target antibody to an unrelated, non-target protein is less than about 10% of the binding of the antibody to target as measured, e.g., by a radioimmunoassay (RIA) or biacore assay. In certain examples, an antibody that binds to a target has a dissociation constant (Kd) of $\leq 1\mu M$, $\leq 100$ nM, $\leq 10$ nM, $\leq 1$ nM, $\leq 0.1$ nM, $\leq 0.01$ nM, or $\leq 0.001$ nM (e.g. 10-8 M or less, e.g. from 10-8 M to 10-13 M, e.g., from 10-9 M to 10-13 M). In certain examples, an anti-target antibody binds to an epitope of a target that is conserved among different species.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. Various methods for carrying out competition assays are well-known in the art.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some examples, the number of amino acid changes are 10 or less, 9 or less, 8 or less, 7 or less, 6 or less, 5 or less, 4 or less, 3 or less, or 2 or less. In some examples, the VL acceptor human framework is identical in sequence to the VL human immunoglobulin framework sequence or human consensus framework sequence.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called $\alpha$, $\delta$, $\epsilon$, $\gamma$, and $\mu$, respectively.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The term includes native sequence Fc regions and variant Fc regions. In one example, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one example, for the VL, the subgroup is subgroup kappa I as in Kabat et al., supra. In one example, for the VH, the subgroup is subgroup III as in Kabat et al., supra.

A "humanized" antibody refers to a chimeric antibody comprising amino acid residues from non-human HVRs and amino acid residues from human FRs. In certain examples, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (L1, L2, L3). HVRs generally comprise amino acid residues from the hypervariable loops and/or from the "complementarity determining regions" (CDRs), the latter typically being of highest sequence variability and/or involved in antigen recognition. An HVR region as used herein comprise any number of residues located within positions 24-36 (for HVRL1), 46-56 (for HVRL2), 89-97 (for HVRL3), 26-35B (for HVRH1), 47-65 (for HVRH2), and 93-102 (for HVRH3).

An "individual" or "patient" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain examples, the individual or patient or subject is a human. In some examples, an "individual" or "patient" or "subject" herein is any single human subject eligible for treatment who is experiencing or has experienced one or more signs, symptoms, or other indicators of asthma or a respiratory condition. Intended to be included as a subject are any subjects involved in clinical research trials not showing any clinical sign of disease, or subjects involved in epidemiological studies, or subjects once used as controls. The subject may have been previously treated with a TH2 pathway inhibitor or another drug, or not so treated. The subject may be naive to a TH2 inhibitor when the treatment herein is started, i.e., the subject may not have been previously treated with, for example, a TH2 inhibitor at "baseline" (i.e., at a set point in time before the administration of a first dose of a TH2 inhibitor in the treatment method herein, such as the day of screening the subject before treatment is commenced). Such "naive" subjects are generally considered to be candidates for treatment with such drug(s).

A "pediatric" individual or patient or subject is a human from birth to 18 years old (or 0 to 18 years old). In some examples, a pediatric individual or patient or subject is from 2 to 6, 2 to 17, 6 to 11, 6 to 18, 6 to 17, 8 to 17, 12 to 17, or 12 to 18 years old.

An "isolated" antibody is one which has been separated from a component of its natural environment. In some examples, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-target antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used according to the methods disclosed herein may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products. The term "package insert" is also used to refer to instructions customarily included in commercial packages of diagnostic products that contain information about the intended use, test principle, preparation and handling of reagents, specimen collection and preparation, calibration of the assay and the assay procedure, performance and precision data such as sensitivity and specificity of the assay.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "target" refers to any native molecule from any vertebrate source, including mammals such as primates (e.g. humans) and rodents (e.g., mice and rats), unless otherwise indicated. The term encompasses "full-length," unprocessed target as well as any form of target that results from processing in the cell. The term also encompasses naturally occurring variants of targets, e.g., splice variants or allelic variants.

The term "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some examples, antibodies are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector" refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

## COMPOSITIONS and METHODS

### Exemplary Antibodies

### Anti-IL13 Antibodies

In one aspect, the disclosure describes isolated antibodies that bind to human IL-13.

In one example, the anti-IL13 antibody comprises a HVR-L1 comprising amino acid sequence SEQ ID NO:14; an HVR-L2 comprising amino acid sequence SEQ ID NO:15; an HVR-L3 comprising amino acid sequence SEQ ID NO:16; an HVR-H1 comprising amino acid sequence SEQ ID NO:11; an HVR-H2 comprising amino acid sequence SEQ ID NO: 12; and an HVR-H3 comprising amino acid sequence SEQ ID NO: 13.

In another example, the antibody comprises the variable region sequences SEQ ID NO:9 and SEQ ID NO:10. In another example, the antibody comprises the variable region sequences SEQ ID NO: 19 and SEQ ID NO: 20. In another example, the antibody comprises the variable region sequences SEQ ID NO: 21 and SEQ ID NO: 20.

In any of the above examples, an anti-IL-13 antibody can be humanized. In one example, an anti-IL-13 antibody comprises HVRs as in any of the above examples, and further comprises an acceptor human framework, e.g. a human immunoglobulin framework or a human consensus framework.

In another aspect, an anti-IL-13 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:9. In certain examples, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-IL-13 antibody comprising that sequence retains the ability to bind to human IL-13. In certain examples, a total of 1 to 10 amino acids have been substituted, altered inserted and/or deleted in SEQ ID NO: 9. In certain examples, substitutions, insertions, or deletions occur in regions outside the HVRs (i.e., in the FRs). Optionally, the anti-IL13 antibody comprises the VH sequence in SEQ ID NO: 9, including post-translational modifications of that sequence. Optionally, the anti-IL13 antibody comprises the VH sequence in SEQ ID NO: 19, including post-translational modifications of that sequence. Optionally, the anti-IL13 antibody comprises the VH sequence in SEQ ID NO: 21, including post-translational modifications of that sequence.

In another aspect, an anti-IL-13 antibody is disclosed, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:10. In certain examples, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-IL-13 antibody comprising that sequence retains the ability to bind to IL-13. In certain examples, a total of 1 to 10 amino acids have been substituted, inserted and/or deleted in SEQ ID NO:10. In certain examples, the substitutions, insertions, or deletions occur in regions outside the HVRs (i.e., in the FRs). Optionally, the anti-IL-13 antibody comprises the VL sequence in SEQ ID NO:10, including post-translational modifications of that sequence. Optionally, the anti-IL-13 antibody comprises the VL sequence in SEQ ID NO: 20, including post-translational modifications of that sequence. Optionally, the anti-IL-13 antibody comprises the VL sequence in SEQ ID NO: 22, including post-translational modifications of that sequence.

In yet another example, the anti-IL-13 antibody comprises a VL region having at least 90%, 91%, 92%, 93%, 94%, 95%, 96%, 97%, 98%, 99%, or 100% sequence identity to the amino acid sequence of SEQ ID NO:10 and a VH region having at least 90%, 91%, 92%, 93%, 94%, 95%, 96%, 97%, 98%, 99%, or 100% sequence identity to the amino acid sequence of SEQ ID NO:9. In yet a further example, the anti-IL-13 antibody comprises a HVR-L1 comprising amino acid sequence SEQ ID NO:14; an HVR-L2 comprising amino acid sequence SEQ ID NO:15; an HVR-L3 comprising amino acid sequence SEQ ID NO: 16; an HVR-H1 comprising amino acid sequence SEQ ID NO:11; an HVR-H2 comprising amino acid sequence SEQ ID NO: 12; and an HVR-H3 comprising amino acid sequence SEQ ID NO: 13.

In another aspect, an anti-IL-13 antibody is disclosed, wherein the antibody comprises a VH as in any of the examples disclosed above, and a VL as in any of the examples disclosed above.

In a further aspect, the disclosure describes an antibody that binds to the same epitope as an anti-IL-13 antibody disclosed herein. For example, in certain examples, an antibody is disclosed that binds to the same epitope as or can by competitively inhibited by an anti-IL-13 antibody comprising a VH sequence of SEQ ID NO:9 and a VL sequence of SEQ ID NO:10.

In a further aspect, an anti-IL-13 antibody according to any of the above example can be a monoclonal antibody, including a chimeric, humanized or human antibody. In one example, an anti-IL13 antibody is an antibody fragment, e.g., a Fv, Fab, Fab', scFv, diabody, or F(ab')2 fragment. In another example, the antibody is a full length antibody, e.g., an intact IgG1 or IgG4 antibody or other antibody class or isotype as defined herein. According to another example, the antibody is a bispecific antibody. In one example, the bispecific antibody comprises the HVRs or comprises the VH and VL regions described above.

In a further aspect, an anti-IL-13 antibody according to any of the above examples may incorporate any of the features, singly or in combination, as described in Sections 1-7 below:

**Anti-M1' Antibodies**

In one aspect, the disclosure describes isolated antibodies that bind to membrane proximal M1' region of surface expressed IgE on human B cells.

In one example, the anti-M1' antibody comprises a HVR-L1 comprising amino acid sequence SEQ ID NO:6; an HVR-L2 comprising amino acid sequence SEQ ID NO:7; an HVR-L3 comprising amino acid sequence SEQ ID NO:8; an HVR-H1 comprising amino acid sequence SEQ ID NO:3; an HVR-H2 comprising amino acid sequence SEQ ID NO:4; and an HVR-H3 comprising amino acid sequence SEQ ID NO:5.

In another example, the antibody comprises the variable region sequences SEQ ID NO:1 and SEQ ID NO:2.

In any of the above examples, an anti-M1' antibody can be humanized. In one example, an anti-M1' antibody comprises HVRs as in any of the above examples, and further comprises an acceptor human framework, e.g. a human immunoglobulin framework or a human consensus framework.

In another aspect, an anti-M1' antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:1. In certain examples, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-M1' antibody comprising that sequence retains the ability to bind to human M1'. In certain examples, a total of 1 to 10 amino acids have been substituted, altered inserted and/or deleted in SEQ ID NO:1. In certain examples, substitutions, insertions, or deletions occur in regions outside the HVRs (i.e., in the FRs). Optionally, the anti-M1' antibody comprises the VH sequence in SEQ ID NO:1, including post-translational modifications of that sequence.

In another aspect, an anti-M1' antibody is disclosed, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:2. In certain examples, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-M1' antibody comprising that sequence retains the ability to bind to M1'. In certain examples, a total of 1 to 10 amino acids have been substituted, inserted and/or deleted in SEQ ID NO:2. In certain examples, the substitutions, insertions, or deletions occur in regions outside the HVRs (i.e., in the FRs). Optionally, the anti-M1' antibody comprises the VL sequence in SEQ ID NO:2, including post-translational modifications of that sequence.

In yet another example, the anti-M1' antibody comprises a VL region having at least 90%, 91%, 92%, 93%, 94%, 95%, 96%, 97%, 98%, 99%, or 100% sequence identity to the amino acid sequence of SEQ ID NO:2 and a VH region having at least 90%, 91%, 92%, 93%, 94%, 95%, 96%, 97%, 98%, 99%, or 100% sequence identity to the amino acid sequence of SEQ ID NO:1. In yet a further example, the anti-M1' antibody comprises a HVR-L1 comprising amino acid sequence SEQ ID NO:6; an HVR-L2 comprising amino acid sequence SEQ ID NO:7; an HVR-L3 comprising amino acid sequence SEQ ID NO:8; an HVR-H1 comprising amino acid sequence SEQ ID NO:3; an HVR-H2 comprising amino acid sequence SEQ ID NO:4; and an HVR-H3 comprising amino acid sequence SEQ ID NO:5.

In another aspect, an anti-M1' antibody is disclosed, wherein the antibody comprises a VH as in any of the examples disclosed above, and a VL as in any of the examples disclosed above.

In a further aspect, the disclosure describes an antibody that binds to the same epitope as an anti-M1' antibody disclosed herein. For example, in certain examples, an antibody is disclosed that binds to the same epitope as or can by competitively inhibited by an anti-M1' antibody comprising a VH sequence of SEQ ID NO:1 and a VL sequence of SEQ ID NO:2.

In a further aspect, an anti-M1' antibody according to any of the above example can be a monoclonal antibody, including a chimeric, humanized or human antibody. In one example, an anti-M1' antibody is an antibody fragment, e.g., a Fv, Fab, Fab', scFv, diabody, or F(ab')2 fragment. In another example, the antibody is a full length antibody, e.g., an intact IgG1 or IgG4 antibody or other antibody class or isotype as defined herein. According to another example, the antibody is a bispecific antibody. In one example, the bispecific antibody comprises the HVRs or comprises the VH and VL regions described above.

In a further aspect, an anti-M1' antibody according to any of the above examples may incorporate any of the features, singly or in combination, as described in Sections 1-7 below:

### 1. Antibody Affinity

In certain examples, an antibody disclosed herein has a dissociation constant (Kd) of $\leq 1\mu$M, $\leq 100$ nM, $\leq 10$ nM, $\leq 1$ nM, $\leq 0.1$ nM, $\leq 0.01$ nM, or $\leq 0.001$ nM (e.g. 10-8 M or less, e.g. from 10-8 M to 10-13 M, e.g., from 10-9 M to 10-13 M).

In one example, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (125I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER® multi-well plates (Thermo Scientific) are coated overnight with 5 μg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [125I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 $\mu$l/well of scintillant (MICROSCINT-20 TM; Packard) is added, and the plates are counted on a TOPCOUNT TM gamma counter (Packard) for ten minutes. Concentrations of each Fab that give less than or equal to 20% of maximal binding are chosen for use in competitive binding assays.

According to another example, Kd is measured using surface plasmon resonance assays using a BIACORE®-2000 or a BIACORE ®-3000 (BIAcore, Inc., Piscataway, NJ) at 25°C with immobilized antigen CM5 chips at -10 response units (RU). Briefly, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 $\mu$g/ml (~0.2 $\mu$M) before injection at a flow rate of 5 $\mu$l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20TM) surfactant (PBST) at 25°C at a flow rate of approximately 25 $\mu$l/min. Association rates (kon) and dissociation rates (koff) are calculated using a simple one-to-one Langmuir binding model (BIACORE ® Evaluation Software version 3.2) by simultaneously fitting the association and dissociation sensorgrams. The equilibrium dissociation constant (Kd) is calculated as the ratio koff/kon. See, e.g., Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25oC of a 20 nM antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO TM spectrophotometer (ThermoSpectronic) with a stirred cuvette.

### 2. Antibody Fragments

In certain examples, an antibody disclosed herein is an antibody fragment. Antibody fragments include, but are not limited to, Fab, Fab', Fab'-SH, F(ab)2, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain examples, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g. E. coli or phage), as described herein.

### 3. Chimeric and Humanized Antibodies

In certain examples, an antibody disclosed herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain examples, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some examples, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci.

13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain examples, an antibody disclosed herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSETM technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain examples, an antibody disclosed herein is a multispecific antibody, e.g. a bispecific antibody. Multi-specific antibodies are monoclonal antibodies that have binding specificities for at least two different sites. In certain examples, one of the binding specificities is for IL-13 and the other is for any other antigen. In certain examples, bispecific antibodies may bind to two different epitopes of IL-13. Bispecific antibodies may also be used to localize cytotoxic agents to cells. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bispecific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to IL-13 as well as another, different antigen (see, US 2008/0069820, for example).

### 7. Antibody Variants

In certain examples, amino acid sequence variants of the antibodies disclosed herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, disclosed that the final construct possesses the desired characteristics, e.g., antigen-binding.

#### Substitution, Insertion, and Deletion Variants

In certain examples, antibody variants having one or more amino acid substitutions are disclosed. Sites of interest for substitutional mutagenesis include the HVRs and FRs. Conservative substitutions are shown in Table 1 under the heading of "conservative substitutions." More substantial changes are disclosed in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

#### TABLE 1

| Original Residue | Exemplary Substitutions | Conservative Substitutions |
|---|---|---|
| Ala (A) | Val; Leu; Ile | Val |
| Arg (R) | Lys; Gln; Asn | Lys |

(continued)

| Original Residue | Exemplary Substitutions | Conservative Substitutions |
|---|---|---|
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions will entail exchanging a member of one of these classes for another class.
One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).
Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some examples of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain examples, substitutions, insertions, or deletions may occur within one or more HVRs so long as such alterations do not substantially reduce the ability of the antibody to bind antigen. For example, conservative alterations (e.g., conservative substitutions as disclosed herein) that do not substantially reduce binding affinity may be made in HVRs. Such alterations may be outside of HVR "hotspots" or SDRs. In certain examples of the variant VH and VL sequences disclosed above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

## Glycosylation variants

In certain examples, an antibody disclosed herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some examples, modifications of the oligosaccharide in an antibody may be made in order to create antibody variants with certain improved properties.

In one example, antibody variants are disclosed having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about $\pm$ 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further disclosed with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also disclosed. Such antibody variants may have improved CDC function. Such antibody variants are described, e.g., in WO 1997/30087 (Patel et al.); WO 1998/58964 (Raju, S.); and WO 1999/22764 (Raju, S.).

**Fc region variants**

In certain examples, one or more amino acid modifications may be introduced into the Fc region of an antibody disclosed herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain examples, an antibody variant is contemplated that possesses some but not all effector functions, which make it a desirable candidate for applications in which the half life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and in vivo clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain examples, an antibody variant comprises an Fc region with one or more amino acid substitutions which improve ADCC, e.g., substitutions at positions 298, 333, and/or 334 of the Fc region (EU numbering of residues).

In some examples, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

## Cysteine engineered antibody variants

In certain examples, it may be desirable to create cysteine engineered antibodies, e.g., "thioMAbs," in which one or more residues of an antibody are substituted with cysteine residues. In particular examples, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain examples, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

## Antibody Derivatives

In certain examples, an antibody disclosed herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another example, conjugates of an antibody and nonproteinaceous moiety that may be selectively heated by exposure to radiation are disclosed. In one example, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

## Recombinant Methods and Compositions

Antibodies may be produced using recombinant methods and compositions, e.g., as described in U.S. Patent No. 4,816,567. In one example, isolated nucleic acid encoding an antibody described herein is disclosed. Such nucleic acid may encode an amino acid sequence comprising the VL and/or an amino acid sequence comprising the VH of the antibody (e.g., the light and/or heavy chains of the antibody). In a further example, one or more vectors (e.g., expression vectors) comprising such nucleic acid are disclosed. In a further example, a host cell comprising such nucleic acid is disclosed. In one such example, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one example, the host cell is eukaryotic, e.g. a Chinese Hamster Ovary (CHO) cell or lymphoid cell (e.g., Y0, NS0, Sp20 cell). In one example, a method of making an antibody is disclosed, wherein the method comprises culturing a host cell comprising a nucleic acid encoding the antibody, as disclosed above, under conditions suitable for expression of the antibody, and optionally recovering the antibody from the host cell (or host cell culture medium).

For recombinant production of an antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US Patent Nos. 5,959,177, 6,040,498, 6,420,548, 7,125,978, and 6,417,429 (describing PLANTIBODIESTM technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR- CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

## Methods and Compositions for Diagnostics and Detection

Herein, the use of specific biomarkers (e.g., one or more of CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2, and combinations thereof) to identify subjects more or less likely to respond to therapeutic treatment with a TH2 pathway inhibitor are described. Thus, the disclosed methods provide convenient, efficient, and potentially cost-effective means to obtain data and information useful in assessing appropriate or effective therapies for treating patients. For example, a sample can be obtained from an asthma patient or a respiratory disorder patient, and the sample could be examined by various *in vitro* assays to measure particular analytes and determine whether the expression level of one or more biomarkers has increased or decreased as compared to the expression level in a reference population. In some examples, if expression levels of at least 1, 2, 3, 4, 5, 6, 7, or more of CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in the sample from the patient is greater than or equal to the expression level in a healthy individual, then the patient is likely to benefit from treatment with a TH2 pathway inhibitor.

Biomarkers, including proteins or nucleic acids, can be detected or measured using methods generally known in the art. Expression levels/amount of a gene or a biomarker can be determined based on any suitable criterion known in the art, including but not limited to mRNA, cDNA, proteins, protein fragments and/or gene copy number. Methods of detection generally encompass methods to quantify the level of a biomarker in the sample (quantitative method) or that determine whether or not a biomarker is present in the sample (qualitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence-based immunoassays, as well as mRNAs or DNAs from a genetic biomarker of interest using Northern, dot-blot, polymerase chain reaction (PCR) analysis, array hybridization, RNase protection assay, or using DNA SNP chip microarrays, which are commercially available, including DNA microarray snapshots. Further suitable methods to detect biomarker include measuring a physical or chemical property specific for the peptide or polypeptide such as its precise molecular mass or NMR spectrum. Said methods comprise, e.g., biosensors, optical devices coupled to immunoassays, biochips, analytical devices such as mass- spectrometers, NMR- analyzers, or chromatography devices. Further, methods include microplate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

In some examples, an mRNA biomarker may be amplified by any method generally known in the art. Amplification generally refers to the production of a plurality of biomarker molecules from a target biomarker, usually a biomarker molecule present in the sample. For example, if the biomarker is a nucleic acid amplifying the nucleic acid refers to the production of a plurality of nucleic acid molecules from a target nucleic acid wherein primers hybridize to specific sites on the target nucleic acid molecules in order to provide an inititation site for extension by, e.g. a polymerase. Amplification can be carried out by any method generally known in the art, such as but not limited to: PCR-based amplification methods, such as standard PCR, long PCR, hot start PCR, qPCR, and RT-PCR; isothermal amplification methods, such as nucleic acid sequence-based amplification (NASBA) and the transcription-mediated amplification (TMA); and hybridization signal amplification methods, such as the branched DNA assay method.

In certain examples, expression/amount of a gene or biomarker in a sample is increased as compared to expression/amount in a reference population if the expression level/amount of the gene or biomarker in the sample is greater than the expression level/amount of the gene or biomarker in reference population. Similarly, expression/amount of a gene or biomarker in a sample is decreased as compared to expression/amount in a reference population if the expression level/amount of the gene or biomarker in the ample is less than the expression level/amount of the gene or biomarker in the reference population.

In certain examples, the samples are normalized for both differences in the amount of RNA or protein assayed and variability in the quality of the RNA or protein samples used, and variability between assay runs. Such normalization may be accomplished by measuring and incorporating the expression of certain normalizing genes, including known housekeeping genes, such as PPIA, ACTB, GAPDH, TFRC, etc. Alternatively, normalization can be based on the mean or median signal of all of the assayed genes or a large subset thereof (global normalization approach). On a gene-by-gene basis, measured normalized amount of a patient tumor mRNA or protein is compared to the amount found in a reference set. Normalized expression levels for each mRNA or protein per tested tumor per patient can be expressed as a percentage of the expression level measured in the reference set. The expression level measured in a particular patient sample to be analyzed will fall at some percentile within this range, which can be determined by methods known in the art.

The expression level of the biomarker(s) of interest and of a housekeeping gene can be measured from a biological sample from an asthma patient or a respiratory disorder patient. The resulting detection data (e.g., Ct data) of the biomarker(s) can be normalized against the detection data for the housekeeping gene resulting in a ΔCt value (ΔCt = Ct(biomarker gene) - Ct (housekeeping gene). The mean value of the ΔCt values of the biomarker(s) tested can be calculated (e.g., triplicate ΔCt values for three biomarkers are added and divided by 9). The expression levels of the same biomarker(s) of interest from a biological sample from two or more healthy persons can be detected using the same methods, and the mean value and standard deviation for the healthy persons data can be calculated. Alternatively, if substitute values (e.g., a control(s)) has been developed for the same method, then those values can be used in place of testing healthy persons.

In some examples, the mean Ct or ΔCt value of the asthma patient or respiratory disorder patient can be compared against the median or mean Ct or ΔCt value of the healthy persons as follows: (1) a threshold value can be set, wherein above the threshold value, the patient would be considered to be EIP and below the threshold value, the patient can be considered to be EIN; (2) in some examples, the threshold value is the median Ct or ΔCt value of healthy persons (or a control). In some examples, the mean Ct or ΔCt value of the asthma patient or respiratory disorder patient can be compared against the median or mean Ct or ΔCt value of the healthy persons as follows: (1) a threshold value can be set, wherein above the threshold value, the patient would be considered to be EIP and below the threshold value, the patient can be considered to be EIN; (2) in some examples, the threshold value is 1.5 times the value of the mean Ct or ΔCt value of healthy persons (or a control) or two standard deviations above the mean Ct or ΔCt value of healthy persons (or control(s)).

Ct is the threshold cycle. The Ct is the cycle number at which the fluorescence generated within a reaction crosses a predefined threshold line.

In some examples, experiments are normalized to a reference RNA, which is a comprehensive mix of RNA from various tissue sources (e.g., reference RNA #636538 from Clontech, Mountain View, CA). In another example, the reference RNA is transferrin receptor (TFRC). In some examples, the same reference RNA is included in each qRT-PCR run, allowing comparison of results between different experimental runs.

A biological sample comprising a target gene or biomarker can be obtained by methods known in the art. In addition, the progress of therapy can be monitored more easily by testing such body samples for target genes or gene products.

For the detection of biomarker proteins a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker. In certain examples, the expression of proteins in a sample is examined using immunohistochemistry ("IHC") and staining protocols. Immunohistochemical staining of tissue sections has been shown to be a reliable method of assessing or detecting presence of proteins in a sample. Immunohistochemistry techniques utilize an antibody to probe and visualize cellular antigens *in situ*, generally by chromogenic or fluorescent methods.

Two general methods are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody typically will be labeled with a detectable moiety. Numerous labels are available which can be generally grouped into the following categories:

(a) Radioisotopes, such as $^{35}$S, $^{14}$C, $^{125}$I, $^{3}$H, and $^{131}$I. The antibody can be labeled with the radioisotope using the techniques described in Current Protocols in Immunology, Volumes 1 and 2, Coligen et al., Ed. Wiley-Interscience, New York, New York, Pubs. (1991) for example and radioactivity can be measured using scintillation counting.
(b) Colloidal gold particles.
(c) Fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above. The fluorescent labels can be conjugated to the antibody using the techniques disclosed in *Current Protocols in Immunology, supra,* for example. Fluorescence can be quantified using a fluorimeter.
(d) Various enzyme-substrate labels are available and U.S. Patent No. 4,275,149 provides a review of some of these. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e.g.*, glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed. J. Langone & H. Van Vunakis), Academic press, New York, 73:147-166 (1981).

Examples of enzyme-substrate combinations include, for example:

(i) Horseradish peroxidase (HRPO) with hydrogen peroxidase as a substrate, wherein the hydrogen peroxidase oxidizes a dye precursor (*e.g.*, orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (*e.g.*, p-nitrophenyl-β-D-galactosidase) or fluorogenic substrate (*e.g.*, 4-methylumbelliferyl-β-D-galactosidase).

Numerous other enzyme-substrate combinations are available to those skilled in the art. For a general review of these, see U.S. Patent Nos. 4,275,149 and 4,318,980. Sometimes, the label is indirectly conjugated with the antibody. The skilled artisan will be aware of various techniques for achieving this. For example, the antibody can be conjugated with biotin and any of the four broad categories of labels mentioned above can be conjugated with avidin, or *vice versa.* Biotin binds selectively to avidin and thus, the label can be conjugated with the antibody in this indirect manner. Alternatively, to achieve indirect conjugation of the label with the antibody, the antibody is conjugated with a small hapten and one of the different types of labels mentioned above is conjugated with an anti-hapten antibody. Thus, indirect conjugation of the label with the antibody can be achieved.

Following an optional blocking step, the sample is exposed to primary antibody for a sufficient period of time and under suitable conditions such that the primary antibody binds to the target protein antigen in the sample. Appropriate conditions for achieving this can be determined by routine experimentation. The extent of binding of antibody to the sample is determined by using any one of the detectable labels discussed above. In certain examples, the label is an enzymatic label (*e.g.* HRPO) which catalyzes a chemical alteration of the chromogenic substrate such as 3,3'-diaminobenzidine chromogen. In one example, the enzymatic label is conjugated to antibody which binds specifically to the primary antibody (*e.g.* the primary antibody is rabbit polyclonal antibody and secondary antibody is goat anti-rabbit antibody).

In some examples, the sample may be contacted with an antibody specific for said biomarker under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available, see, e.g., U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used assays. A number of variations of the sandwich assay technique exist. Briefly, in a typical forward assay, an unlabeled antibody is immobilized on a solid substrate, and the sample to be tested brought into contact with the bound molecule. After a suitable period of incubation, for a period of time sufficient to allow formation of an antibody-antigen complex, a second antibody specific to the antigen, labeled with a reporter molecule capable of producing a detectable signal is then added and incubated, allowing time sufficient for the formation of another complex of antibody-antigen-labeled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labeled antibody are added simultaneously to the bound antibody. These techniques are known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to 40°C such as between 25° C and 32° C inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

An alternative method involves immobilizing the target biomarkers in the sample and then exposing the immobilized target to specific antibody which may or may not be labeled with a reporter molecule. Depending on the amount of target and the strength of the reporter molecule signal, a bound target may be detectable by direct labelling with the antibody. Alternatively, a second labeled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, -galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. As in the EIA, the fluorescent labeled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed.

Examples of methods further include protocols which examine the presence and/or expression of mRNAs of the at least 1, 2, 3, 4, 5, 6, 7 or more of CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 and combinations thereof in a sample. Methods for the evaluation of mRNAs in cells are known and include, for example, hybridization assays using complementary DNA probes (such as *in situ* hybridization using labeled riboprobes specific for the one or more genes, Northern blot and related techniques) and various nucleic acid amplification assays (such as RT-PCR using complementary primers specific for one or more of the genes, and other amplification type detection methods, such as, for example, branched DNA, SISBA, TMA and the like).

Tissue or other samples from mammals can be conveniently assayed for mRNAs using Northern, dot blot or PCR analysis. For example, RT-PCR assays such as quantitative PCR (qPCR) assays are known in the art. In some examples, the qPCR is performed on a Roche Cobas® system. In an illustrative example, a method for detecting a target mRNA in a biological sample comprises producing cDNA from the sample by reverse transcription using at least one primer; amplifying the cDNA so produced using a target polynucleotide as sense and antisense primers to amplify target cDNAs therein. In addition, such methods can include one or more steps that allow one to determine the levels of target mRNA in a biological sample (*e.g.*, by simultaneously examining the levels of a comparative control mRNA sequence of a "housekeeping" gene such as an actin family member or GAPDH). Optionally, the sequence of the amplified target cDNA can be determined.

Optional methods of the invention include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes whose expression correlate with increased or reduced clinical benefit of anti-angiogenic therapy may be arrayed on a solid support. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment, (*see, e.g.*, WO 01/75166 published October 11, 2001; (*see*, for example, U.S. 5,700,637, U.S. Patent 5,445,934, and U.S. Patent 5,807,522, Lockart, Nature Biotechnology, 14:1675-1680 (1996); Cheung, V.G. et al., Nature Genetics 21(Suppl):15-19 (1999) for a discussion of array fabrication). DNA microarrays are miniature arrays containing gene fragments that are either synthesized directly onto or spotted onto glass or other substrates. Thousands of genes are usually represented in a single array. A typical microarray experiment involves the following steps: 1) preparation of fluorescently labeled target from RNA isolated from the sample, 2) hybridization of the labeled target to the microarray, 3) washing, staining, and scanning of the array, 4) analysis of the scanned image and 5) generation of gene expression profiles. Currently two main types of DNA microarrays are being used: oligonucleotide (usually 25 to 70 mers) arrays and gene expression arrays containing PCR products prepared from cDNAs. In forming an array, oligonucleotides can be either prefabricated and spotted to the surface or directly synthesized on to the surface (in situ).

The Affymetrix GeneChip® system is a commercially available microarray system which comprises arrays fabricated by direct synthesis of oligonucleotides on a glass surface. Probe/Gene Arrays: Oligonucleotides, usually 25 mers, are directly synthesized onto a glass wafer by a combination of semiconductor-based photolithography and solid phase chemical synthesis technologies. Each array contains up to 400,000 different oligos and each oligo is present in millions of copies. Since oligonucleotide probes are synthesized in known locations on the array, the hybridization patterns and signal intensities can be interpreted in terms of gene identity and relative expression levels by the Affymetrix Microarray Suite software. Each gene is represented on the array by a series of different oligonucleotide probes. Each probe pair consists of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, disturbing the binding of the target gene transcript. This helps to determine the background and nonspecific hybridization that contributes to the signal measured for the perfect match oligo. The Microarray Suite software subtracts the hybridization intensities of the mismatch probes from those of the perfect match probes to determine the absolute or specific intensity value for each probe set. Probes are chosen based on current information from Genbank and other nucleotide repositories. The sequences are believed to recognize unique regions of the 3' end of the gene. A GeneChip Hybridization Oven ("rotisserie" oven) is used to carry out the hybridization of up to 64 arrays at one time. The fluidics station performs washing and staining of the probe arrays. It is completely automated and contains four modules, with each module holding one probe array. Each module is controlled independently through Microarray Suite software using preprogrammed fluidics protocols. The scanner is a confocal laser fluorescence scanner which measures fluorescence intensity emitted by the labeled cRNA bound to the probe arrays. The computer workstation with Microarray Suite software controls the fluidics station and the scanner. Microarray Suite software can control up to eight fluidics stations using preprogrammed hybridization, wash, and stain protocols for the probe array. The software also acquires and converts hybridization intensity data into a presence/absence call for each gene using appropriate algorithms. Finally, the software detects changes in gene expression between experiments by comparison analysis and formats the output into .txt files, which can be used with other software programs for further data analysis.

Expression of a selected gene or biomarker in a tissue or cell sample may also be examined by way of functional or activity-based assays. For instance, if the biomarker is an enzyme, one may conduct assays known in the art to determine or detect the presence of the given enzymatic activity in the tissue or cell sample.

The eosinophilic inflammation status (e.g., EIP or EIN) of a patient based on the test results may be disclosed in a report. The report may be in any form of written materials (e.g., in paper or digital form, or on internet) or oral presentation(s) (e.g., either in person (live) or as recorded). The report may further indicate to a health professional (e.g., a physician) that the patient may benefit from or is likely to respond to an interferon inhibitor treatment.

In certain examples, a kit comprises a container, a label on said container, and a composition contained within said container; wherein the composition includes one or more primary antibodies that bind to one or more target polypeptide sequences corresponding to one or more biomarkers, the label on the container indicating that the composition can be used to evaluate the presence of one or more target proteins in at least one type of mammalian cell, and instructions for using the antibodies for evaluating the presence of one or more target proteins in at least one type of mammalian cell. The kit can further comprise a set of instructions and materials for preparing a tissue sample and applying antibody and probe to the same section of a tissue sample. The kit may include both a primary and secondary antibody, wherein the secondary antibody is conjugated to a label, *e.g.*, an enzymatic label.

The term "detecting" encompasses quantitative or qualitative detection. In certain examples, a biological sample comprises a cell or tissue, such as serum, plasma, nasal swabs and sputum.

## Pharmaceutical Formulations

Pharmaceutical formulations of an anti-IL-13 antibody or other TH2 pathway inhibitors as described herein are prepared by mixing such antibody or molecule having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide a controller with the TH2 pathway inhibitor. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for in vivo administration are generally sterile. Sterility may be readily accomplished, e.g., by filtration through sterile filtration membranes.

## Therapeutic Methods and Compositions

Eosinophilic inflammation is associated with a variety of illnesses, both allergic and non-allergic (Gonlugur (2006) Immunol. Invest. 35(1):29-45). Inflammation is a restorative response of living tissues to injury. A characteristic of inflammatory reactions is the accumulation of leukocytes in injured tissue due to certain chemicals produced in the tissue itself. Eosinophil leukocytes accumulate in a wide variety of conditions such as allergic disorders, helminthic infections, and neoplastic diseases (Kudlacz et al., (2002) Inflammation 26: 111-119). Eosinophil leukocytes, a component of the immune system, are defensive elements of mucosal surfaces. They respond not only to antigens but to parasites, chemicals, and trauma.

Tissue eosinophilia occurs in skin diseases such as eczema, pemphigus, acute urticaria, and toxic epidermal necrolysis as well as in atopic dermatitis (Rzany et al., Br. J. Dermatol. 135: 6-11 (1996)). Eosinophils accumulate in the tissue and empty granule proteins in IgE-mediated allergic skin reactions (Nielsen et al., Ann. Allergy Asthma Immunol., 85: 489-494 (2001)). Eosinophils combined with mast cells are likely to cause joint inflammation (Miossec, J. Clin. Rheumatol. 3: 81-83 (1997)). Eosinophilic inflammation sometimes accompanies joint trauma. Synovial fluid eosinophilia can be associated with diseases such as rheumatoid arthritis, parasitic disease, hypereosinophilic syndrome, Lyme disease, and allergic processes, as well as hemarthrosis and arthrography (Atanes et al., Scand. J. Rheumatol., 25: 183-185 (1996)). Eosinophilic inflammation can affect bones as well (Yetiser et al., Int. J. Pediatr. Otorhinolaryngol., 62: 169-173 (2002)). Examples of eosinophilic muscle disease include eosinophilic perimyositis, eosinophilic polymyositis, and focal eosinophilic myositis (Lakhanpal et al., Semin. Arthritis Rheum., 17: 331-231 (1988)). Eosinophilic inflammations affecting skeletal muscles may be associated with parasite infections or drugs or features of some systemic disorders of hypereosinophilia (e.g., idiopathic hypereosinophilic syndrome and eosinophilia-myalgia syndrome. Eosinophils participate in the inflammatory response to epitopes recognized by autoimmune antibodies (Engineer et al., Cytokine, 13: 32-38 (2001)). Connective tissue diseases may lead to neutrophilic, eosinophilic, or lymphocytic vascular inflammations (Chen et al., J. Am. Acad. Dermatol., 35: 173-182 (1996)). Tissue and peripheral blood eosinophilia can occur in active rheumatismal diseases. Elevation of serum ECP levels in ankylosing spondylitis, a kind of connective tissue disease, suggests that eosinophils are also involved in the underlying process (Feltelius et al., Ann. Rheum. Dis., 46: 403-407 (1987)). Wegener's granulomatosis can rarely present with pulmonary nodules, pleural effusion, and peripheral blood eosinophilia (Krupsky et al., Chest, 104: 1290-1292 (1993)).

Peripheral blood eosinophilia of at least 400/mm3 can occur in 7% of cases of systemic sclerosis, 31% of cases of localized scleroderma, and 61% of cases of eosinophilic fasciitis (Falanga, et al., J. Am. Acad. Dermatol., 17: 648-656 (1987)). Scleroderma yields an inflammatory process closely resembling Meissner's and Auerbach's plexuses and consists of mast cells and eosinophil leukocytes in the gastrointestinal system. Eosinophil-derived neurotoxins can contribute to gastrointestinal motor dysfunction, as occurs in scleroderma (DeSchryver-Kecskemeti, et al. Arch. Pathol. Lab Med., 113: 394-398 (1989)).

Eosinophils can accompany localized (Varga, et al., Curr. Opin. Rheumatol., 9: 562-570 (1997)) or systemic (Bouros et al., Am. J. Respir. Crit. Care Med., 165: 1581-1586 (2002)) connective tissue proliferation. They can incite fibrosis by inhibiting proteoglycan degradation in fibroblasts (Hernnas et al., Eur. J. Cell Biol., 59: 352-363 (1992)), and fibroblasts mediate eosinophil survival by secreting GM-CSF (Vancheri et al., Am. J. Respir. Cell Mol. Biol., 1: 289-214 (1989)). Eosinophils can be found in nasal (Bacherct et al., J. allergy Clin. Immunol., 107: 607-614 (2001)), bronchial (Arguelles, et al., Arch. Intern. Med., 143: 570-571 (1983)), and gastrointestinal polyp tissues (Assarian, et al., Hum. Pathol., 16: 311-312 (1985)). Likewise, eosinophils can be localized in inflammatory pseudotumors (myofibroblastic tumor). Eosinophils often accompany inflammatory pseudotumors in the orbital region, in which case the condition can mimic angioedema or allergic rhinoconjunctivitis (Li et al., Ann. Allergy, 69: 101-105 (1992)).

Eosinophilic inflammation can be found in tissue trauma (e.g., as a result of surgery or injury). Eosinophilic inflammation can also be associated with cardiovascular illnesses (e.g., eosinophilic myocarditis, eosinophilic coronary arteritis, ischemic heart disease, acute myocardial infarction, cardiac rupture). Necrotic inflammatory processes can also involve eosinophililic inflammation (polymyositis, coronary artery dissection, necrotizing lesions of neuro-Behcet's disease, dementia, cerebral infarction).

Among noninvasive biomarkers of the Th2-driven/eosinophilic asthma subphenotype are serum periostin, fractional exhaled nitric oxide (FeNO), and peripheral blood eosinophil count. See Arron et al. (2013) Adv Pharmacol 66: 1-49. Of these markers, serum periostin has been advanced as a predictive diagnostic for lebrikizumab because it was the best single predictor of airway eosinophil status (as determined by a composite of sputum and tissue eosinophilia) in the BOBCAT observation study of severe asthma (Jia et al. (2012) J Allergy Clin Immunol 130: 647-654 e10), it exhibited substantially less intra-patient variability than FeNO or blood eosinophils across two pre-dose visits in the MILLY study (Corren et al. (2011) N Engl J Med 365: 1088-98), and can be available on a standardized, broadly available assay platform that requires neither a specialized point-of-care instrument (such as FeNO), nor is dependent on automated cell counters that are not broadly standardized across existing clinical laboratories (such as blood eosinophils). While serum periostin appears to be a robust and consistent biomarker for the Th2/eosinophilic subtype of adult asthma, whether it can be applied to pediatric asthma was unknown.

In the MILLY study, adults with poorly controlled asthma despite ICS who had serum periostin levels above 50 ng/ml at baseline exhibited a mean 14.4% reduction in serum periostin after 12 weeks of lebrikizumab treatment (p=0.001), while patients with baseline serum periostin levels below 50 ng/ml exhibited a non-significant 2.9% reduction in serum periostin during the treatment period (p=0.3). *See* Scheerens et al. (2012) Am J Respir Crit Care Med 185: A3960. The distribution of serum periostin levels in asthma patients after 12 weeks of lebrikizumab treatment overlapped with the distribution of serum periostin levels in healthy control adults (Arron et al, Annals Am. Thoracic Soc., in press (2013),, DOI: 10.1513/AnnalsATS.201303-047AW). These results suggest that, in adult asthmatic patients with high serum periostin, the excess periostin above background levels is due to the activity of IL13 in the airways, and this excess constitutes about 10-15% of total systemic periostin.

Periostin was initially identified as a product of osteoblasts, the cells that lay down bone matrix. *See* Horiuchi et al. (1999) J Bone Miner Res 14: 1239-49. Anatomically, periostin expression in bone is localized to sites of endochondral and intramembranous ossification during development, suggesting that periostin expression levels may be correlated with the rate of bone growth. In juvenile mice, systemic periostin levels and markers of bone turnover are elevated, decreasing as animals mature and attaining relatively stable levels from the age of 8 weeks throughout adulthood. *See* Contie et al. (2010) Calcif Tissue Int 87: 341-5. In humans, while asthma in the pediatric population is more commonly associated with atopy and type 2 inflammation than in adults, there remains evidence for eosinophilic and non-eosinophilic airway inflammatory subsets in asthmatic children. *See* Baraldo et al. (2011) Eur Respir J 38: 575-83. Hence biomarkers that identify asthmatic children with increased Th2/eosinophilic airway inflammation may be useful to enable patient selection to demonstrate clinical benefit from anti-IL13 and other therapeutics targeting type 2 inflammation.

It was found that periostin levels are elevated in pediatric subjects younger than 18 years old but exhibit no age dependence in asthma patients older than the age of 18. To identify biomarkers related to eosinophilic airway inflammation but unlikely to be confounded by bone growth, genome-wide expression analyses of a large cohort of moderate-severe asthmatics to identify transcripts correlated with peripheral blood eosinophil counts were conducted. A subset of those transcripts as biomarkers predictive of enhanced clinical benefit from lebrikizumab in the MILLY study were then validated. It was subsequently verified that a subset of peripheral blood transcripts predictive of enhanced clinical benefit from lebrikizumab in adult asthma patients exhibit similar correlations to blood eosinophil percentage and minimal age dependence in adult and pediatric asthma patients.

Disclosed herein are methods of identifying Eosinophilic Inflammation Positive (EIP) patients predictive for a response to treatment with a TH2 pathway inhibitor (or that will be responsive to) by measuring levels of at least one eosinophilic inflammation marker in a biological sample from a patient, wherein one or more of the markers is selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 in a sample from the patient.

Also disclosed herein are methods of treating asthma, an Eosinophilic Disorder, an IL-13 mediated Disorder, an IL4 mediated Disorder, an IL9 mediated Disorder, an IL5 mediated Disorder, an IL33 mediated Disorder, an IL25 mediated Disorder, an TSLP mediated Disorder, an IgE-mediated Disorder or Asthma-Like Symptoms comprising administering a TH2 pathway inhibitor to an Eosinophilic Inflammation Positive Patient, wherein the patient was diagnosed as being EIP using an EID Assay.

In certain examples, methods of treating asthma, an Eosinophilic Disorder, an IL-13 mediated Disorder, IL-4 mediated Disorder or an IgE-mediated Disorder comprising administering lebrikizumab to a Eosinophilic Inflammation Positive Patient are disclosed.

In certain examples, methods of treating asthma, an Eosinophilic Disorder, an IL-13 mediated Disorder, IL-4 mediated Disorder or an IgE-mediated Disorder comprising administering a 125-500mg flat dose of lebrikizumab every 4 weeks to the patient suffering from the disorder are disclosed.

Also disclosed are methods of treating asthma (or Respiratory Disease) comprising administering a therapeutically effective amount of Lebrikizumab to the asthma patient, wherein the treatment results in a relative change in $FEV_1$ of greater than 5%. In another example, the $FEV_1$ is greater than 6%, 7%, 8%, 9% or 10% $FEV_1$. In another example, the patient has been diagnosed as EIP using an EID Assay.

In certain examples, methods of treating asthma (or Respiratory Disease) comprising administering a therapeutically effective amount of Lebrikizumab to the asthma patient, wherein the treatment results in a reduction in exacerbation rate of greater than 35%. (other examples greater than 36%, 37%, 38%, 39%, 40%, 41%, 42%, 43%, 44%, 45%, 46%, 47%, 48%, 49%, 50%, 51%, up to 85%; another example, wherein the patient has been diagnosed as EIP) are disclosed.

In certain examples, methods of treating asthma (or Respiratory Disease) comprising administering a therapeutically effective amount of Lebrikizumab to the asthma patient, wherein the treatment results in a reduction in nocturnal awakenings are disclosed. In one example, the patient is diagnosed using an EID Assay. In another example, the asthma of the patient is uncontrolled on a corticosteroid. I another example, the patient is diagnosed with EIP.

Also disclosed are methods of treating asthma (or Respiratory Disease) comprising administering a therapeutically effective amount of Lebrikizumab to the asthma patient, wherein the treatment results in an improvement in asthma control. In one example, the patient is diagnosed using an EID Assay. In another example, the asthma is uncontrolled on a corticosteroid treatment. In another example, the patient is diagnosed with EIP

Methods of treating Asthma (or Respiratory Disease) comprising administering a therapeutically effective amount of Lebrikizumab to the asthma patient, wherein the treatment results in a reduction of inflammation in the lungs are disclosed. In one example, the patient is diagnosed using an EID Assay. In another example, the asthma is uncontrollable on a corticosteroid treatment. In another example, the patient is diagnosed with EIP

In certain examples, methods of treating an Eosinophilic Disorder in a patient suffering from the Eosinophilic Disorder and being treated with a corticosteroid comprising administering a therapeutically effective amount of Lebrikizumab to the asthma patient, wherein the treatment results in a reduction or elimination of corticosteroid treatment (amount or frequency) used to treat the disease are disclosed. In one example, the patient is diagnosed using an EID Assay. In another example, the patient's asthma is uncontrollable on a corticosteroid. In another example, the patient is diagnosed with EIP prior to the treatment.

Also disclosed are methods of treating of a patient suffering from asthma (or Respiratory Disease) comprising diagnosing the patient as EIP using an EID Assay, administering a therapeutically effective amount of TH2 pathway inhibitor to the asthma patient, diagnosing the patients EIP status, and retreating the patient with the TH2 pathway inhibitor if the status is EIP. The diagnosis may be made using an EID Assay alone or in combination with $FE_{NO}$ levels. In some examples, the patient to be treated has a $FE_{NO}$ level greater than 21 ppb. In some examples, the patient to be treated has a $FE_{NO}$ level greater than 35 ppb.

In some examples, methods of identifying patients that are Eosinophilic Inflammation Negative (EIN) using an EID Assay and determing that the patient is EIN are disclosed. In some examples, the method comprises measuring levels of at least one eosinophilic inflammation marker in a biological sample from a patient, wherein at least one, at least two, or at least three of the markers are selected from CSF1, MEIS2, LGALS12, IDOI, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2. In some examples, at least one, at least two, or at least three of the markers are selected from CSF1, MEIS2, CCL23, HSD3B7, SORD, CACNG6, MGAT3, SLC47A1, and ABTB2. In some examples, at least one, at least two, or at least three of the markers are selected from MEIS2, LGALS12, IDOI, ALOX15, SIGLEC8, CCL23, PYROXD2, HSD3B7, CACNG6, and GPR44. In some examples, at least one, at least two, or at least three of the markers are selected from CCL23, IDOI, HSD3B7, and CACNG6. In some examples, at least one, at least two, or three of the markers are selected from CCL23, IDOI, and CACNG6. In some examples, at least one, at least two, or three of the markers are selected from HSD3B7, SIGLEC8, and GPR44. In some examples, at least one, at least two, at least three, or four of the markers are selected from SIGLEC8, CCL23, CACNG6, and GPR44.

Any of the TH2 pathway inhibitors disclosed herein may be used in therapeutic methods described herein, especially asthma. In one example, the asthma patient is being treated with a corticosteroid, and has been diagnosed as responsive a TH2 pathway inhibitor using an EID Assay described herein. In a further example, the asthma patient is suffering from moderate to severe asthma. In another example, the patient is suffering from mild asthma but is not being treated with a corticosteroid.

An antibody (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 $\mu$g/kg to 15 mg/kg (e.g. 0.1mg/kg-10mg/kg) of antibody can be an initial candidate dosage for administration to the patient, whether, for example, by one or more separate administrations, or by continuous infusion. One typical daily dosage might range from about 1 $\mu$g/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). However, other dosage regimens may be useful. The progress of this therapy is easily monitored by conventional techniques and assays.

In certain examples, an antibody is administered as a flat dose (i.e., not weight dependent) of 37.5 mg, or a flat dose of 125 mg, or a flat dose of 250 mg. In certain examples, the dose is administered by subcutaneous injection once every 4 weeks for a period of time. In certain examples, the period of time is 6 months, one year, two years, five years, ten years, 15 years, 20 years, or the lifetime of the patient. In certain examples, the asthma is severe asthma and the patient is inadequately controlled or uncontrolled on inhaled corticosteroids plus a second controller medication. In another example, the patient is diagnosed with EIP status using an EID Assay to determine EIP status and the patient is selected for treatment with an anti-IL13 antibody as described above. In another example, the method comprises treating an asthma patient with an anti-IL13 antibody as described above where the patient was previously diagnosed with EIP status using an EID Assay to determine EIP status. In one example, the asthma patient is age 18 or older. In one example, the asthma patient is age 12 to 17 and the anti-IL13 is administered in as a flat dose of 250 mg or a flat dose of 125 mg. In one example, the asthma patient is age 6 to 11 and the anti-IL13 antibody is administered in as a flat dose of 125 mg.

It is understood that any of the above formulations or therapeutic methods may be carried out using an immunoconjugate in place of or in addition to an anti-target antibody.

## Articles of Manufacture

In another aspect, an article of manufacture containing materials useful for the treatment, prevention and/or diagnosis of the disorders described above is disclosed. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody. The label or package insert indicates that the composition is used for treating the condition of choice. Moreover, the article of manufacture may comprise (a) a first container with a composition contained therein, wherein the composition comprises an antibody; and (b) a second container with a composition contained therein, wherein the composition comprises a further cytotoxic or otherwise therapeutic agent. The article of manufacture in this example may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate in place of or in addition to an anti-target antibody.

## EXAMPLES

EXAMPLE 1 - Methods

Blood was collected in PAXgene RNA tubes (PreAnalytiX); total RNA was extracted using commercially available kits according to manufacturer instructions (Qiagen).

Expression of candidate biomarker genes in blood samples from the EXTRA study was assessed by Human Genome U133 Plus 2.0 arrays (Affymetrix Inc., Santa Clara, CA). The EXTRA study is described, e.g., in Hanania, et al., Am. J. RCCM, 187: 804-811 (2013); and Hanania et al., Ann. Intern. Med., 154: 573-582 (2011). Microarray hybridization was performed by Asuragen Inc. (Austin, TX). Raw CEL file data was summarized and normalized using Robust Multi-array Averaging (RMA) and analyzed using R and Bioconductor.

Expression of candidate biomarker genes in MILLY, BOBCAT, and GALA II blood samples was quantified by Fluidigm qPCR assays.

qPCR assay selection

Gene expression was measured by qPCR with specific primers and probes. The selected assays with FAM reporters were purchased from Applied Biosystems Inc (ABI) TaqMan® Gene Expression Assays with the following two guidelines:

1. The primers should span at least two different exons if possible to prevent any PCR amplification from any possible residual genomic DNA;
2. The assay has been verified for the robustness and recommended by the company.

The assays ID and ABI supplied context sequences of the genes included are listed in Table 2.

Table 2: Potential biomarker gene assays

| | Gene Symbol | Assay ID | Context Sequence | SEQ ID NO |
|---|---|---|---|---|
| 1 | ABTB2 | Hs00377559_m1 | AAGAACGCCAATGGTGTCCTCTCCC | 23 |
| 2 | ACOT11 | Hs00374982_m1 | CACACCATTAGTGTTGGACAAGTGG | 24 |
| 3 | ALOX15 | Hs00609608_m1 | CCTATCTTCAAGCTTATAATTCCCC | 25 |
| 4 | ASB2 | Hs00387867_m1 | TTAGCCAAGTACGGTGCTGACATCA | 26 |
| 5 | BACE2 | Hs00273238_m1 | CACTTGCCAAGCCATCAAGTTCTCT | 27 |
| 6 | CACNG6 | Hs00230428_m1 | GGAGCTGCCCGGAGAAGCAAACTGC | 28 |
| 7 | CCL23 | Hs00270756_m1 | GGTGTCATCTTCCTCACCAAGAAGG | 29 |
| 8 | CCR3 | Hs00266213_s1 | TGTGCCCCCGCTGTACTCCCTGGTG | 30 |
| 9 | CD9 | Hs01124022_m1 | TCTACACAGGAGTCTATATTCTGAT | 31 |
| 10 | CLC | Hs00171342_m1 | CCTGTTTCTTGAATGAACCATATCT | 32 |
| 11 | CSF1 | Hs00174164_m1 | AGCATGACAAGGCCTGCGTCCGAAC | 33 |
| 12 | CYP4F12 | Hs02515808_s1 | AGCGGCGTCGCACCCTCCCCACTCA | 34 |
| 13 | CYSLTR1 | Hs00272624_s1 | TCAACGTACCATTCACCTTCATTTT | 35 |
| 14 | CYSLTR2 | Hs00252658_s1 | TTAGTTGACCTTGCTGCAGTTCTCC | 36 |
| 15 | DACH1 | Hs00974297_m1 | AAAGAATAGAGCCATAGTTCAAAAG | 37 |
| 16 | FAM124B | Hs01902988_s1 | CGGGAATGTCAGTGGACCCCAAAGA | 38 |
| 17 | GPR44 | Hs00173717_m1 | TCTGTGCCCAGAGCCCCACGATGTC | 39 |
| 18 | HRH4 | Hs00222094_m1 | ACTTCTTTGTGGGTGTGATCTCCAT | 40 |
| 19 | HSD3B7 | Hs00986913_g1 | AGGAGCTGAAGACAGGGCCTGTGAG | 41 |
| 20 | IDO1 | Hs00984148_m1 | TTCTGCAATCAAAGTAATTCCTACT | 42 |
| 21 | IL1RL1 | Hs00545033_ml1 | ATTGTCAGAAGTCCCACATTCAATA | 43 |
| 22 | IL5RA | Hs00602482_m1 | AAGGAATGGATATTTGCAGATAGAA | 44 |
| 23 | KBTBD11 | Hs00362847_m1 | CGCGGCGAGGAACAAGAGTGTGGTG | 45 |
| 24 | KIAA1024 | Hs00324407_m1 | TCTAAAGAGGCAGACAGGCAGTACG | 46 |
| 25 | LGALS12 | Hs01041389_m1 | GAATGAGGAAGTGAAGGTGAGTGTG | 47 |
| 26 | LRRC17 | Hs00180581_m1 | CAAGTTCAGCCTAGGGACTCCACGT | 48 |
| 27 | MEIS2 | Hs00542638_m1 | AAATCTCGCTGACCATAACCCTTCT | 49 |

(continued)

| | Gene Symbol | Assay ID | Context Sequence | SEQ ID NO |
|---|---|---|---|---|
| 28 | MGAT3 | Hs02379589_s1 | CCGTGTGGGAGACCGGCCTGCCAGG | 50 |
| 29 | MYB | Hs00920554_m1 | AAGACCCCGGCACAGCATATATAGC | 51 |
| 30 | OLIG1 | Hs00744293_s1 | CCCGCACCTGGTCCCGGCCAGCCTG | 52 |
| 31 | OLIG2 | Hs00377820_m1 | TCAAATCGCATCCAGATTTTCGGGT | 53 |
| 32 | P2RY14 | Hs00961463_m1 | AAAAATCTTAAAAGGCCTCTGCCTT | 54 |
| 33 | PMP22 | Hs00165556_m1 | CATCACCAAACGAATGGCTGCAGTC | 55 |
| 34 | PRSS33 | Hs00960785_g1 | CTTCCCCAGGAGGGCACTGCCAGCT | 56 |
| 35 | PYROXD2 | Hs01550625_m1 | GGGGCTCATCCTGGAGGAGGTGTGA | 57 |
| 36 | RNASE2 | Hs03047029_s1 | CAGTGGAAGCCAGGTGCCTTTAATC | 58 |
| 37 | SIGLEC8 | Hs00274289_m1 | TGAGGGCACAGGCACCTCAAGACCT | 59 |
| 38 | SLC16A14 | Hs00541300_m1 | ACCACCTTTTGCAGGGTGGATCTAT | 60 |
| 38 | SLC29A1 | Hs01085706_m1 | CAGCCTCAGGACAGATACAAAGCTG | 61 |
| 40 | SLC47A1 | Hs00217320_m1 | GCTGGCCCCGCGTTCTTGGTTCAGC | 62 |
| 41 | SMPD3 | Hs00920354_m1 | CAACCTGCAGAAGGTCCTGGAGAGT | 63 |
| 42 | SORD | Hs00973148_m1 | TCAAGTAGTGGTGACTGATCTGTCT | 64 |
| 43 | SPNS3 | Hs00699394_m1 | GGCAGCCTGTCACAGGGACCCCAGA | 65 |
| 44 | THBS4 | Hs00170261_m1 | TGACATGTGTGTGTGGAGTCGGTTG | 66 |
| 45 | UGT2B28 | Hs00852540_s1 | AGCTCTGGGAGTTGTGGAAAGGTGC | 67 |

The assays for three housekeeping genes as gene specific endogenous control used for PCR normalization were also selected from the ABI TaqMan® Endogenous Controls inventor, and are shown in Table 3.

Table 3: Endogenous control gene assays

| Gene | Part Number |
|---|---|
| PPIA | 4333763F |
| GAPDH | 4333764F |
| TFRC | 4333770F |

Reverse transcription of RNA

Reverse transcription (RT) of PAXgene RNA of clinical samples to single-stranded cDNA was performed in a reaction volume of 20 μl using the High Capacity cDNA Reverse Transcription Kit following the protocol provided by the vendor (ABI). Briefly, the master mix with RNase inhibitor and reverse transcriptase was prepared and mixed with total RNA at 50 ng/μl in equal volume in a 96-well PCR microplate. The RT reaction was conducted on a thermocycler. The RT products were then diluted with nuclease free water to 10 ng/μl and stored at -20°C.

Pre-amplification of cDNA

To achieve optimal results for low gene expression in the Fluidigm system, Specific Target Amplification (STA) is recommended. Specific Target Amplification (STA) utilizes the TaqMan® PreAmp Master Mix and TaqMan® Gene Expression Assays, both from Applied Biosystems (ABI). The 45 gene expression assays shown in the table above for eosinophil-related candidate genes and 3 control expression assays for housekeeping genes were pooled and diluted to 0.2 X with nuclease free TE buffer. The pre-amplification was performed in volume of 5 μl containing 12.5 ng of cDNA for the recommended 14 cycles with pooled gene expression assays in the reaction as the source of primers only to the targets of interest following the protocol provided in PreAmp kit. The pre-amplified DNA was diluted by 5 fold with nuclease free TE buffer and stored at 4°C or -20°C for long term. Two internal references, one derived from purified human eosinophils and the other purchased from Clontech Laboratories were also included in the pre-amplification procedure.

Fluidigm platform PCR reaction

qPCR for gene expression was conducted with 96.96 Dynamic Array Integrated Fluidic Circuits (IFCs) of the BioMark™ System from Fluidigm following vendor guidelines. Briefly, Dynamic Array IFC was primed with control line fluid on an IFC controller. The samples and assays were prepared separately under a DNA-free environment in 5 $\mu$l each for each sample and assay respectively. The 96 samples and 48 assays in duplicate were placed on the Dynamic Array IFC and loaded using an IFC Controller. The Dynamic Array IFC was then run on the BioMark System for real-time PCR and data collection. Each 96 sample set applied to each IFC includes 88 clinical samples, negative controls in quadruplicate to ensure minimal contamination, and two internal universal references, each in duplicate, for normalization of gene expression between experiments and quality control evaluation.

Data collection and processing

The PCR results collected from BioMark system were analyzed and graphed for ∆Rn as the function of Ct value. The results of each gene expression over all samples were manually assessed for optimal threshold to determine Ct values and fail / pass call based on the curve analog and exponential phase of amplification. The gene expression level represented by Ct value for each gene in the same IFC chip was normalized against the normalization index in the same sample to obtain delta Ct (dCt). The normalization index for each sample was calculated as the geometric mean of Ct values from three selected housekeeping genes. The delta delta Ct (ddCt) of each gene expression in each sample was then calculated against the same gene from the universal reference derived from eosinophils in the same experimental IFC chip. The ddCt of each gene from the second universal reference from different experimental IFC chips were used for quality control validation with a pre-defined acceptable range of coefficient of variation (CV) less than 10% for the genes with detectable expression. The ddCts of each gene for the samples in each individual IFC chip were then merged as a metadata set for bio-statistical analysis. Gene expression below the detection limit was labeled as NA.

Data analysis was performed in the R statistical programming language, version 2.15. Expression data was calculated as follows:

For each individual i and gene j on each plate,

Index(i) = geometric mean of Ct values from three housekeeping genes

$$dCt(gene) = Ct(i,j) - Index(i);$$

$$ddCt = dCt(i,j) - dCt(Ref1, j).$$

ddCt values from each chip were merged together. ddCt of each gene in Ref 2 was used for quality control, and the coefficient of variance was calculated to ensure less than 10% for all genes in detectable range.

Samples with no result were assumed missing at random and not imputed. Samples or genes with more than 20% missing values were excluded from analysis. Remaining missing data was examined relative to distributions of adjacent complete vectors of data to determine whether to treat them as missing at random or below limit of detection. For summary statistics, samples with a missing value were excluded from calculation (e.g., excluded from the total number of samples for a given treatment and biomarker).

Distributions of data were examined visually to ensure both that genes are normally distributed within samples and that samples are normally distributed for each gene. Batch effects (e.g. qPCR plate) were examined and accounted for by blocking if necessary. Genes with extreme batch effects were excluded. Combinations of gene expression predictive variables were calculated by arithmetic mean of log-scale data.

For all variables, baseline is defined as the last assessment prior to the prespecified treatment administration (Day 0). Key demographics and baseline characteristics (including stratification variables and any variables with known prognostic significance) and efficacy outcomes were compared between biomarker and non-biomarker populations to investigate any potential selection biases associated with the missing status of the biomarker.

Overall distribution of each biomarker at baseline was plotted and descriptive statistics (e.g., mean, standard deviation, median and range) were used to summarize baseline biomarker values for the patient population.

The predictive effect of the biomarker(s) measured at baseline was evaluated by two methods. The first method was performing a linear regression of each outcome variable and each biomarker as follows:

$$\Delta\text{FEV}_1 \sim \text{treatment} + \text{expression} + \text{treatment: expression}$$

and testing for significant predictive power of each biomarker by examining the statistical significance and effect size of the interaction term. The second method was splitting the study subjects into two groups, those above and those below median level for each biomarker and calculating the difference in the effect of treatment between the two groups. A biomarker was considered successful in the first method as a potential predictor of treatment benefit if the following criteria were met:

1. There is strong evidence of biomarker-treatment interaction (p-value < 0.05)
2. The magnitude of the treatment effect in the diagnostic positive population is at least 8%
3. The direction of the effect is positive, i.e. higher gene expression is related to improved treatment benefit

A biomarker was considered successful in the second method as a potential predictor of treatment benefit if the following criteria were met:

1. There is strong evidence of different benefit level in the biomarker high vs. biomarker low group (non-overlapping confidence intervals in mean estimate)
2. The direction of the effect is positive, i.e. higher gene expression is related to improved treatment benefit

As a secondary measure of success, performance of biomarkers will be compared to the performance of serum periostin, fractional exhaled nitric oxide (FeNO), and peripheral blood eosinophil count.

EXAMPLE 2 - Serum periostin is elevated in pediatric patients

We measured periostin in serum samples from asthma patients ranging in age from 6-75 years taken prior to treatment in previously conducted clinical trials. *See, e.g.*, Hanania et al. (2013) Am J Respir Crit Care Med 187: 804-11; Hanania et al. (2011) Ann Intern Med 154: 573-82; Milgrom et al. (2001) Pediatrics 108: E36; Milgrom et al. (1999) N Engl J Med 341: 1966-73; Busse et al. (2001) J Allergy Clin Immunol 108: 184-90; Holgate et al. (2004) Clin Exp Allergy 34: 632-8; and Soler et al. (2001) Eur Respir J 18: 254-61. Periostin levels observed a relatively consistent distribution in adult patients (Fig. 1) between the ages of 18 and 75 years old with a median level of 48.9 ng/ml and a range of 22.1-108.7 ng/ml (Table 4). In pediatric patients (age 6-17 years), serum periostin levels were markedly elevated relative to adult patients (Fig. 1), with median levels of 119.6 ng/ml in the 6-11 year old group and 104.3 ng/ml in the 12-17 year old group with a wide range of levels from 41.4-352.2 ng/ml observed across all pediatric patients (Table 4). Notably, the median levels in pediatric patients exceeded the upper end of the overall range observed in adult patients. Furthermore, the levels appeared to be considerably more variable in pediatric asthma patients relative to adults. These findings are consistent with observations made in juvenile and adult mice (*see* Contie et al. (2010) Calcif Tissue Int 87: 341-50) and suggest that serum periostin cutoffs to select patients for anti-IL13 therapy derived in adults may not be applicable to pediatric patients.

Table 4: Range and distribution of serum periostin levels according to age in omalizumab studies 008, 009, 010, and EXTRA. Values are ng/ml.

|  | AGE: 6-11 yrs | AGE: 12-17 yrs | AGE: 18-75 yrs |
|---|---|---|---|
| n | 107 | 57 | 659 |
| Mean (SD) | 129.0 (43.5) | 100.5 (33.0) | 51.9 (15.3) |
| **Median** | **119.6** | **104.3** | **48.9** |
| Range | 59.3 - 352.2 | 41.4 - 177.2 | 22.1 - 108.7 |

EXAMPLE 3 - Blood eosinophils and serum periostin are correlated in moderate-severe adult but not pediatric asthmatics

While blood eosinophil counts tend to be slightly higher in pediatric asthma patients than in adults, the distributions are overlapping and, unlike serum periostin, blood eosinophil counts observe a smooth and continuous slightly downward trend over age, extending out until about the age of 40 where it levels off (Fig. 2). It has previously been shown that blood eosinophil and serum periostin levels are positively correlated in severe asthma patients. *See*, *e.g.*,

Jia et al. (2012) J Allergy Clin Immunol 130: 647-654 e10. To confirm this finding in additional larger sample sets, pre-treatment levels of blood eosinophils in two large clinical trial cohorts of moderate-severe asthma in adults (EXTRA (Hanania et al. (2013) Am J Respir Crit Care Med 187: 804-11) and MILLY (Corren et al. (2011) N Engl J Med 365: 1088-98)) were assessed; moderate but statistically significant positive correlations between serum periostin and blood eosinophils was found (Fig. 3A, EXTRA; Fig. 3B, MILLY). However, in pediatric asthma patients, blood eosinophils and serum periostin are not positively correlated (Fig. 3C, patients age 12-17 in EXTRA; 3D, patients age 6-12 in study 010). The slightly elevated blood eosinophil counts in pediatric asthma patients may be a function of a greater propensity toward atopy and are unlikely to be due to an independent physiological cause as appears to be the case for serum periostin.

EXAMPLE 4 - Gene expression patterns in peripheral blood are related to eosinophil counts

As serum periostin predicts both airway eosinophilia (Jia et al. (2012) J Allergy Clin Immunol 130: 647-654 e10) and responsiveness to lebrikizumab (Corren et al. (2011) N Engl J Med 365: 1088-98) in adult asthmatics, and as blood eosinophil counts correlate to airway eosinophils in both adult (Jia et al. (2012) J Allergy Clin Immunol 130: 647-654 e10) and pediatric (Baraldo et al. (2011) Eur Respir J 38: 575-83) asthma patients, it was hypothesized that gene expression patterns in peripheral blood that correspond to blood eosinophil counts might be suitable biomarkers to predict clinical benefit from lebrikizumab. To identify transcripts in peripheral blood associated with blood eosinophil counts in moderate-severe asthma patients, genome-wide expression microarray analyses of whole blood (PAXgene) samples from 321 subjects at baseline in the EXTRA study was conducted. *See* Hanania et al. (2013) Am J Respir Crit Care Med 187: 804-11; Hanania et al. (2011) Ann Intern Med 154: 573-82. Over 150 transcripts that were expressed at significantly higher levels in the top vs. bottom tertile of blood eosinophil counts (q-value < 0.05) were identified. Table 5 lists the top 150 differentially expressed transcripts between the top and bottom tertiles of blood eosinophil counts. It was confirmed that these transcripts were related continuously to blood eosinophil counts by examining rank-order (Spearman's) correlations between transcript levels and eosinophil counts.

Table 5: Top 150 differentially expressed transcripts between the top and bottom tertiles of blood eosinophil counts

| Gene symbol | Gene name | Entrez ID | probeset ID | expression ratio, high: low tertile (log2) | q-value | r(s) vs. eosinophil count | Expression pattern |
|---|---|---|---|---|---|---|---|
| CCL23 | chemokine (C-C motif) ligand 23 | 6368 | 210548_at | 2.74 | 2.72E-46 | 0.63 | other |
| PRSS33 | protease, serine, 33 | 260429 | 1552349_a_at | 2.25 | 1.18E-44 | 0.65 | |
| OLIG2 | oligodendrocyte lineage transcription factor 2 | 10215 | 213825_at | 2.15 | 5.57E-47 | 0.67 | eo |
| SIGLEC8 | sialic acid binding Ig-like lectin 8 | 27181 | 208253_at | 2.03 | 4.85E-47 | 0.67 | eo |
| ALOX15 | arachidonate 15-lipoxygenase | 246 | 207328_at | 1.95 | 4.50E-34 | 0.61 | other |
| PMP22 | peripheral myelin protein 22 | 5376 | 210139_s_at | 1.82 | 1.35E-34 | 0.57 | other |
| IL5RA | interleukin 5 receptor, alpha | 3568 | 211517_s_at | 1.76 | 5.30E-43 | 0.63 | eobaso |
| IDOI | indoleamine 2,3-dioxygenase 1 | 3620 | 210029_at | 1.61 | 2.11E-29 | 0.55 | other |
| SLC16A14 | solute carrier family 16, member 14 | 151473 | 238029_s_at | 1.52 | 2.14E-27 | 0.52 | |
| SLC29A1 | solute carrier family 29 (nucleoside transporters), member 1 | 2030 | 201802_at | 1.48 | 7.63E-50 | 0.67 | |
| SMPD3 | sphingomyelin phosphodiesterase 3, neutral membrane | 55512 | 219695_at | 1.39 | 1.20E-46 | 0.66 | eo |
| CACNG6 | calcium channel, voltage-dependent, gamma subunit 6 | 59285 | 1552863_a_at | 1.36 | 1.07E-27 | 0.55 | other |
| GPR44 | G protein-coupled receptor 44 | 11251 | 206361_at | 1.25 | 2.11E-38 | 0.60 | eo |
| HRH4 | histamine receptor H4 | 59340 | 221170_at | 1.24 | 8.08E-29 | 0.55 | other |

| Gene symbol | Gene name | Entrez ID | probeset ID | expression ratio, high: low tertile (log2) | q-value | r(s) vs. eosinophil count | Expression pattern |
|---|---|---|---|---|---|---|---|
| IL1RL1 | interleukin 1 receptor-like 1 | 9173 | 242809_at | 1.23 | 6.86E-24 | 0.52 | |
| CD9 | CD9 molecule | 928 | 233317_at | 1.20 | 3.31E-19 | 0.47 | |
| OLIG1 | oligodendrocyte transcription factor 1 | 116448 | 228170_at | 1.06 | 2.05E-30 | 0.56 | eobaso |
| CYSLTR2 | cysteinyl leukotriene receptor 2 | 57105 | 220813_at | 1.05 | 1.18E-26 | 0.51 | eobaso |
| SORD | sorbitol dehydrogenase | 6652 | 230782_at | 1.01 | 2.89E-28 | 0.53 | |
| LRRC17 | leucine rich repeat containing 17 | 10234 | 205381_at | 0.97 | 1.84E-26 | 0.54 | |
| UGT2B28 | UDP glucuronosyltransferase 2 family, polypeptide B28 | 54490 | 211682_x_at | 0.95 | 5.78E-24 | 0.49 | |
| CCR3 | chemokine (C-C motif) receptor 3 | 1232 | 208304_at | 0.92 | 8.97E-19 | 0.45 | |
| HSD3B7 | hydroxy-delta-5 -steroid dehydrogenase, 3 beta- and steroid delta-isomerase 7 | 80270 | 222817_at | 0.91 | 1.12E-24 | 0.55 | eo |
| KIAA1024 | KIAA1024 | 23251 | 215081_at | 0.89 | 1.50E-17 | 0.43 | |
| CLC | Charcot-Leyden crystal protein | 1178 | 206207_at | 0.89 | 2.59E-33 | 0.62 | eo |
| BACE2 | beta-site APP-cleaving enzyme 2 | 25825 | 222446_s_at | 0.88 | 1.16E-25 | 0.48 | |
| MEIS2 | Meis homeobox 2 | 4212 | 207480_s_at | 0.88 | 4.57E-24 | 0.52 | eo |
| CYP4F12 | cytochrome P450, family 4, subfamily F, polypeptide 12 | 66002 | 206539_s_at | 0.88 | 4.47E-17 | 0.41 | |

| Gene symbol | Gene name | Entrez ID | probeset ID | expression ratio, high: low tertile (log2) | q-value | r(s) vs. eosinophil count | Expression pattern |
|---|---|---|---|---|---|---|---|
| ASB2 | ankyrin repeat and SOCS box containing 2 | 51676 | 227915_at | 0.87 | 5.35E-26 | 0.53 | eo |
| LGALS12 | lectin, galactoside-binding, soluble, 12 | 85329 | 223828_s_at | 0.87 | 7. 11E-23 | 0.47 | |
| SPNS3 | spinster homolog 3 (Drosophila) | 201305 | 235900_at | 0.83 | 9.31E-24 | 0.50 | other |
| P2RY14 | purinergic receptor P2Y, G-protein coupled, 14 | 9934 | 206637_at | 0.82 | 3.40E-21 | 0.47 | other |
| PYROXD2 | pyridine nucleotide-disulphide oxidoreductase domain 2 | 84795 | 228384_s_at | 0.76 | 2.41E-23 | 0.52 | eobaso |
| THBS4 | thrombospondin 4 | 7060 | 204776_at | 0.75 | 8.13E-30 | 0.51 | |
| DACH1 | dachshund homolog 1 (Drosophila) | 1602 | 205471_s_at | 0.75 | 8.69E-16 | 0.43 | eobaso |
| P2RY2 | purinergic receptor P2Y, G-protein coupled, 2 | 5029 | 206277_at | 0.68 | 1.05E-13 | 0.42 | |
| CSF1 | colony stimulating factor 1 (macrophage) | 1435 | 209716_at | 0.64 | 1.24E-18 | 0.51 | |
| MYB | v-myb myeloblastosis viral oncogene homolog (avian) | 4602 | 204798_at | 0.64 | 1.58E-22 | 0.48 | eo |
| SLC47A1 | solute carrier family 47, member 1 | 55244 | 219525_at | 0.63 | 2.18E-07 | 0.32 | eobaso |
| ACOT11 | acyl-CoA thioesterase 11 | 26027 | 214763_at | 0.63 | 5.07E-26 | 0.53 | |
| ABTB2 | ankyrin repeat and BTB (POZ) domain containing 2 | 25841 | 213497_at | 0.61 | 8.40E-22 | 0.50 | other |
| FAM124B | family with sequence similarity 124B | 79843 | 220637_at | 0.60 | 7.12E-11 | 0.35 | |

EP 3 060 685 B1

54

| Gene symbol | Gene name | Entrez ID | probeset ID | expression ratio, high: low tertile (log2) | q-value | r(s) vs. eosinophil count | Expression pattern |
|---|---|---|---|---|---|---|---|
| RNASE2 | ribonuclease, RNase A family, 2 (liver, eosinophil-derived neurotoxin) | 6036 | 206111_at | 0.50 | 5.03E-09 | 0.31 | other |
| CYSLTR1 | cysteinyl leukotriene receptor 1 | 10800 | 216288_at | 0.47 | 2.78E-07 | 0.32 | eobaso |
| MGAT3 | mannosyl (beta-1,4-)-glycoprotein beta-1,4-N-acetylglucosaminyltransferase | 4248 | 209764_at | 0.36 | 2.42E-09 | 0.38 | |
| IDO2 | indoleamine 2,3-dioxygenase 2 | 169355 | 1568638_a_at | 0.32 | 2.45E-06 | 0.28 | |
| KBTBD11 | kelch repeat and BTB (POZ) domain containing 11 | 9920 | 204301_at | 0.26 | 1.35E-04 | 0.30 | |
| GLOD5 | glyoxalase domain containing 5 | 392465 | 241564_at | 1.59 | 2.31E-22 | 0.53 | eo |
| ADORA3 | adenosine A3 receptor | 140 | 206171_at | 1.49 | 3.10E-38 | 0.60 | eo |
| ABP1 | amiloride binding protein 1 (amine oxidase (copper-containing)) | 26 | 203559_s_at | 1.37 | 5.21E-13 | 0.38 | |
| GPR82 | G protein-coupled receptor 82 | 27197 | 1553317_s_at | 1.31 | 1.88E-27 | 0.51 | |
| HRASLS5 | HRAS-like suppressor family, member 5 | 117245 | 231050_at | 1.23 | 6.36E-41 | 0.61 | other |
| C21orf130 | chromosome 21 open reading frame 130 | 284835 | 240068_at | 1.21 | 1.00E-24 | 0.51 | other |
| CEBPE | CCAAT/enhancer binding protein (C/EBP), epsilon | 1053 | 214523_at | 1.18 | 1.35E-34 | 0.58 | eo |
| PIK3R6 | phosphoinositide-3-kinase, regulatory subunit 6 | 146850 | 1558770_a_at | 1.12 | 3.32E-36 | 0.61 | eo |
| TRPC6 | transient receptor potential cation channel, subfamily C, member 6 | 7225 | 206528_at | 1.06 | 2.26E-18 | 0.42 | other |

| Gene symbol | Gene name | Entrez ID | probeset ID | expression ratio, high: low tertile (log2) | q-value | r(s) vs. eosinophil count | Expression pattern |
|---|---|---|---|---|---|---|---|
| CAT | catalase | 847 | 215573_at | 0.99 | 3.10E-23 | 0.53 | other |
| CACNA1D | calcium channel, voltage-dependent, L type, alpha 1D subunit | 776 | 243334_at | 0.99 | 1.01E-23 | 0.50 | |
| FGFR2 | fibroblast growth factor receptor 2 | 2263 | 203638_s_at | 0.96 | 5.24E-07 | 0.30 | other |
| VSTM1 | V-set and transmembrane domain containing 1 | 284415 | 235818_at | 0.94 | 1.32E-17 | 0.46 | other |
| PDE4D | phosphodiesterase 4D, cAMP-specific | 5144 | 1554717_a_at | 0.91 | 1.69E-23 | 0.49 | eo |
| STAC | SH3 and cysteine rich domain | 6769 | 205743_at | 0.88 | 1.91E-11 | 0.34 | |
| SVOPL | SVOP-like | 136306 | 1554300_a_at | 0.88 | 3.62E-22 | 0.47 | |
| HES1 | hairy and enhancer of split 1, (Drosophila) | 3280 | 203394_s_at | 0.81 | 2.58E-22 | 0.46 | |
| CDK15 | cyclin-dependent kinase 15 | 65061 | 239201_at | 0.80 | 1.24E-17 | 0.44 | |
| CD24 | CD24 molecule | 100133941 | 208650_s_at | 0.76 | 2.08E-13 | 0.37 | |
| SLC7A8 | solute carrier family 7 (amino acid transporter, L-type), member 8 | 23428 | 216092_s_at | 0.76 | 5.28E-15 | 0.39 | other |
| VLDLR | very low density lipoprotein receptor | 7436 | 209822_s_at | 0.74 | 1.16E-11 | 0.36 | other |
| RPS6KA2 | ribosomal protein S6 kinase, 90kDa, polypeptide 2 | 6196 | 212912_at | 0.73 | 4.76E-22 | 0.48 | other |
| SPATA9 | spermatogenesis associated 9 | 83890 | 223840_s_at | 0.72 | 2.31E-22 | 0.44 | eo |

| Gene symbol | Gene name | Entrez ID | probeset ID | expression ratio, high: low tertile (log2) | q-value | r(s) vs. eosinophil count | Expression pattern |
|---|---|---|---|---|---|---|---|
| SYNE1 | spectrin repeat containing, nuclear envelope 1 | 23345 | 215350_at | 0.72 | 4.85E-12 | 0.32 | other |
| EPAS1 | endothelial PAS domain protein 1 | 2034 | 200878_at | 0.69 | 3.59E-13 | 0.40 | eo |
| TOX2 | TOX high mobility group box family member 2 | 84969 | 228737_at | 0.68 | 2.52E-14 | 0.40 | |
| CHST13 | carbohydrate (chondroitin 4) sulfotransferase 13 | 166012 | 239647_at | 0.67 | 2.12E-07 | 0.30 | eo |
| PLEKHA7 | pleckstrin homology domain containing, family A member 7 | 144100 | 228450_at | 0.66 | 7.60E-12 | 0.37 | |
| EEF2K | eukaryotic elongation factor-2 kinase | 29904 | 225546_at | 0.65 | 1.47E-17 | 0.44 | |
| MYCT1 | myc target 1 | 80177 | 231947_at | 0.65 | 7.75E-13 | 0.35 | other |
| FBN1 | fibrillin 1 | 2200 | 202766_s_at | 0.63 | 1.45E-13 | 0.41 | |
| EMR1 | egf-like module containing, mucin-like, hormone receptor-like 1 | 2015 | 207111_at | 0.63 | 1.25E-15 | 0.39 | other |
| SEMA7A | semaphorin 7A, GPI membrane anchor | 8482 | 230345_at | 0.62 | 6.52E-25 | 0.52 | |
| LOC283454 | hypothetical protein LOC283454 | 283454 | 229552_at | 0.61 | 1.01E-05 | 0.26 | eo |
| C6orf114 | chromosome 6 open reading frame 114 | 85411 | 1554486_a_at | 0.60 | 1.21E-11 | 0.37 | |
| GPR114 | G protein-coupled receptor 114 | 221188 | 229971_at | 0.57 | 1.00E-16 | 0.46 | |
| GFOD1 | glucose-fructose oxidoreductase domain containing 1 | 54438 | 219821_s_at | 0.57 | 4.39E-14 | 0.40 | other |

| Gene symbol | Gene name | Entrez ID | probeset ID | expression ratio, high: low tertile (log2) | q-value | r(s) vs. eosinophil count | Expression pattern |
|---|---|---|---|---|---|---|---|
| LOC645638 | WDNM1-like pseudogene | 645638 | 229566_at | 0.57 | 1.00E-13 | 0.34 | other |
| ACACB | acetyl-CoA carboxylase beta | 32 | 49452_at | 0.56 | 8.02E-13 | 0.39 | |
| EPN2 | epsin 2 | 22905 | 241239_at | 0.56 | 2.00E-13 | 0.37 | other |
| C10orf128 | chromosome 10 open reading frame 128 | 170371 | 228372_at | 0.56 | 1.91E-14 | 0.42 | eo |
| ENPP2 | ectonucleotide pyrophosphatase/ phosphodiesterase 2 | 5168 | 209392_at | 0.56 | 6.03E-08 | 0.31 | eo |
| PIK3R3 | phosphoinositide-3 -kinase, regulatory subunit 3 (gamma) | 8503 | 202743_at | 0.55 | 1.81E-10 | 0.35 | other |
| GPR34 | G protein-coupled receptor 34 | 2857 | 223620_at | 0.55 | 1.53E-06 | 0.26 | other |
| DIXDC 1 | DIX domain containing 1 | 85458 | 214724_at | 0.54 | 8.66E-09 | 0.31 | other |
| TFF3 | trefoil factor 3 (intestinal) | 7033 | 204623_at | 0.54 | 8.90E-13 | 0.41 | other |
| EFNB2 | ephrin-B2 | 1948 | 202668_at | 0.54 | 3.05E-11 | 0.34 | other |
| ACSF2 | acyl-CoA synthetase family member 2 | 80221 | 218844_at | 0.52 | 2.64E-17 | 0.46 | eobaso |
| SRGAP3 | SLIT-ROBO Rho GTPase activating protein 3 | 9901 | 209794_at | 0.52 | 1.88E-13 | 0.42 | |
| PNPLA6 | patatin-like phospholipase domain containing 6 | 10908 | 203718_at | 0.51 | 1.52E-14 | 0.40 | |
| GPR39 | G protein-coupled receptor 39 | 2863 | 208600_s_at | 0.51 | 1.52E-14 | 0.38 | |

| Gene symbol | Gene name | Entrez ID | probeset ID | expression ratio, high: low tertile (log2) | q-value | r(s) vs. eosinophil count | Expression pattern |
|---|---|---|---|---|---|---|---|
| ASRGL1 | asparaginase like 1 | 80150 | 218857_s_at | 0.51 | 1.91E-11 | 0.37 | |
| CCDC141 | coiled-coil domain containing 141 | 285025 | 1553645_at | 0.50 | 3.55E-09 | 0.28 | |
| GFI1B | growth factor independent 1B transcription repressor | 8328 | 237403_at | 0.50 | 5.24E-07 | 0.29 | other |
| ATP8B3 | ATPase, aminophospholipid transporter, class I, type 8B, member 3 | 148229 | 239457_at | 0.50 | 6.72E-13 | 0.37 | other |
| PHKG2 | phosphorylase kinase, gamma 2 (testis) | 5261 | 1556369_a_at | 0.50 | 2.10E-10 | 0.37 | eo |
| ZBTB42 | zinc finger and BTB domain containing 42 | 100128927 | 229691_at | 0.49 | 2.54E-18 | 0.44 | |
| KHDRBS3 | KH domain containing, RNA binding, signal transduction associated 3 | 10656 | 209781_s_at | 0.48 | 3.76E-14 | 0.44 | other |
| COL9A2 | collagen, type IX, alpha 2 | 1298 | 213622_at | 0.47 | 1.78E-05 | 0.28 | other |
| CNKSR3 | CNKSR family member 3 | 154043 | 227481_at | 0.46 | 1.31E-10 | 0.33 | |
| TEC | tec protein tyrosine kinase | 7006 | 206301_at | 0.46 | 2.80E-14 | 0.44 | |
| DAPK2 | death-associated protein kinase 2 | 23604 | 215184_at | 0.46 | 1.22E-06 | 0.33 | |
| LOC285812 | hypothetical protein LOC285812 | 285812 | 230179_at | 0.45 | 4.32E-13 | 0.37 | eo |
| VSIG10 | V-set and immunoglobulin domain containing 10 | 54621 | 226485_at | 0.45 | 3.45E-04 | 0.26 | other |
| ZNRF3 | zinc and ring finger 3 | 84133 | 226360_at | 0.45 | 5.50E-11 | 0.35 | eo |

EP 3 060 685 B1

| Gene symbol | Gene name | Entrez ID | probeset ID | expression ratio, high: low tertile (log2) | q-value | r(s) vs. eosinophil count | Expression pattern |
|---|---|---|---|---|---|---|---|
| CD101 | CD101 molecule | 9398 | 207167_at | 0.45 | 8.59E-08 | 0.30 | other |
| AKR1C1 | aldo-keto reductase family 1, member C1 | 1645 | 204151_x_at | 0.45 | 7.18E-07 | 0.28 | |
| FHL3 | four and a half LIM domains 3 | 2275 | 218818_at | 0.43 | 1.22E-07 | 0.34 | other |
| BLM | Bloom syndrome, RecQ helicase-like | 641 | 205733_at | 0.43 | 1.07E-08 | 0.31 | |
| CAMK1 | calcium/calmodulin-dependent protein kinase I | 8536 | 1558556_at | 0.43 | 9.12E-07 | 0.27 | other |
| C6orf97 | chromosome 6 open reading frame 97 | 80129 | 220581_at | 0.43 | 2.44E-09 | 0.31 | eo |
| ZNF823 | zinc finger protein 823 | 55552 | 229732_at | 0.43 | 2.44E-09 | 0.35 | |
| LYPD1 | LY6/PLAUR domain containing 1 | 116372 | 212909_at | 0.42 | 6.12E-12 | 0.38 | other |
| SAG | S-antigen; retina and pineal gland (arrestin) | 6295 | 206671_at | 0.42 | 2.77E-12 | 0.39 | |
| KLHL13 | kelch-like 13 (Drosophila) | 90293 | 227875_at | 0.42 | 6.12E-12 | 0.33 | eo |
| LOC81691 | exonuclease NEF-sp | 81691 | 208107_s_at | 0.42 | 1.81E-09 | 0.34 | eo |
| OXER1 | oxoeicosanoid (OXE) receptor 1 | 165140 | 1553222_at | 0.41 | 1.07E-10 | 0.38 | other |
| INPP1 | inositol polyphosphate-1 -phosphatase | 3628 | 202794_at | 0.41 | 2.89E-15 | 0.42 | other |
| HCRP1 | hepatocellular carcinoma-related HCRP1 | 387535 | 216176_at | 0.40 | 2.00E-04 | 0.29 | |

EP 3 060 685 B1

(continued)

| Gene symbol | Gene name | Entrez ID | probeset ID | expression ratio, high: low tertile (log2) | q-value | r(s) vs. eosinophil count | Expression pattern |
|---|---|---|---|---|---|---|---|
| KIAA0649 | KIAA0649 | 9858 | 203955_at | 0.40 | 2.94E-11 | 0.37 | other |
| ACSM3 | acyl-CoA synthetase medium-chain family member 3 | 6296 | 210377_at | 0.40 | 1.72E-07 | 0.29 | other |
| KSR1 | kinase suppressor of ras 1 | 8844 | 235252_at | 0.39 | 2.14E-07 | 0.33 | |
| HYAL3 | hyaluronoglucosaminidase 3 | 8372 | 211728_s_at | 0.38 | 5.81E-07 | 0.25 | eobaso |
| AJAP1 | adherens junctions associated protein 1 | 55966 | 206460_at | 0.38 | 3.39E-10 | 0.36 | |
| BRI3BP | BRI3 binding protein | 140707 | 231810_at | 0.36 | 9.94E-11 | 0.34 | |
| KIF23 | kinesin family member 23 | 9493 | 244427_at | 0.35 | 2.45E-06 | 0.31 | other |
| KLHL6 | kelch-like 6 (Drosophila) | 89857 | 1560396_at | 0.35 | 5.59E-04 | 0.27 | other |
| CKB | creatine kinase, brain | 1152 | 200884_at | 0.35 | 4.72E-07 | 0.25 | |
| FBP1 | fructose-1,6-bisphosphatase 1 | 2203 | 209696_at | 0.34 | 6.06E-07 | 0.29 | |
| GAPT | GRB2-binding adaptor protein, transmembrane | 202309 | 1552386_at | 0.34 | 1.14E-06 | 0.26 | |
| HIC1 | hypermethylated in cancer 1 | 3090 | 230218_at | 0.32 | 4.69E-06 | 0.27 | eo |
| PLIN2 | perilipin 2 | 123 | 209122_at | 0.32 | 7.81E-08 | 0.34 | |
| FAM46B | family with sequence similarity 46, member B | 115572 | 229518_at | 0.32 | 2.14E-07 | 0.29 | |

| Gene symbol | Gene name | Entrez ID | probeset ID | expression ratio, high: low tertile (log2) | q-value | r(s) vs. eosinophil count | Expression pattern |
|---|---|---|---|---|---|---|---|
| LOC115110 | hypothetical LOC115110 | 115110 | 233960_s_at | 0.32 | 2.84E-08 | 0.31 | other |
| GOLIM4 | golgi integral membrane protein 4 | 27333 | 204324_s_at | 0.31 | 9.66E-05 | 0.30 | eo |
| MARCKSL1 | MARCKS-like 1 | 65108 | 200644_at | 0.31 | 4.32E-08 | 0.33 | |
| OLFM2 | olfactomedin 2 | 93145 | 223601_at | 0.31 | 8.46E-06 | 0.27 | |
| TMEM38A | transmembrane protein 38A | 79041 | 222896_at | 0.30 | 1.07E-04 | 0.27 | other |
| C14orfl02 | chromosome 14 open reading frame 102 | 55051 | 221231_s_at | 0.30 | 2.28E-06 | 0.26 | other |
| C13orf27 | chromosome 13 open reading frame 27 | 93081 | 213346_at | 0.30 | 7.18E-07 | 0.26 | |
| SEP11 | septin 11 | 55752 | 230071_at | 0.29 | 9.32E-05 | 0.26 | other |
| FAAH | fatty acid amide hydrolase | 2166 | 204231_s_at | 0.29 | 5.52E-04 | 0.26 | |
| KCNK6 | potassium channel, subfamily K, member 6 | 9424 | 223658_at | 0.28 | 5.60E-06 | 0.31 | other |
| GRAMD1B | GRAM domain containing 1B | 57476 | 212906_at | 0.28 | 3.27E-04 | 0.29 | other |
| KBTBD11 | kelch repeat and BTB (POZ) domain containing 11 | 9920 | 204301_at | 0.26 | 1.35E-04 | 0.30 | eobaso |

EXAMPLE 5 - Transcripts correlated with eosinophil counts include eosinophil-restricted and broadly expressed genes

In asthma patients, the inflammatory cytokines IL5 and IL13 in the airway may contribute to systemic eosinophilia in complementary ways: 1) IL5's primary function is to promote eosinophil hematopoiesis, mobilization, and survival, thus elevated IL5 expression in the airways sends a signal to the bone marrow to produce eosinophils; 2) among IL13's many functions is to induce the expression of CCR3-binding chemokines such as CCL11, 13, 23, 24, and 26 in structural cells of the bronchial mucosa such as epithelial cells and fibroblasts, thus the eosinophils mobilized by IL5 migrate toward the site of IL13 expression along a chemokine gradient. *See*, *e.g.*, Wen et al. (2013) Proc Natl Acad Sci U S A 110: 6067-72. In addition to promoting eosinophil chemotaxis via chemokine induction, IL13 exerts multiple additional effects on the airway that may lead to altered levels of soluble and cellular mediators in the peripheral blood. *See*, *e.g.*, Arron et al. (2013) Adv Pharmacol 66: 1-49. Hence, a process that promotes peripheral eosinophilia may also lead to other eosinophil-independent effects on gene expression in blood cells.

To determine whether transcripts related to peripheral blood eosinophil count were due to their expression in eosinophils or in other cell types, the relative expression levels of eosinophil-related transcripts in a publicly available dataset (GSE3982) comprising sorted blood cells was examined. *See* Liu et al. (2006) J Allergy Clin Immunol 118: 496-503. Among the most highly eosinophil-related transcripts in the EXTRA dataset that were also represented in the GSE3982 dataset, three major patterns of expression were identified: 1) eosinophil-restricted, where expression of the transcript is predominantly found in eosinophils from among all the cell types examined; 2) eosinophil/basophil-restricted, where expression is found in both eosinophils and basophils but not in other cell types, and 3) broad expression, where the transcript is found in multiple blood cell types, which may or may not include eosinophils. Interestingly, none of the selected transcripts were uniquely expressed in basophils despite many known transcripts specific to basophils among peripheral blood leukocytes (which may also be found in tissue-resident mast cells), such as FcεRI and tryptase genes, which suggests that the eosinophil/basophil-restricted transcripts were significant in our analysis because of their expression in eosinophils but not necessarily in basophils. Table 5 lists the expression patterns for transcripts represented in both datasets where "eo" denotes predominantly eosinophil-restricted expression, "eobaso" denotes expression mainly restricted to eosinophils and basophils, and "other" denotes broader expression and/or expression restricted to leukocytes other than eosinophils or basophils. Many transcripts were not represented in the GSE3982 dataset and thus are not annotated. Examples of the three patterns are illustrated in Fig. 4, where SIGLEC8 is largely restricted to eosinophils (Figure 4A), CLC is expressed predominantly in eosinophils and basophils (Figure 4B), and CSF1 is more broadly expressed across multiple cell types (Figure 4C), despite the fact that all three transcripts are highly correlated to blood eosinophil count. These data suggest that some eosinophil-related transcripts in peripheral blood may reflect biological processes that are associated with, but not strictly due to eosinophil count, which has the potential to add robustness to the power of peripheral blood transcripts to predict pathophysiological processes in the airways beyond simply counting eosinophils.

EXAMPLE 6 - Eosinophil-related transcripts in peripheral blood identify moderate-severe asthma patients with increased clinical benefit from lebrikizumab

We have shown in the phase II MILLY study that moderate-severe patients with poorly controlled asthma despite ICS with pre-treatment serum periostin or FeNO levels above the median level in the study exhibited significantly improved lung function on lebrikizumab treatment relative to placebo whereas patients with baseline serum periostin or FeNO levels below the median did not show a significant benefit from lebrikizumab relative to placebo. *See*, *e.g.*, Corren et al. (2011) N Engl J Med 365: 1088-98; Arron et al. (2011) N Engl J Med 365: 2433-34. To determine whether eosinophil-related gene expression in peripheral blood could similarly predict clinical benefit from lebrikizumab, expression of 47 eosinophil-related transcripts by qPCR in baseline PAXgene blood RNA samples from patients in the MILLY study was assessed. Samples were taken immediately prior to the first dose of study drug at randomization and were available for 208 of the 219 patients in the modified intent-to-treat population.

The first step in assessment of the expression data was consideration of quality. PCR amplification with the selected primer and probe sets failed for 3 genes (IDO2, KBTBD11, and P2RY2). IL5RA exhibited unacceptable technical performance (plate effects) during qPCR and was omitted from further analyses. LRRC17 data was missing in over 300over 25% of samples and was omitted from further analysis on that basis. Eight samples were missing data for over 25% of genes and were omitted. Remaining missing data were imputed because they were few and apparently randomly distributed.

The outcome assessed was the difference in placebo-adjusted change in $FEV_1$ at 12 weeks with patients dichotomized around the median expression level of each gene at baseline. To ensure consistency of response, post-hoc analyses examined the performance of the dichotomized groups to predict $FEV_1$ response continuously over the 32-week course of the study.

Over 20 transcripts performed comparably to or better than periostin, FeNO, and blood eosinophils to enrich

for placebo-adjusted $FEV_1$ benefit from lebrikizumab at week 12. When dichotomized according to the median expression level at baseline, patients with expression levels of CSF1, MEIS2, LGALS12, IDO1, THBS4, OLIG2, ALOX15, SIGLEC8, CCL23, PYROXD2, HSD3B7, SORD, ASB2, CACNG6, GPR44, MGAT3, SLC47A1, SMPD3, CCR3, CLC, CYP4F12, and ABTB2 above the median all exhibited significantly greater placebo-adjusted $FEV_1$ improvement from lebrikizumab at 12 weeks than patients with expression levels of those genes below the median (95% confidence intervals do not include zero, Fig. 5). The expression of several genes including CD9, P2RY14, PMP22, BACE2, RNASE2, FAM124B, UGT2828, ACOT11, CYSLTR1, IL1RL1, and MYB did not substantially enrich for placebo-adjusted $FEV_1$ benefit from lebrikizumab, while others including OLIG1, PRSS33, HRH4, SPNS3, SLC29A1, CYSLTR2, DACH1, and SLC16A14 enriched for clinical benefit but with 95% confidence intervals for placebo-adjusted $FEV_1$ improvement including zero (Fig. 5). Using baseline values dichotomized around the median, genes that differentiated for placebo-adjusted $FEV_1$ benefit at 12 weeks tended to enrich for $FEV_1$ benefit at all timepoints throughout the duration of the study (Fig. 6). Taken together, these data show that over 20 individual gene transcripts in peripheral blood can enrich for a population of moderate-severe asthma patients likely to experience enhanced clinical benefit from lebrikizumab.

EXAMPLE 7 - Peripheral blood gene expression levels are correlated to blood eosinophils in adult and pediatric subjects

As serum periostin is expressed at markedly different levels and is not correlated with blood eosinophils in pediatric asthma patients, it was determined whether eosinophil-related transcripts in peripheral blood were: 1) correlated with blood eosinophils in pediatric subjects and 2) scaled comparably in adult and pediatric subjects. Matched PAXgene blood RNA samples, blood eosinophil percentage, FeNO, and serum IgE levels from 411 subjects aged 8-21 in the "GALA II" study (Nishimura et al. (2013) Am J Respir Crit Care Med 188: 309-18; Kumar et al. (2013) J Allergy Clin Immunol doi: 10.1016/j.jaci.2013.02.046. [Epub ahead of print]; Borrell et al. (2013) Am J Respir Crit Care Med 187: 697-702), of whom 277 had physician-diagnosed asthma and 134 were age-matched healthy controls, were obtained. The asthmatic subjects in GALA had significantly lower $FEV_1$ % predicted and significantly higher serum IgE, blood eosinophils, and FeNO than the control subjects. The pediatric asthma subjects in GALA had significantly intercorrelated blood eosinophil percentage and FeNO levels, the range and distribution of which overlapped with blood eosinophil percentage and FeNO from adult moderate-severe asthmatic subjects in the BOBCAT cohort (Jia et al. (2012) J Allergy Clin Immunol 130: 647-654 e10) (Table 6). In both asthmatic subjects and healthy controls in GALA, there was a weak but significant negative correlation between blood eosinophil percentage and age (Fig. 7, similar to observations shown in other cohorts in Fig. 3), however blood eosinophil percentage was not substantially skewed relative to adult subjects.

Table 6: Clinical and demographic features of BOBCAT (adult) and GALA II (pediatric) cohorts

| | BOBCAT (N=88) | GALA (asthma; N=277) | GALA (control; N=134) |
|---|---|---|---|
| Age, median (range) | 47 (20-67) | 15 (8-21) | 15 (10-21) |
| Sex (M:F) | 32:34 | 133:144 | 59:75 |
| FEV1 % pred, med (IQR) | 61 (53-79) | 88 (80-99)* | 96 (85-105) |
| serum IgE | 160 (39-373) | 255 (89-634)* | 110 (36-290) |
| blood eos count/µl | 235 (115-375) | 300 (100-500)* | 200 (100-300) |
| blood eos, % | 3.7 (1.7-5.1) | 3.8 (2.2-6.7)* | 2.7 (1.5-5.8) |
| FeNO, ppb | 24 (16-49) | 25 (10-44)* | 13 (8-25) |

*p<0.0001 vs. GALA control

| | BOBCAT | GALA | |
|---|---|---|---|
| blood eos vs. FeNO, $r_S$ | 0.40 (p < 0.002) | 0.57 (p < 0.001) | |

Disclosed herein are anti-RSPO antibodies, in particular anti-RSPO2 antibodies and/or anti-RSPO3 antibodies, and methods of using the same.
To assess whether transcripts related to blood eosinophilia in adult asthmatics were related to blood eosinophilia in pediatric subjects and scaled comparably, the 43 genes described above were assessed by qPCR in GALA (the first GALA study is described, for example, in Corvol et al., Pharmacogenet Genomics. 2009 Jul;19(7):489-96) and 88 baseline samples from BOBCAT simultaneously to mitigate batch effects. Most genes observed similar relationships between expression level and blood eosinophil percentage in adult asthmatics and pediatric subjects with and without asthma, suggesting that the ability of gene expression to predict blood eosinophilia was consistent independent of age or diagnosis (e.g. CCL23, Fig. 8A). However, some transcripts observed divergent patterns between GALA and BOBCAT, e.g. CLC, which had comparable correlation coefficients relative to blood eosinophil percentage in adult and pediatric subjects but which was expressed at substantially higher levels for a given blood eosinophil percentage in adults than in pediatric subjects (Fig. 8B); or CSF1, which observed both divergent correlation coefficients and divergent scaling in adult and pediatric subjects (Fig. 8C). To identify the transcripts that were both highly related to blood eosinophil counts and minimally dependent on age, a linear regression model incorporating all data from BOBCAT, MILLY, and GALA in which gene expression was assessed as a function of blood eosinophil percentage, age, the interaction term between age and blood eosinophil percentage, and batch was constructed. Genes that were maximized in the model estimate for blood eosinophil percentage and minimized in the model estimate for age were identified (Fig. 9). Additional considerations for selecting genes included the ability of baseline gene expression level to identify patients with enhanced clinical benefit from lebrikizumab in the MILLY study and increased dynamic range of expression levels in peripheral blood. Table 7 shows the top 20 genes ranked by placebo-adjusted change in $FEV_1$ at week 12 in MILLY (Fig. 5), the Pearson correlation coefficients between gene expression and blood eosinophil percentage in BOBCAT and asthmatic subjects in GALA, the ratio of Y-intercept for the regression of $\Delta\Delta Ct$ vs. (blood eosinophil percentage)-2 as illustrated in Fig. 8 between pediatric and adult asthmatic subjects, and the dynamic range of expression as determined by the difference in $\Delta\Delta Ct$ between the 10th and 90th percentile of expression levels observed in all GALA and BOBCAT samples for each gene. Genes with comparable correlation coefficients (0-10%) between BOBCAT and GALA were deemed to have a higher priority than genes with divergent correlation coefficients; genes with smaller intercept ratios (0-10%) were deemed higher priority than genes with higher intercept ratios, and genes with larger dynamic ranges (> 3 cycles) were deemed higher priority than genes with smaller dynamic ranges.

Taken together, the performance of many genes in the linear regression model (Fig. 9) and categorical analyses (Table 7) suggest that they may be suitable as biomarkers predictive of enhanced clinical benefit from therapies targeting type 2 inflammatory mediators in pediatric asthma patients.

Table 7: Summary of blood eosinophil-related transcripts predicting lebrikizumab clinical benefit and relationships between gene expression and blood eosinophil percentage in adult and pediatric asthma

| Gene | MILLY $\Delta\Delta FEV_1$ (%) | $r_p$ vs. sqrt % eos BOBCAT | $r_p$ vs. sqrt % eos GALA (CASE) | $\Delta$ intercept ratio GALA vs. BOBCAT | dynamic range (ddCt) |
|---|---|---|---|---|---|
| CSF1 | 11.9 | 0.57 | 0.54 | 0.28** | 2.3* |
| MEIS2 | 10.3 | 0.37 | 0.41 | 0.14* | 3.4 |
| LGALS12 | 9.8 | 0.53* | 0.67* | 0.10 | 2.7* |
| IDO1 | 9.7 | 0.72 | 0.76 | 0.03 | 3.4 |
| THBS4 | 9.6 | 0.71 | 0.77 | 0.17* | 3.5 |
| OLIG2 | 9.5 | 0.78 | 0.82 | 0.26** | 3.8 |
| ALOX15 | 9.0 | 0.74 | 0.76 | 0.11* | 4.7 |
| SIGLEC8 | 8.6 | 0.82 | 0.79 | 0.11* | 4.4 |
| CCL23 | 8.5 | 0.71 | 0.79 | 0.04 | 4.9 |
| PYROXD2 | 8.4 | 0.59 | 0.62 | 0.10 | 2.3* |
| HSD3B7 | 8.2 | 0.44 | 0.46 | 0.08 | 3.7 |
| SORD | 8.2 | 0.57 | 0.60 | 0.15* | 2.3* |
| ASB2 | 8.1 | 0.69 | 0.73 | 0.11* | 2.6* |
| CACNG6 | 6.7 | 0.70 | 0.69 | 0.09 | 3.3 |
| GPR44 | 6.1 | 0.76 | 0.80 | 0.11* | 3.9 |
| MGAT3 | 6.0 | 0.50** | 0.17** | 0.94** | 4.5 |
| SLC47A1 | 5.9 | 0.26* | 0.41* | 0.18* | 3.0 |

(continued)

| Gene | MILLY $\Delta\Delta FEV_1$ (%) | $r_p$ vs. sqrt % eos BOBCAT | $r_p$ vs. sqrt % eos GALA (CASE) | $\Delta$intercept ratio GALA vs. BOBCAT | dynamic range (ddCt) |
|---|---|---|---|---|---|
| SMPD3 | 5.8 | 0.86 | 0.83 | 0.14* | 3.2 |
| CCR3 | 5.8 | 0.48* | 0.58* | 0.03 | 2.4* |
| CLC | 5.5 | 0.74 | 0.83 | -0.22** | 4.4 |
| ABTB2 | 5.1 | 0.69 | 0.68 | 0.27** | 2.9* |
| CYP4F12 | 5.1 | 0.38* | 0.24* | 0.46** | 4.1 |
| Note: All 0-10% >3 cycles, except * 10-15% 2-3 cycles and ** >15% <2 cycles. | | | | | |

EXAMPLE 8 - References

1. Arron, JR, H Scheerens, and JG Matthews. (2013) Redefining approaches to asthma: developing targeted biologic therapies. Adv Pharmacol 66: 1-49.

2. Jia, G, RW Erickson, DF Choy, S Mosesova, LC Wu, et al. (2012) Periostin is a systemic biomarker of eosinophilic airway inflammation in asthmatic patients. J Allergy Clin Immunol 130: 647-654 e10.

3. Corren, J, RF Lemanske, NA Hanania, PE Korenblat, MV Parsey, et al. (2011) Lebrikizumab treatment in adults with asthma. N Engl J Med 365: 1088-98.

4. Scheerens, H, JR Arron, DF Choy, S Mosesova, P Lal, et al. (2012) Lebrikizumab Treatment Reduces Serum Periostin Levels In Asthma Patients With Elevated Baseline Levels Of Periostin. Am J Respir Crit Care Med 185: A3960.

5. Horiuchi, K, N Amizuka, S Takeshita, H Takamatsu, M Katsuura, et al. (1999) Identification and characterization of a novel protein, periostin, with restricted expression to periosteum and periodontal ligament and increased expression by transforming growth factor beta. J Bone Miner Res 14: 1239-49.

6. Contie, S, N Voorzanger-Rousselot, J Litvin, N Bonnet, S Ferrari, et al. (2010) Development of a new ELISA for serum periostin: evaluation of growth-related changes and bisphosphonate treatment in mice. Calcif Tissue Int 87: 341-50.

7. Baraldo, S, G Turato, E Bazzan, A Ballarin, M Damin, et al. (2011) Noneosinophilic asthma in children: relation with airway remodelling. Eur Respir J 38: 575-83.

8. Hanania, NA, S Wenzel, K Rosen, HJ Hsieh, S Mosesova, et al. (2013) Exploring the effects of omalizumab in allergic asthma: an analysis of biomarkers in the EXTRA study. Am J Respir Crit Care Med 187: 804-11.

9. Hanania, NA, O Alpan, DL Hamilos, JJ Condemi, I Reyes-Rivera, et al. (2011) Omalizumab in severe allergic asthma inadequately controlled with standard therapy: a randomized trial. Ann Intern Med 154: 573-82.

10. Milgrom, H, W Berger, A Nayak, N Gupta, S Pollard, et al. (2001) Treatment of childhood asthma with anti-immunoglobulin E antibody (omalizumab). Pediatrics 108: E36.

11. Milgrom, H, RB Fick, Jr., JQ Su, JD Reimann, RK Bush, et al. (1999) Treatment of allergic asthma with monoclonal anti-IgE antibody. rhuMAb-E25 Study Group. N Engl J Med 341: 1966-73.

12. Busse, W, J Corren, BQ Lanier, M McAlary, A Fowler-Taylor, et al. (2001) Omalizumab, anti-IgE recombinant humanized monoclonal antibody, for the treatment of severe allergic asthma. J Allergy Clin Immunol 108: 184-90.

13. Holgate, ST, AG Chuchalin, J Hebert, J Lotvall, GB Persson, et al. (2004) Efficacy and safety of a recombinant anti-immunoglobulin E antibody (omalizumab) in severe allergic asthma. Clin Exp Allergy 34: 632-8.

14. Soler, M, J Matz, R Townley, R Buhl, J O'Brien, et al. (2001) The anti-IgE antibody omalizumab reduces exacerbations and steroid requirement in allergic asthmatics. Eur Respir J 18: 254-61.

15. Wen, T, JA Besse, MK Mingler, PC Fulkerson, and ME Rothenberg. (2013) Eosinophil adoptive transfer system to directly evaluate pulmonary eosinophil trafficking in vivo. Proc Natl Acad Sci U S A 110: 6067-72.

16. Liu, SM, R Xavier, KL Good, T Chtanova, R Newton, et al. (2006) Immune cell transcriptome datasets reveal novel leukocyte subset-specific genes and genes associated with allergic processes. J Allergy Clin Immunol 118: 496-503.

17. Floyd, H, J Ni, AL Cornish, Z Zeng, D Liu, et al. (2000) Siglec-8. A novel eosinophil-specific member of the immunoglobulin superfamily. J Biol Chem 275: 861-6.

18. Tavernier, J, R Devos, S Cornelis, T Tuypens, J Van der Heyden, et al. (1991) A human high affinity interleukin-5 receptor (IL5R) is composed of an IL5-specific alpha chain and a beta chain shared with the receptor for GM-CSF. Cell 66: 1175-84.

19. Nagata, K, H Hirai, K Tanaka, K Ogawa, T Aso, et al. (1999) CRTH2, an orphan receptor of T-helper-2-cells, is expressed on basophils and eosinophils and responds to mast cell-derived factor(s). FEBS Lett 459: 195-9.

20. Mita, H, M Hasegawa, H Saito, and K Akiyama. (2001) Levels of cysteinyl leukotriene receptor mRNA in human peripheral leucocytes: significantly higher expression of cysteinyl leukotriene receptor 2 mRNA in eosinophils. Clin Exp Allergy 31: 1714-23.

21. Turk, J, RL Maas, AR Brash, LJ Roberts, 2nd, and JA Oates. (1982) Arachidonic acid 15-lipoxygenase products from human eosinophils. J Biol Chem 257: 7068-76.

22. Gleich, GJ, DA Loegering, KG Mann, and JE Maldonado. (1976) Comparative properties of the Charcot-Leyden crystal protein and the major basic protein from human eosinophils. J Clin Invest 57: 633-40.

23. Ponath, PD, S Qin, TW Post, J Wang, L Wu, et al. (1996) Molecular cloning and characterization of a human eotaxin receptor expressed selectively on eosinophils. J Exp Med 183: 2437-48.

24. Ponath, PD, S Qin, DJ Ringler, I Clark-Lewis, J Wang, et al. (1996) Cloning of the human eosinophil chemoattractant, eotaxin. Expression, receptor binding, and functional properties suggest a mechanism for the selective recruitment of eosinophils. J Clin Invest 97: 604-12.

25. Kitaura, M, T Nakajima, T Imai, S Harada, C Combadiere, et al. (1996) Molecular cloning of human eotaxin, an eosinophil-selective CC chemokine, and identification of a specific eosinophil eotaxin receptor, CC chemokine receptor 3. J Biol Chem 271: 7725-30.

26. Daugherty, BL, SJ Siciliano, JA DeMartino, L Malkowitz, A Sirotina, et al. (1996) Cloning, expression, and characterization of the human eosinophil eotaxin receptor. J Exp Med 183: 2349-54.

27. Rothenberg, ME, R Ownbey, PD Mehlhop, PM Loiselle, M van de Rijn, et al. (1996) Eotaxin triggers eosinophil-selective chemotaxis and calcium flux via a distinct receptor and induces pulmonary eosinophilia in the presence of interleukin 5 in mice. Mol Med 2: 334-48.

28. Combadiere, C, SK Ahuja, and PM Murphy. (1996) Cloning and functional expression of a human eosinophil CC chemokine receptor. J Biol Chem 271: 11034.

29. Hansel, TT, JB Braunstein, C Walker, K Blaser, PL Bruijnzeel, et al. (1991) Sputum eosinophils from asthmatics express ICAM-1 and HLA-DR. Clin Exp Immunol 86: 271-7.

30. Kohno, Y, X Ji, SD Mawhorter, M Koshiba, and KA Jacobson. (1996) Activation of A3 adenosine receptors on human eosinophils elevates intracellular calcium. Blood 88: 3569-74.

31. Hamann, KJ, RM Ten, DA Loegering, RB Jenkins, MT Heise, et al. (1990) Structure and chromosome localization of the human eosinophil-derived neurotoxin and eosinophil cationic protein genes: evidence for intronless coding sequences in the ribonuclease gene superfamily. Genomics 7: 535-46.

32. Novak, H, A Muller, N Harrer, C Gunther, JM Carballido, et al. (2007) CCL23 expression is induced by IL-4 in a STAT6-dependent fashion. J Immunol 178: 4335-41.

33. Syed, F, CC Huang, K Li, V Liu, T Shang, et al. (2007) Identification of interleukin-13 related biomarkers using peripheral blood mononuclear cells. Biomarkers 12: 414-23.

34. Hamilton, JA. (2008) Colony-stimulating factors in inflammation and autoimmunity. Nat Rev Immunol 8: 533-44.

35. von Bubnoff, D and T Bieber. (2012) The indoleamine 2,3-dioxygenase (IDO) pathway controls allergy. Allergy 67: 718-25.

36. Esnault, S, EA Kelly, EA Schwantes, LY Liu, LP DeLain, et al. (2013) Identification of genes expressed by human airway eosinophils after an in vivo allergen challenge. PLoS One 8: e67560.

37. Lee, JS, JH Kim, JS Bae, JY Kim, TJ Park, et al. (2010) Association of CACNG6 polymorphisms with aspirin-intolerance asthmatics in a Korean population. BMC Med Genet 11: 138.

38. Burgess, DL, LA Gefrides, PJ Foreman, and JL Noebels. (2001) A cluster of three novel Ca2+ channel gamma subunit genes on chromosome 19q13.4: evolution and expression profile of the gamma subunit gene family. Genomics 71: 339-50.

39. Schmitz, J, A Owyang, E Oldham, Y Song, E Murphy, et al. (2005) IL-33, an interleukin-1-like cytokine that signals via the IL-1 receptor-related protein ST2 and induces T helper type 2-associated cytokines. Immunity 23: 479-90.

40. Gudbjartsson, DF, US Bjornsdottir, E Halapi, A Helgadottir, P Sulem, et al. (2009) Sequence variants affecting eosinophil numbers associate with asthma and myocardial infarction. Nat Genet 41: 342-7.

41. Meijer, DH, MF Kane, S Mehta, H Liu, E Harrington, et al. (2012) Separated at birth? The functional and molecular divergence of OLIG1 and OLIG2. Nat Rev Neurosci 13: 819-31.

42. Quarles, RH. (2002) Myelin sheaths: glycoproteins involved in their formation, maintenance and degeneration. Cell Mol Life Sci 59: 1851-71.

43. Arron, JR, J Corren, and JG Matthews. (2011) Author response to correspondence on "Lebrikizumab treatment in adults with asthma". N Engl J Med 365: 2433-34.

44. Nishimura, KK, JM Galanter, LA Roth, SS Oh, N Thakur, et al. (2013) Early-Life Air Pollution and Asthma Risk in Minority Children. The GALA II and SAGE II Studies. Am J Respir Crit Care Med 188: 309-18.

45. Kumar, R, EA Nguyen, LA Roth, SS Oh, CR Gignoux, et al. (2013) Factors associated with degree of atopy in Latino children in a nationwide pediatric sample: The Genes-environments and Admixture in Latino Asthmatics (GALA II) study. J Allergy Clin Immunol

46. Borrell, LN, EA Nguyen, LA Roth, SS Oh, H Tcheurekdjian, et al. (2013) Childhood obesity and asthma control in the GALA II and SAGE II studies. Am J Respir Crit Care Med 187: 697-702.

47. Aoki, T, Y Matsumoto, K Hirata, K Ochiai, M Okada, et al. (2009) Expression profiling of genes related to asthma exacerbations. Clin Exp Allergy 39: 213-221.

TABLE OF SEQUENCES

| SEQ ID NO: | Description | Sequence |
|---|---|---|
| 1 | Anti-M1' antibody (quilizumab) heavy chain variable region | EVQLVESGGG LVQPGGSLRL SCAASGFTFS DYGIAWVRQA PGKGLEWVAF ISDLAYTIYY ADTVTGRFTI SRDNSKNTLY LQMNSLRAED TAVYYCARDN WDAMDYWGQG TLVTVSS |
| 2 | Anti-M1' antibody (quilizumab) light chain variable region | DIQMTQSPSS LSASVGDRVT ITCRSSQSLV HNNANTYLHW YQQKPGKAPK LLIYKVSNRF SGVPSRFSGS GSGTDFTLTI SSLQPEDFAT YYCSQNTLVP WTFGQGTKVE IK |
| 3 | Anti-M1' (quilizumab) HVR-H1 | GFTFSDYGIA |
| 4 | Anti-M1' (quilizumab) HVR-H2 | AFISDLAYTIYYADTVTG |
| 5 | Anti-M1' (quilizumab) HVR-H3 | ARDNWDAMDY |
| 6 | Anti-M1' (quilizumab) HVR-L1 | RSSQSLVHNNANTYLH |
| 7 | Anti-M1' (quilizumab) HVR-L2 | KVSNRFS |
| 8 | Anti-M1' (quilizumab) HVR-L3 | SQNTLVPWT |
| 9 | lebrikizumab heavy chain variable region | VTLRESGPAL VKPTQTLTLT CTVSGFSLSA YSVNWIRQPP GKALEWLAMI WGDGKIVYNS ALKSRLTISK DTSKNQVVLT MTNMDPVDTA TYYCAGDGYY PYAMDNWGQG SLVTVSS |
| 10 | lebrikizumab light chain variable region | DIVMTQSPDS LSVSLGERAT INCRASKSVD SYGNSFMHWY QQKPGQPPKL LIYLASNLES GVPDRFSGSG SGTDFTLTIS SLQAEDVAVY YCQQNNEDPR TFGGGTKVEI K |
| 11 | lebrikizumab HVR-H1 | AYSVN |
| 12 | lebrikizumab HVR-H2 | MIWGDGKIVYNSALKS |
| 13 | lebrikizumab HVR-H3 | DGYYPYAMDN |
| 14 | lebrikizumab HVR-L1 | RASKSVDSYGNSFMH |
| 15 | lebrikizumab HVR-L1 | LASNLES |
| 16 | lebrikizumab HVR-L1 | QQNNEDPRT |
| 17 | Anti-IgE antibody light chain variable region | Asp Ile Gln Leu Thr Gln Ser Pro Ser Ser Leu Ser Ala Ser Val Gly Asp Arg Val Thr Ile Thr Cys Arg Ala Ser Gln Ser Val Asp Tyr Asp Gly Asp Ser Tyr Met Asn Trp Tyr Gln Gln Lys Pro Gly Lys Ala Pro Lys Leu Leu Ile Tyr Ala Ala Ser Tyr Leu Glu Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Ser His Glu Asp Pro Tyr Thr Phe Gly Gln Gly Thr Lys Val Glu Ile Lys Arg Thr Val |

(continued)

| SEQ ID NO: | Description | Sequence |
|---|---|---|
| 18 | Anti-IgE antibody heavy chain variable region | Glu Val Gln Leu Val Glu Ser Gly Gly Gly Leu Val<br>Gln Pro Gly Gly Ser Leu Arg Leu Ser Cys Ala Val<br>Ser Gly Tyr Ser Ile Thr Ser Gly Tyr Ser Trp Asn<br>Trp Ile Arg Gln Ala Pro Gly Lys Gly Leu Glu Trp<br>Val Ala Ser Ile Thr Tyr Asp Gly Ser Thr Asn Tyr<br>Asn Pro Ser Val Lys Gly Arg Ile Thr Ile Ser Arg<br>Asp Asp Ser Lys Asn Thr Phe Tyr Leu Gln Met Asn<br>Ser Leu Arg Ala Glu Asp Thr Ala Val Tyr Tyr Cys<br>Ala Arg Gly Ser His Tyr Phe Gly His Trp His Phe<br>Ala Val Trp Gly Gln Gly |
| 19 | Alternate lebrikizumab VH | QVTLRESGPA LVKPTQTLTL TCTVSGFSLS AYSVNWIRQP<br>PGKALEWLAM IWGDGKIVYN SALKSRLTIS KDTSKNQVVL<br>TMTNMDPVDT ATYYCAGDGY YPYAMDNWGQ GSLVTVSS |
| 20 | Alternate lebrikizumab VL | DIVMTQSPDS LSVSLGERAT INCRASKSVD SYGNSFMHWY<br>QQKPGQPPKL LIYLASNLES GVPDRFSGSG SGTDFTLTIS<br>SLQAEDVAVY YCQQNNEDPR TFGGGTKVEI KR |
| 21 | lebrikizumab VH Q1E | EVTLRESGPA LVKPTQTLTL TCTVSGFSLS AYSVNWIRQP<br>PGKALEWLAM IWGDGKIVYN SALKSRLTIS KDTSKNQVVL<br>TMTNMDPVDT ATYYCAGDGY YPYAMDNWGQ GSLVTVSS |
| 22 | lebrikizumab VL M4L | DIVLTQSPDS LSVSLGERAT INCRASKSVD SYGNSFMHWY<br>QQKPGQPPKL LIYLASNLES GVPDRFSGSG SGTDFTLTIS<br>SLQAEDVAVY YCQQNNEDPR TFGGGTKVEI KR |

**Claims**

1. A method of identifying a patient suffering from asthma as likely to respond to a therapy comprising lebrikizumab, the method comprising:

    (a) measuring the mRNA level of marker CSF1 in a biological sample from the patient;
    (b) comparing the mRNA level measured in (a) to a reference level; and
    (c) identifying the patient as more likely to respond to the therapy comprising lebrikizumab when the mRNA level measured in (a) is above the reference level.

2. The method according to claim 1, wherein measuring the mRNA levels comprises amplifying the mRNA and detecting the amplified product, thereby measuring the level of the mRNA.

3. The method according to claim 1 or 2, wherein the reference level is the median level of the respective marker in a reference population.

4. Lebrikizumab for use in the treatment of asthma in a patient, wherein a biological sample obtained from the patient has been determined to have elevated mRNA levels of marker CSF1, compared to the mRNA levels of the respective markers in a reference population.

5. Lebrikizumab for use in the treatment according to claim 4, wherein the mRNA levels were determined by a method comprising amplification.

6. Lebrikizumab for use in the treatment according to claim 5, wherein the mRNA levels were determined by a method comprising quantitative PCR.

7. Lebrikizumab for use in the treatment according to any one of claims 4 to 6, wherein the mRNA levels were determined by a method comprising amplifying the mRNA and detecting the amplified product.

8. Lebrikizumab for use in the treatment according to any one of claims 4 to 7, wherein the reference level is the median level of the respective marker in a reference population.

9. Lebrikizumab for use in the treatment according to claim 4, wherein the patient is administered a flat dose of 37.5 mg, or 125 mg or 250 mg every four weeks.

10. Lebrikizumab for use in the treatment according to claim 9, wherein lebrikizumab is administered subcutaneously.

11. Lebrikizumab for use in the treatment according to any one of claims 4 to 10, wherein if the mRNA level is elevated, the patient is Eosinophilic Inflammation Positive (EIP).

12. Lebrikizumab for use in the treatment according to any one of claims 4 to 11, wherein the patient is suffering from moderate to severe asthma.

13. Lebrikizumab for use in the treatment according to any one of claims 4 to 12, wherein the asthma is uncontrolled on a corticosteroid.

14. Lebrikizumab for use in the treatment according to claim 13, wherein the corticosteroid is an inhaled corticosteroid.

15. Lebrikizumab for use in the treatment according to claim 14, wherein the inhaled corticosteroid is Qvar®, Pulmicort®, Symbicort®, Aerobid®, Flovent®, Flonase®, Advair® or Azmacort®.

16. Lebrikizumab for use in the treatment according to any one of claims 4 to 15, wherein the patient is being treated with a second controller.

17. Lebrikizumab for use in the treatment according to claim 16, wherein the second controller is a long acting bronchial dilator (LABD).

18. Lebrikizumab for use in the treatment according to claim 17, wherein the LABD is a long-acting beta-2 agonist (LABA), leukotriene receptor antagonist (LTRA), long-acting muscarinic antagonist (LAMA), theophylline, or oral corticosteroids (OCS).

19. Lebrikizumabfor use in the treatment according to claim 18, wherein the LABD is Symbicort®, Advair®, Brovana®, Foradil®, Perforomist™ or Serevent®.

20. Lebrikizumabfor use in the treatment according to any one of claims 4 to 19, wherein the patient is 0-17 years old, 2-17 years old, 2-6 years old, 6-11 years old, 8-17 years old, 12-17 years old, 2 years old or older, 6 years old or older, or 12 years old or older.

21. Lebrikizumabfor use in the treatment according to any one of claims 4 to 20, wherein the biological sample is selected from blood, serum, plasma, and peripheral blood mononuclear cells (PBMCs)

22. Lebrikizumabfor use in the treatment according to claim 21, wherein the biological sample is PBMCs.


**Patentansprüche**

1. Verfahren zum Identifizieren eines an Asthma leidenden Patienten, der voraussichtlich auf eine Therapie, die Lebrikizumab umfasst, ansprechen wird, wobei das Verfahren Folgendes umfasst:

   (a) Messen des mRNA-Spiegels des Markers CSF1 in einer biologischen Probe des Patienten;
   (b) Vergleichen des in (a) gemessenen mRNA-Spiegels mit einem Referenzspiegel; und
   (c) Identifizieren des Patienten als einen, der mit höherer Wahrscheinlichkeit auf die Therapie, die Lebrikizumab umfasst, ansprechen wird, wenn der in (a) gemessene mRNA-Spiegel oberhalb des Referenzspiegels liegt.

2. Verfahren nach Anspruch 1, wobei das Messen der mRNA-Spiegel das Amplifizieren der mRNA und das Nachweisen des amplifizierten Produkts umfasst, wodurch der Spiegel der mRNA gemessen wird.

3. Verfahren nach Anspruch 1 oder 2, wobei der Referenzspiegel der mediane Spiegel des jeweiligen Markers in einer Referenzpopulation ist.

4. Lebrikizumab zur Verwendung in der Behandlung von Asthma bei einem Patienten, wobei bestimmt wurde, dass eine von dem Patienten gewonnene biologische Probe erhöhte mRNA-Spiegel des Markers CSF1 im Vergleich zu den mRNA-Spiegeln des jeweiligen Markers in einer Referenzpopulation aufweist.

5. Lebrikizumab zur Verwendung in der Behandlung nach Anspruch 4, wobei die mRNA-Spiegel durch ein Verfahren bestimmt wurden, das Amplifikation umfasst.

6. Lebrikizumab zur Verwendung in der Behandlung nach Anspruch 5, wobei die mRNA-Spiegel durch ein Verfahren bestimmt wurden, das quantitative PCR umfasst.

7. Lebrikizumab zur Verwendung in der Behandlung nach einem der Ansprüche 4 bis 6, wobei die mRNA-Spiegel durch ein Verfahren bestimmt wurden, das das Amplifizieren der mRNA und das Nachweisen des amplifizierten Produkts umfasst.

8. Lebrikizumab zur Verwendung in der Behandlung nach einem der Ansprüche 4 bis 7, wobei der Referenzspiegel der mediane Spiegel des jeweiligen Markers in einer Referenzpopulation ist.

9. Lebrikizumab zur Verwendung in der Behandlung nach Anspruch 4, wobei dem Patienten eine einheitliche Dosis von 37,5 mg oder 125 mg oder 250 mg alle vier Wochen verabreicht wird.

10. Lebrikizumab zur Verwendung in der Behandlung nach Anspruch 9, wobei Lebrikizumab subkutan verabreicht wird.

11. Lebrikizumab zur Verwendung in der Behandlung nach einem der Ansprüche 4 bis 10, wobei der Patient, wenn der mRNA-Spiegel erhöht ist, positiv für eosinophile Entzündung (EIP) ist.

12. Lebrikizumab zur Verwendung in der Behandlung nach einem der Ansprüche 4 bis 11, wobei der Patient an mittelschwerem bis schwerem Asthma leidet.

**13.** Lebrikizumab zur Verwendung in der Behandlung nach einem der Ansprüche 4 bis 12, wobei das Asthma unter einem Kortikosteroid unkontrolliert ist.

**14.** Lebrikizumab zur Verwendung in der Behandlung nach Anspruch 13, wobei das Kortikosteroid ein inhaliertes Kortikosteroid ist.

**15.** Lebrikizumab zur Verwendung in der Behandlung nach Anspruch 14, wobei das inhalierte Kortikosteroid Qvar®, Pulmicort®, Symbicort®, Aerobid®, Flovent®, Flonase®, Advair® oder Azmacort® ist.

**16.** Lebrikizumab zur Verwendung in der Behandlung nach einem der Ansprüche 4 bis 15, wobei der Patient mit einem zweiten Mittel behandelt wird.

**17.** Lebrikizumab zur Verwendung in der Behandlung nach Anspruch 16, wobei das zweite Mittel ein Bronchodilatator mit lang anhaltender Wirkung (LABD) ist.

**18.** Lebrikizumab zur Verwendung in der Behandlung nach Anspruch 17, wobei der LABD ein Beta-2-Agonist mit lang anhaltender Wirkung (LABA), ein Leukotrienrezeptorantagonist (LTRA), ein Muscarinantagonist mit lang anhaltender Wirkung (LAMA), Theophyllin oder orale Kortikosteroide (OCS) ist.

**19.** Lebrikizumab zur Verwendung in der Behandlung nach Anspruch 18, wobei der LABD Symbicort®, Advair®, Brovana®, Foradil®, Perforomist™ oder Serevent® ist.

**20.** Lebrikizumab zur Verwendung in der Behandlung nach einem der Ansprüche 4 bis 19, wobei der Patient 0-17 Jahre alt, 2-17 Jahre alt, 2-6 Jahre alt, 6-11 Jahre alt, 8-17 Jahre alt, 12-17 Jahre alt, 2 Jahre alt oder älter, 6 Jahre alt oder älter oder 12 Jahre alt oder älter ist.

**21.** Lebrikizumab zur Verwendung in der Behandlung nach einem der Ansprüche 4 bis 20, wobei die biologische Probe ausgewählt ist aus Blut, Serum, Plasma und mononukleären Zellen aus peripherem Blut (PBMC).

**22.** Lebrikizumab zur Verwendung in der Behandlung nach Anspruch 21, wobei die biologische Probe PBMC ist.

**Revendications**

**1.** Méthode d'identification d'un patient souffrant d'asthme comme étant susceptible de répondre à un traitement comprenant du lébrikizumab, la méthode comprenant :

(a) la mesure du taux d'ARNm du marqueur CSF1 dans un échantillon biologique provenant du patient ;
(b) la comparaison du taux d'ARNm mesuré en (a) à un taux de référence ; et
(c) l'identification du patient comme étant davantage susceptible de répondre au traitement comprenant du lébrikizumab lorsque le taux d'ARNm mesuré en (a) est supérieur au taux de référence.

**2.** Méthode selon la revendication 1, dans laquelle la mesure des taux d'ARNm comprend l'amplification de l'ARNm et la détection du produit amplifié, mesurant ainsi le taux d'ARNm.

**3.** Méthode selon la revendication 1 ou 2, dans laquelle le taux de référence est le taux médian du marqueur respectif dans une population de référence.

**4.** Lébrikizumab pour une utilisation dans le traitement de l'asthme chez un patient, dans lequel un échantillon biologique obtenu auprès du patient a été déterminé comme ayant des taux élevés d'ARNm du marqueur CSF1, comparé aux taux d'ARNm des marqueurs respectifs dans une population de référence.

**5.** Lébrikizumab pour une utilisation dans le traitement selon la revendication 4, dans lequel les taux d'ARNm ont été déterminés par une méthode comprenant l'amplification.

**6.** Lébrikizumab pour une utilisation dans le traitement selon la revendication 5, dans lequel les taux d'ARNm ont été déterminés par une méthode comprenant la PCR quantitative.

**7.** Lébrikizumab pour une utilisation dans le traitement selon l'une quelconque des revendications 4 à 6, dans lequel les taux d'ARNm ont été déterminés par une méthode comprenant l'amplification de l'ARNm et la détection du produit amplifié.

**8.** Lébrikizumab pour une utilisation dans le traitement selon l'une quelconque des revendications 4 à 7, dans lequel le taux de référence est le taux médian du marqueur respectif dans une population de référence.

**9.** Lébrikizumab pour une utilisation dans le traitement selon la revendication 4, dans lequel le patient se voit administrer une dose fixe de 37,5 mg, ou de 125 mg ou de 250 mg toutes les quatre semaines.

**10.** Lébrikizumab pour une utilisation dans le traitement selon la revendication 9, dans lequel le lébrikizumab est administré par voie sous-cutanée.

**11.** Lébrikizumab pour une utilisation dans le traitement selon l'une quelconque des revendications 4 à 10, dans lequel si le taux d'ARNm est élevé, le patient est positif à une inflammation éosinophilique (EIP).

**12.** Lébrikizumab pour une utilisation dans le traitement selon l'une quelconque des revendications 4 à 11, dans lequel le patient souffre d'asthme modéré à sévère.

**13.** Lébrikizumab pour une utilisation dans le traitement selon l'une quelconque des revendications 4 à 12, dans lequel l'asthme n'est pas contrôlé par un corticostéroïde.

**14.** Lébrikizumab pour une utilisation dans le traitement selon la revendication 13, dans lequel le corticostéroïde est un corticostéroïde inhalé.

**15.** Lébrikizumab pour une utilisation dans le traitement selon la revendication 14, dans lequel le corticostéroïde inhalé est Qvar®, Pulmicort®, Symbicort®, Aerobid®, Flovent®, Flonase®, Advair® ou Azmacort®.

**16.** Lébrikizumab pour une utilisation dans le traitement selon l'une quelconque des revendications 4 à 15, dans lequel le patient est traité avec un second contrôleur.

**17.** Lébrikizumab pour une utilisation dans le traitement selon la revendication 16, dans lequel le second contrôleur est un bronchodilatateur de longue durée d'action (BDLA).

**18.** Lébrikizumab pour une utilisation dans le traitement selon la revendication 17, le BDLA est un bêta-2 agoniste de longue durée d'action (LABA), un antagoniste des récepteurs des leucotriènes (LTRA), un antagoniste muscarinique de longue durée d'action (LAMA), la théophylline ou des corticostéroïdes oraux (CSO).

**19.** Lébrikizumab pour une utilisation dans le traitement selon la revendication 18, dans lequel le BDLA est Symbicort®, Advair®, Brovana®, Foradil®, Perforomist™ ou Serevent®.

**20.** Lébrikizumab pour une utilisation dans le traitement selon l'une quelconque des revendications 4 à 19, dans lequel le patient est âgé de 0 à 17 ans, de 2 à 17 ans, de 2 à 6 ans, de 6 à 11 ans, de 8 à 17 ans, de 12 à 17 ans, de 2 ans ou plus, de 6 ou plus ou de 12 ans ou plus.

**21.** Lébrikizumab pour une utilisation dans le traitement selon l'une quelconque des revendications 4 à 20, dans lequel l'échantillon biologique est choisi parmi le sang, le sérum, le plasma et les cellules mononucléées du sang périphérique (CMSP).

**22.** Lébrikizumab pour une utilisation dans le traitement selon la revendication 21, dans lequel l'échantillon biologique est des CMSP.

*FIG. 1A*

*FIG. 1B*

*FIG. 2B*

**FIG. 2A**

*FIG. 3A*

*FIG. 3B*

*FIG. 3C*

*FIG. 3D*

GSE3982 Expression for SIGLEC8

FIG. 4A

GSE3982 Expression for CLC

FIG. 4B

GSE3982 Expression for CSF1

*FIG. 4C*

GSE3982 Expression for OLIG2

*FIG. 4D*

FIG. 4E

FIG. 5

**FIG. 6A**

FIG. 6B

FIG. 6C

## GPR44

### GPR44 Above 50%ile

Legend: Lebrikizumab 250 mg SC (n=47) — Placebo (n=53)

Y-axis: Change in FEV1 (% Mean +- 95% CI)
X-axis: Time (Weeks)

## GPR44

### GPR44 Below 50%ile

Legend: Lebrikizumab 250 mg SC (n=51) — Placebo (n=49)

Y-axis: Change in FEV1 (% Mean +- 95% CI)
X-axis: Time (Weeks)

## FIG. 6D

## CSF1

### CSF1 Above 50%ile

### CSF1

### CSF1 Below 50%ile

## *FIG. 6E*

## MEIS2

### MEIS2 Above 50%ile

Legend: Lebrikizumab 250 mg SC (n=49) — Placebo (n=49)

Y-axis: Change in FEV1 (% Mean +- 95% CI)
X-axis: Time (Weeks)

## MEIS2

### MEIS2 Below 50%ile

Legend: Lebrikizumab 250 mg SC (n=48) — Placebo (n=53)

Y-axis: Change in FEV1 (% Mean +- 95% CI)
X-axis: Time (Weeks)

## FIG. 6F

FIG. 6G

FIG. 6H

**FIG. 7**

FIG. 8A

FIG. 8B

FIG. 8C

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012083132 A
- US 20130243750 A
- US 2007077585 A
- US 2012156194 A
- WO 2008086043 A
- WO 2005116088 A
- WO 2008086395 A
- WO 2006085938 A
- US 7615213 B
- US 7501121 B
- WO 2007080174 A
- US 7807788 B
- WO 2005007699 A
- WO 2007036745 A
- WO 2009009775 A
- WO 2007082068 A
- WO 2010073119 A
- WO 2007045477 A
- WO 2008134724 A
- US 20090047277 A
- WO 2008127271 A
- WO 2008116149 A
- WO 9316185 A
- US 5571894 A
- US 5587458 A
- US 5869046 A
- EP 404097 A
- WO 199301161 A
- US 6248516 B1
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A1
- US 4676980 A
- US 20060025576 A1
- US 20080069820 A
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 5500362 A
- WO 5821337 A
- WO 2006029879 A
- WO 2005100402 A
- US 6737056 B
- US 7332581 B
- WO 2004056312 A
- US 6194551 B
- WO 9951642 A
- US 20050014934 A1
- US 7371826 B
- US 5648260 A
- US 5624821 A
- WO 9429351 A
- US 7521541 B
- US 5648237 A
- US 5789199 A

- US 5840523 A
- US 5959177 A
- US 6040498 A
- US 6420548 B
- US 7125978 B
- US 6417429 B
- US 4016043 A
- US 4424279 A
- US 4018653 A
- US 4275149 A
- US 4737456 A

- US 4318980 A
- WO 0175166 A
- US 5700637 A
- US 5807522 A
- US 20050260186 A
- US 20060104968 A
- US 6267958 B
- US 6171586 B
- WO 2006044908 A
- WO 61894831 A

**Non-patent literature cited in the description**

- **CHANG et al.** *J Allergy Clin Immunol.,* 2006, vol. 117 (6), 1203-12
- **WINCHESTER et al.** *N. Engl. J. Med.,* 2006, vol. 355 (12), 1281-2
- **BRODLIE et al.** *Arch Dis Child.,* 2012, vol. 97 (7), 604-9
- **BOUSQUET et al.** *Chest,* 2004, vol. 125 (4), 1378-86
- **SCHULMAN, ES.** *Am J Respir Crit Care Med.,* 2001, vol. 164, 6-11
- **CHANG et al.** *Adv Immunol.,* 2007, vol. 93, 63-119
- **CORREN et al.** *N Engl J Med,* 2011, vol. 365, 1088-98
- **SCHEERENS et al.** *Am J Respir Crit Care Med,* 2012, vol. 185, A3960
- **JIA et al.** *J Allergy Clin Immunol,* 2012, vol. 130, 647-654 e10
- **HALDAR et al.** *N Engl J Med.,* 05 March 2009, vol. 360 (10), 973-84
- **NAIR et al.** *N Engl J Med.,* 05 March 2009, vol. 360 (10), 985-93
- **PAVORD et al.** *The Lancet,* vol. 380 (9842), 651-659
- *The New England Journal Of Medicine,* 22 September 2011, 1088-1098
- **AFFYMETRIX.** *A GeneChip Human Genome Arrays,* 01 January 2013
- **GAO, P-S et al.** *European Journal of Human Genetics,* 2010, vol. 18 (6), 713-719
- *CHEMICAL ABSTRACTS,* 196078-29-2
- *CHEMICAL ABSTRACTS,* 1044515-88-9
- *CHEMICAL ABSTRACTS,* 1044511-01-4
- **LIU et al.** *J Allergy Clin Immunol,* 2006, vol. 118, 496-503
- **SINGLETON et al.** Dictionary of Microbiology and Molecular Biology. J. Wiley & Sons, 1994
- Advanced Organic Chemistry Reactions. **MARCH.** Mechanisms and Structure. John Wiley & Sons, 1992

- *J Allergy Clin Immunol,* 2010, vol. 126 (5), 926-938
- **MUNITZ A ; LEVI-SCHAFFER F.** *Allergy,* 2004, vol. 59, 268-75
- **ADAMKO et al.** *Allergy,* 2005, vol. 60, 13-22
- **OLDHOFF et al.** *Allergy,* 2005, vol. 60, 693-6
- **JUNIPER et al.** *Am. J. Respir. Crit. Care Med.,* 2000, vol. 162 (4), 1330-1334
- **KABAT et al.** Sequences of Proteins of Immunological Interest. Health Service, National Institutes of Health, 1991
- **KABAT et al.** Sequences of Proteins of Immunological Interest. NIH Publication, 1991, vol. 1-3, 91-3242
- **FLATMAN et al.** *J. Chromatogr. B,* 2007, vol. 848, 79-87
- **KINDT et al.** Kuby Immunology. W.H. Freeman and Co, 2007
- **PORTOLANO et al.** *J. Immunol.,* 1993, vol. 150, 880-887
- **CLARKSON et al.** *Nature,* 1991, vol. 352, 624-628
- **CHEN et al.** *J. Mol. Biol.,* 1999, vol. 293, 865-881
- **PRESTA et al.** *Cancer Res.,* 1997, vol. 57, 4593-4599
- **HUDSON et al.** *Nat. Med.,* 2003, vol. 9, 129-134
- **PLUCKTHÜN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **ALMAGRO ; FRANSSON.** *Front. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34

- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **LI et al.** *Proc. Natl. Acad. Sci. USA,* 2006, vol. 103, 3557-3562
- *Ni, Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** Methods in Molecular Biology. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MILSTEIN ; CUELLO.** *Nature,* 1983, vol. 305, 537
- **TRAUNECKER et al.** *EMBO J.,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CHOWDHURY.** *Methods Mol. Biol.,* 2008, vol. 207, 179-196
- **HOOGENBOOM et al.** *Methods in Molecular Biology,* vol. 178, 1-37
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- **KAM et al.** *Proc. Natl. Acad. Sci. USA,* 2005, vol. 102, 11600-11605
- **CHARLTON.** Methods in Molecular Biology. Humana Press, 2003, vol. 248, 245-254

- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- *Mather, Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- **COLIGEN et al.** Current Protocols in Immunology. Wiley-Interscience, 1991, vol. 1, 2
- Methods for the Preparation of Enzyme-Antibody Conjugates for use in Enzyme Immunoassay. **O'SULLIVAN et al.** Methods in Enzym. Academic press, 1981, vol. 73, 147-166
- **LOCKART.** *Nature Biotechnology,* 1996, vol. 14, 1675-1680
- **CHEUNG, V.G. et al.** *Nature Genetics,* 1999, vol. 21, 15-19
- Remington's Pharmaceutical Sciences. 1980
- **GONLUGUR.** *Immunol. Invest.,* 2006, vol. 35 (1), 29-45
- **KUDLACZ et al.** *Inflammation,* 2002, vol. 26, 111-119
- **RZANY et al.** *Br. J. Dermatol.,* 1996, vol. 135, 6-11
- **NIELSEN et al.** *Ann. Allergy Asthma Immunol.,* 2001, vol. 85, 489-494
- **MIOSSEC.** *J. Clin. Rheumatol.,* 1997, vol. 3, 81-83
- **ATANES et al.** *Scand. J. Rheumatol.,* 1996, vol. 25, 183-185
- **YETISER et al.** *Int. J. Pediatr. Otorhinolaryngol.,* 2002, vol. 62, 169-173
- **LAKHANPAL et al.** *Semin. Arthritis Rheum.,* 1988, vol. 17, 331-231
- **ENGINEER et al.** *Cytokine,* 2001, vol. 13, 32-38
- **CHEN et al.** *J. Am. Acad. Dermatol.,* 1996, vol. 35, 173-182
- **FELTELIUS et al.** *Ann. Rheum. Dis.,* 1987, vol. 46, 403-407
- **KRUPSKY et al.** *Chest,* 1993, vol. 104, 1290-1292
- **FALANGA et al.** *J. Am. Acad. Dermatol.,* 1987, vol. 17, 648-656
- **DESCHRYVER-KECSKEMETI et al.** *Arch. Pathol. Lab Med.,* 1989, vol. 113, 394-398
- **VARGA et al.** *Curr. Opin. Rheumatol.,* 1997, vol. 9, 562-570
- **BOUROS et al.** *Am. J. Respir. Crit. Care Med.,* 2002, vol. 165, 1581-1586
- **HERNNAS et al.** *Eur. J. Cell Biol.,* 1992, vol. 59, 352-363
- **VANCHERI et al.** *Am. J. Respir. Cell Mol. Biol.,* 1989, vol. 1, 289-214
- **BACHERCT et al.** *J. allergy Clin. Immunol.,* 2001, vol. 107, 607-614
- **ARGUELLES et al.** *Arch. Intern. Med.,* 1983, vol. 143, 570-571
- **ASSARIAN et al.** *Hum. Pathol.,* 1985, vol. 16, 311-312
- **LI et al.** *Ann. Allergy,* 1992, vol. 69, 101-105
- **ARRON et al.** *Adv Pharmacol,* 2013, vol. 66, 1-49
- **ARRON et al.** Annals Am. Thoracic Soc. in press, 2013
- **HORIUCHI et al.** *J Bone Miner Res,* 1999, vol. 14, 1239-49
- **CONTIE et al.** *Calcif Tissue Int,* 2010, vol. 87, 341-5
- **BARALDO et al.** *Eur Respir J,* 2011, vol. 38, 575-83
- **HANANIA et al.** *Am. J. RCCM,* 2013, vol. 187, 804-811
- **HANANIA et al.** *Ann. Intern. Med.,* 2011, vol. 154, 573-582
- **HANANIA et al.** *Am J Respir Crit Care Med,* 2013, vol. 187, 804-11
- **HANANIA et al.** *Ann Intern Med,* 2011, vol. 154, 573-82
- **MILGROM et al.** *Pediatrics,* 2001, vol. 108, E36
- **MILGROM et al.** *N Engl J Med,* 1999, vol. 341, 1966-73
- **BUSSE et al.** *J Allergy Clin Immunol,* 2001, vol. 108, 184-90
- **HOLGATE et al.** *Clin Exp Allergy,* 2004, vol. 34, 632-8
- **SOLER et al.** *Eur Respir J,* 2001, vol. 18, 254-61
- **CONTIE et al.** *Calcif Tissue Int,* 2010, vol. 87, 341-50
- **WEN et al.** *Proc Natl Acad Sci U S A,* 2013, vol. 110, 6067-72
- **ARRON et al.** *N Engl J Med,* 2011, vol. 365, 2433-34
- **NISHIMURA et al.** *Am J Respir Crit Care Med,* 2013, vol. 188, 309-18
- **KUMAR et al.** *J Allergy Clin Immunol,* 2013
- **BORRELL et al.** *Am J Respir Crit Care Med,* 2013, vol. 187, 697-702
- **CORVOL et al.** *Pharmacogenet Genomics,* July 2009, vol. 19 (7), 489-96
- **ARRON, JR ; H SCHEERENS ; JG MATTHEWS.** Redefining approaches to asthma: developing targeted biologic therapies. *Adv Pharmacol,* 2013, vol. 66, 1-49
- **JIA, G ; RW ERICKSON ; DF CHOY ; S MOSESOVA ; LC WU et al.** Periostin is a systemic biomarker of eosinophilic airway inflammation in asthmatic patients. *J Allergy Clin Immunol,* 2012, vol. 130, 647-654 e10

- **CORREN, J ; RF LEMANSKE ; NA HANANIA ; PE KORENBLAT ; MV PARSEY et al.** Lebrikizumab treatment in adults with asthma. *N Engl J Med,* 2011, vol. 365, 1088-98
- **SCHEERENS, H ; JR ARRON ; DF CHOY ; S MOSESOVA ; P LAL et al.** Lebrikizumab Treatment Reduces Serum Periostin Levels In Asthma Patients With Elevated Baseline Levels Of Periostin. *Am J Respir Crit Care Med,* 2012, vol. 185, A3960
- **HORIUCHI, K ; N AMIZUKA ; S TAKESHITA ; H TAKAMATSU ; M KATSUURA et al.** Identification and characterization of a novel protein, periostin, with restricted expression to periosteum and periodontal ligament and increased expression by transforming growth factor beta. *J Bone Miner Res,* 1999, vol. 14, 1239-49
- **CONTIE, S ; N VOORZANGER-ROUSSELOT ; J LITVIN ; N BONNET ; S FERRARI et al.** Development of a new ELISA for serum periostin: evaluation of growth-related changes and bisphosphonate treatment in mice. *Calcif Tissue Int,* 2010, vol. 87, 341-50
- **BARALDO, S ; G TURATO ; E BAZZAN ; A BALLARIN ; M DAMIN et al.** Noneosinophilic asthma in children: relation with airway remodelling. *Eur Respir J,* 2011, vol. 38, 575-83
- **HANANIA, NA ; S WENZEL ; K ROSEN ; HJ HSIEH ; S MOSESOVA et al.** Exploring the effects of omalizumab in allergic asthma: an analysis of biomarkers in the EXTRA study. *Am J Respir Crit Care Med,* 2013, vol. 187, 804-11
- **HANANIA, NA ; O ALPAN ; DL HAMILOS ; JJ CONDEMI ; I REYES-RIVERA et al.** Omalizumab in severe allergic asthma inadequately controlled with standard therapy: a randomized trial. *Ann Intern Med,* 2011, vol. 154, 573-82
- **MILGROM, H ; W BERGER ; A NAYAK ; N GUPTA ; S POLLARD et al.** Treatment of childhood asthma with anti-immunoglobulin E antibody (omalizumab). *Pediatrics,* 2001, vol. 108, E36
- **MILGROM, H ; RB FICK, JR. ; JQ SU ; JD REIMANN ; RK BUSH et al.** Treatment of allergic asthma with monoclonal anti-IgE antibody. rhuMAb-E25 Study Group. *N Engl J Med,* 1999, vol. 341, 1966-73
- **BUSSE, W ; J CORREN ; BQ LANIER ; M MCALARY ; A FOWLER-TAYLOR et al.** Omalizumab, anti-IgE recombinant humanized monoclonal antibody, for the treatment of severe allergic asthma. *J Allergy Clin Immunol,* 2001, vol. 108, 184-90
- **HOLGATE, ST ; AG CHUCHALIN ; J HEBERT ; J LOTVALL ; GB PERSSON et al.** Efficacy and safety of a recombinant anti-immunoglobulin E antibody (omalizumab) in severe allergic asthma. *Clin Exp Allergy,* 2004, vol. 34, 632-8
- **SOLER, M ; J MATZ ; R TOWNLEY ; R BUHL ; J O'BRIEN et al.** The anti-IgE antibody omalizumab reduces exacerbations and steroid requirement in allergic asthmatics. *Eur Respir J,* 2001, vol. 18, 254-61
- **WEN, T ; JA BESSE ; MK MINGLER ; PC FULKERSON ; ME ROTHENBERG.** Eosinophil adoptive transfer system to directly evaluate pulmonary eosinophil trafficking in vivo. *Proc Natl Acad Sci U S A,* 2013, vol. 110, 6067-72
- **LIU, SM ; R XAVIER ; KL GOOD ; T CHTANOVA ; R NEWTON et al.** Immune cell transcriptome datasets reveal novel leukocyte subset-specific genes and genes associated with allergic processes. *J Allergy Clin Immunol,* 2006, vol. 118, 496-503
- **FLOYD, H ; J NI ; AL CORNISH ; Z ZENG ; D LIU et al.** Siglec-8. A novel eosinophil-specific member of the immunoglobulin superfamily. *J Biol Chem,* 2000, vol. 275, 861-6
- **TAVERNIER, J ; R DEVOS ; S CORNELIS ; T TUYPENS ; J VAN DER HEYDEN et al.** A human high affinity interleukin-5 receptor (IL5R) is composed of an IL5-specific alpha chain and a beta chain shared with the receptor for GM-CSF. *Cell,* 1991, vol. 66, 1175-84
- **NAGATA, K ; H HIRAI ; K TANAKA ; K OGAWA ; T ASO et al.** CRTH2, an orphan receptor of T-helper-2-cells, is expressed on basophils and eosinophils and responds to mast cell-derived factor(s). *FEBS Lett,* 1999, vol. 459, 195-9
- **MITA, H ; M HASEGAWA ; H SAITO ; K AKIYAMA.** Levels of cysteinyl leukotriene receptor mRNA in human peripheral leucocytes: significantly higher expression of cysteinyl leukotriene receptor 2 mRNA in eosinophils. *Clin Exp Allergy,* 2001, vol. 31, 1714-23
- **TURK, J ; RL MAAS ; AR BRASH ; LJ ROBERTS ; JA OATES.** Arachidonic acid 15-lipoxygenase products from human eosinophils. *J Biol Chem,* 1982, vol. 257, 7068-76
- **GLEICH, GJ ; DA LOEGERING ; KG MANN ; JE MALDONADO.** Comparative properties of the Charcot-Leyden crystal protein and the major basic protein from human eosinophils. *J Clin Invest,* 1976, vol. 57, 633-40
- **PONATH, PD ; TW POST ; J WANG ; L WU et al.** Molecular cloning and characterization of a human eotaxin receptor expressed selectively on eosinophils. *J Exp Med,* 1996, vol. 183, 2437-48
- **PONATH, PD ; S QIN ; DJ RINGLER ; I CLARK-LEWIS ; J WANG et al.** Cloning of the human eosinophil chemoattractant, eotaxin. Expression, receptor binding, and functional properties suggest a mechanism for the selective recruitment of eosinophils. *J Clin Invest,* 1996, vol. 97, 604-12

- **KITAURA, M ; T NAKAJIMA ; T IMAI ; S HARADA ; C COMBADIERE et al.** Molecular cloning of human eotaxin, an eosinophil-selective CC chemokine, and identification of a specific eosinophil eotaxin receptor, CC chemokine receptor 3. *J Biol Chem,* 1996, vol. 271, 7725-30
- **DAUGHERTY, BL ; SJ SICILIANO ; JA DEMARTINO ; L MALKOWITZ ; A SIROTINA et al.** Cloning, expression, and characterization of the human eosinophil eotaxin receptor. *J Exp Med,* 1996, vol. 183, 2349-54
- **ROTHENBERG, ME ; R OWNBEY ; PD MEHLHOP ; PM LOISELLE ; M VAN DE RIJN et al.** Eotaxin triggers eosinophil-selective chemotaxis and calcium flux via a distinct receptor and induces pulmonary eosinophilia in the presence of interleukin 5 in mice. *Mol Med,* 1996, vol. 2, 334-48
- **COMBADIERE, C ; SK AHUJA ; PM MURPHY.** Cloning and functional expression of a human eosinophil CC chemokine receptor. *J Biol Chem,* 1996, vol. 271, 11034
- **HANSEL, TT ; JB BRAUNSTEIN ; C WALKER ; K BLASER ; PL BRUIJNZEEL et al.** Sputum eosinophils from asthmatics express ICAM-1 and HLA-DR. *Clin Exp Immunol,* 1991, vol. 86, 271-7
- **KOHNO, Y ; X JI ; SD MAWHORTER ; M KOSHIBA ; KA JACOBSON.** Activation of A3 adenosine receptors on human eosinophils elevates intracellular calcium. *Blood,* 1996, vol. 88, 3569-74
- **HAMANN, KJ ; RM TEN ; DA LOEGERING ; RB JENKINS ; MT HEISE et al.** Structure and chromosome localization of the human eosinophil-derived neurotoxin and eosinophil cationic protein genes: evidence for intronless coding sequences in the ribonuclease gene superfamily. *Genomics,* 1990, vol. 7, 535-46
- **NOVAK, H ; A MULLER ; N HARRER ; C GUNTHER ; M CARBALLIDO et al.** CCL23 expression is induced by IL-4 in a STAT6-dependent fashion. *J Immunol,* 2007, vol. 178, 4335-41
- **SYED, F ; CC HUANG ; K LI ; V LIU ; T SHANG et al.** Identification of interleukin-13 related biomarkers using peripheral blood mononuclear cells. *Biomarkers,* 2007, vol. 12, 414-23
- **HAMILTON, JA.** Colony-stimulating factors in inflammation and autoimmunity. *Nat Rev Immunol,* 2008, vol. 8, 533-44
- **VON BUBNOFF, D ; T BIEBER.** The indoleamine 2,3-dioxygenase (IDO) pathway controls allergy. *Allergy,* 2012, vol. 67, 718-25
- **ESNAULT, S ; EA KELLY ; EA SCHWANTES ; LY LIU ; LP DELAIN et al.** Identification of genes expressed by human airway eosinophils after an in vivo allergen challenge. *PLoS One,* 2013, vol. 8, e67560
- **LEE, JS ; JH KIM ; JS BAE ; JY KIM ; TJ PARK et al.** Association of CACNG6 polymorphisms with aspirin-intolerance asthmatics in a Korean population. *BMC Med Genet,* 2010, vol. 11, 138
- **BURGESS, DL ; LA GEFRIDES ; PJ FOREMAN ; JL NOEBELS.** A cluster of three novel Ca2+ channel gamma subunit genes on chromosome 19q13.4: evolution and expression profile of the gamma subunit gene family. *Genomics,* 2001, vol. 71, 339-50
- **SCHMITZ, J ; A OWYANG ; E OLDHAM ; Y SONG ; E MURPHY et al.** IL-33, an interleukin-1-like cytokine that signals via the IL-1 receptor-related protein ST2 and induces T helper type 2-associated cytokines. *Immunity,* 2005, vol. 23, 479-90
- **GUDBJARTSSON, DF ; US BJORNSDOTTIR ; E HALAPI ; A HELGADOTTIR ; P SULEM et al.** Sequence variants affecting eosinophil numbers associate with asthma and myocardial infarction. *Nat Genet,* 2009, vol. 41, 342-7
- **MEIJER, DH ; MF KANE ; S MEHTA ; H LIU ; E HARRINGTON et al.** Separated at birth? The functional and molecular divergence of OLIG1 and OLIG2. *Nat Rev Neurosci,* 2012, vol. 13, 819-31
- **QUARLES, RH.** Myelin sheaths: glycoproteins involved in their formation, maintenance and degeneration. *Cell Mol Life Sci,* 2002, vol. 59, 1851-71
- **ARRON, JR ; J CORREN ; JG MATTHEWS.** Author response to correspondence on ''Lebrikizumab treatment in adults with asthma. *N Engl J Med,* 2011, vol. 365, 2433-34
- **NISHIMURA, KK ; JM GALANTER ; LA ROTH ; SS OH ; N THAKUR et al.** Early-Life Air Pollution and Asthma Risk in Minority Children. The GALA II and SAGE II Studies. *Am J Respir Crit Care Med,* 2013, vol. 188, 309-18
- **KUMAR, R ; EA NGUYEN ; LA ROTH ; SS OH ; CR GIGNOUX et al.** Factors associated with degree of atopy in Latino children in a nationwide pediatric sample: The Genes-environments and Admixture in Latino Asthmatics (GALA II) study. *J Allergy Clin Immunol,* 2013
- **BORRELL, LN ; EA NGUYEN ; LA ROTH ; SS OH ; H TCHEUREKDJIAN et al.** Childhood obesity and asthma control in the GALA II and SAGE II studies. *Am J Respir Crit Care Med,* 2013, vol. 187, 697-702
- **AOKI, T ; Y MATSUMOTO ; K HIRATA ; K OCHIAI ; M OKADA et al.** Expression profiling of genes related to asthma exacerbations. *Clin Exp Allergy,* 2009, vol. 39, 213-221

SEQUENCE LISTING

<110> GENENTECH, INC. ET AL.

<120> METHODS OF DIAGNOSING AND TREATING EOSINOPHILIC DISORDERS

<130> P5689R1-WO

<140>
<141>

<150> 61/894,831
<151> 2013-10-23

<160> 67

<170> PatentIn version 3.5

<210> 1
<211> 117
<212> PRT
<213> Artificial Sequence

<220>
<223> Description of Artificial Sequence: Synthetic polypeptide

<400> 1


Glu Val Gln Leu Val Glu Ser Gly Gly Gly Leu Val Gln Pro Gly Gly
1               5                  10                 15

Ser Leu Arg Leu Ser Cys Ala Ala Ser Gly Phe Thr Phe Ser Asp Tyr
            20                  25                  30

Gly Ile Ala Trp Val Arg Gln Ala Pro Gly Lys Gly Leu Glu Trp Val
            35                  40                  45

Ala Phe Ile Ser Asp Leu Ala Tyr Thr Ile Tyr Tyr Ala Asp Thr Val
        50                  55                  60

Thr Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Asn Trp Asp Ala Met Asp Tyr Trp Gly Gln Gly Thr Leu
            100                 105                 110

Val Thr Val Ser Ser
            115

<210> 2
<211> 112
<212> PRT
<213> Artificial Sequence

<220>
<223> Description of Artificial Sequence: Synthetic polypeptide

<400> 2

```
Asp Ile Gln Met Thr Gln Ser Pro Ser Ser Leu Ser Ala Ser Val Gly
1               5               10                  15

Asp Arg Val Thr Ile Thr Cys Arg Ser Ser Gln Ser Leu Val His Asn
            20                  25                  30

Asn Ala Asn Thr Tyr Leu His Trp Tyr Gln Gln Lys Pro Gly Lys Ala
            35                  40                  45

Pro Lys Leu Leu Ile Tyr Lys Val Ser Asn Arg Phe Ser Gly Val Pro
        50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Ser Gln Asn
                85                  90                  95

Thr Leu Val Pro Trp Thr Phe Gly Gln Gly Thr Lys Val Glu Ile Lys
                100                 105                 110
```

<210> 3
<211> 10
<212> PRT
<213> Artificial Sequence

<220>
<223> Description of Artificial Sequence: Synthetic peptide

<400> 3

```
Gly Phe Thr Phe Ser Asp Tyr Gly Ile Ala
1               5               10
```

<210> 4
<211> 18
<212> PRT
<213> Artificial Sequence

<220>
<223> Description of Artificial Sequence: Synthetic peptide

<400> 4

```
Ala Phe Ile Ser Asp Leu Ala Tyr Thr Ile Tyr Tyr Ala Asp Thr Val
1               5               10                  15

Thr Gly
```

<210> 5
<211> 10
<212> PRT

<213> Artificial Sequence

<220>
<223> Description of Artificial Sequence: Synthetic peptide

<400> 5

```
            Ala Arg Asp Asn Trp Asp Ala Met Asp Tyr
            1               5               10
```

<210> 6
<211> 16
<212> PRT
<213> Artificial Sequence

<220>
<223> Description of Artificial Sequence: Synthetic peptide

<400> 6

```
        Arg Ser Ser Gln Ser Leu Val His Asn Asn Ala Asn Thr Tyr Leu His
        1           5               10                  15
```

<210> 7
<211> 7
<212> PRT
<213> Artificial Sequence

<220>
<223> Description of Artificial Sequence: Synthetic peptide

<400> 7

```
                    Lys Val Ser Asn Arg Phe Ser
                    1               5
```

<210> 8
<211> 9
<212> PRT
<213> Artificial Sequence

<220>
<223> Description of Artificial Sequence: Synthetic peptide

<400> 8

```
                Ser Gln Asn Thr Leu Val Pro Trp Thr
                1               5
```

<210> 9
<211> 117
<212> PRT
<213> Artificial Sequence

<220>
<223> Description of Artificial Sequence: Synthetic polypeptide

<400> 9

```
Val Thr Leu Arg Glu Ser Gly Pro Ala Leu Val Lys Pro Thr Gln Thr
1               5                   10                  15

Leu Thr Leu Thr Cys Thr Val Ser Gly Phe Ser Leu Ser Ala Tyr Ser
            20                  25                  30

Val Asn Trp Ile Arg Gln Pro Pro Gly Lys Ala Leu Glu Trp Leu Ala
            35                  40                  45

Met Ile Trp Gly Asp Gly Lys Ile Val Tyr Asn Ser Ala Leu Lys Ser
    50                  55                  60

Arg Leu Thr Ile Ser Lys Asp Thr Ser Lys Asn Gln Val Val Leu Thr
65                  70                  75                  80

Met Thr Asn Met Asp Pro Val Asp Thr Ala Thr Tyr Tyr Cys Ala Gly
                85                  90                  95

Asp Gly Tyr Tyr Pro Tyr Ala Met Asp Asn Trp Gly Gln Gly Ser Leu
            100                 105                 110

Val Thr Val Ser Ser
            115
```

<210> 10
<211> 111
<212> PRT
<213> Artificial Sequence

<220>

<223> Description of Artificial Sequence: Synthetic polypeptide

<400> 10

```
Asp Ile Val Met Thr Gln Ser Pro Asp Ser Leu Ser Val Ser Leu Gly
1               5                   10                  15

Glu Arg Ala Thr Ile Asn Cys Arg Ala Ser Lys Ser Val Asp Ser Tyr
            20                  25                  30

Gly Asn Ser Phe Met His Trp Tyr Gln Gln Lys Pro Gly Gln Pro Pro
            35                  40                  45

Lys Leu Leu Ile Tyr Leu Ala Ser Asn Leu Glu Ser Gly Val Pro Asp
    50                  55                  60
```

74

```
Arg Phe Ser Gly Ser Gly Ser Gly Thr Asp Phe Thr Leu Thr Ile Ser
65              70              75                  80


Ser Leu Gln Ala Glu Asp Val Ala Val Tyr Tyr Cys Gln Gln Asn Asn
                85              90                  95


Glu Asp Pro Arg Thr Phe Gly Gly Gly Thr Lys Val Glu Ile Lys
            100             105             110
```

<210> 11
<211> 5
<212> PRT
<213> Artificial Sequence

<220>
<223> Description of Artificial Sequence: Synthetic peptide

<400> 11

```
Ala Tyr Ser Val Asn
1               5
```

<210> 12
<211> 16
<212> PRT
<213> Artificial Sequence

<220>
<223> Description of Artificial Sequence: Synthetic peptide

<400> 12

```
Met Ile Trp Gly Asp Gly Lys Ile Val Tyr Asn Ser Ala Leu Lys Ser
1           5               10              15
```

<210> 13
<211> 10
<212> PRT
<213> Artificial Sequence

<220>
<223> Description of Artificial Sequence: Synthetic peptide

<400> 13

```
Asp Gly Tyr Tyr Pro Tyr Ala Met Asp Asn
1           5               10
```

<210> 14
<211> 15
<212> PRT
<213> Artificial Sequence

<220>
<223> Description of Artificial Sequence: Synthetic peptide

<400> 14

```
Arg Ala Ser Lys Ser Val Asp Ser Tyr Gly Asn Ser Phe Met His
1               5                   10                  15
```

<210> 15
<211> 7
<212> PRT
<213> Artificial Sequence

<220>
<223> Description of Artificial Sequence: Synthetic peptide

<400> 15

```
Leu Ala Ser Asn Leu Glu Ser
1               5
```

<210> 16
<211> 9
<212> PRT
<213> Artificial Sequence

<220>
<223> Description of Artificial Sequence: Synthetic peptide

<400> 16

```
Gln Gln Asn Asn Glu Asp Pro Arg Thr
1               5
```

<210> 17
<211> 114
<212> PRT
<213> Artificial Sequence

<220>
<223> Description of Artificial Sequence: Synthetic polypeptide

<400> 17

```
Asp Ile Gln Leu Thr Gln Ser Pro Ser Ser Leu Ser Ala Ser Val Gly
1               5                   10                  15


Asp Arg Val Thr Ile Thr Cys Arg Ala Ser Gln Ser Val Asp Tyr Asp
            20                  25                  30


Gly Asp Ser Tyr Met Asn Trp Tyr Gln Gln Lys Pro Gly Lys Ala Pro
            35                  40                  45


Lys Leu Leu Ile Tyr Ala Ala Ser Tyr Leu Glu Ser Gly Val Pro Ser
        50                  55                  60


Arg Phe Ser Gly Ser Gly Ser Gly Thr Asp Phe Thr Leu Thr Ile Ser
65                  70                  75                  80
```

```
Ser Leu Gln Pro Glu Asp Phe Ala Thr Tyr Tyr Cys Gln Gln Ser His
                85                  90                  95

Glu Asp Pro Tyr Thr Phe Gly Gln Gly Thr Lys Val Glu Ile Lys Arg
                100                 105                 110

Thr Val
```

<210> 18
<211> 114
<212> PRT
<213> Artificial Sequence

<220>
<223> Description of Artificial Sequence: Synthetic polypeptide

<400> 18

```
Glu Val Gln Leu Val Glu Ser Gly Gly Gly Leu Val Gln Pro Gly Gly
1               5               10                  15

Ser Leu Arg Leu Ser Cys Ala Val Ser Gly Tyr Ser Ile Thr Ser Gly
                20                  25                  30

Tyr Ser Trp Asn Trp Ile Arg Gln Ala Pro Gly Lys Gly Leu Glu Trp
                35                  40                  45

Val Ala Ser Ile Thr Tyr Asp Gly Ser Thr Asn Tyr Asn Pro Ser Val
                50                  55                  60

Lys Gly Arg Ile Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ser His Tyr Phe Gly His Trp His Phe Ala Val Trp Gly
                100                 105                 110

Gln Gly
```

<210> 19
<211> 118
<212> PRT
<213> Artificial Sequence

<220>
<223> Description of Artificial Sequence: Synthetic polypeptide

<400> 19

```
Gln Val Thr Leu Arg Glu Ser Gly Pro Ala Leu Val Lys Pro Thr Gln
1                   5                   10                  15

Thr Leu Thr Leu Thr Cys Thr Val Ser Gly Phe Ser Leu Ser Ala Tyr
            20                  25                  30

Ser Val Asn Trp Ile Arg Gln Pro Pro Gly Lys Ala Leu Glu Trp Leu
            35                  40                  45

Ala Met Ile Trp Gly Asp Gly Lys Ile Val Tyr Asn Ser Ala Leu Lys
    50                  55                  60

Ser Arg Leu Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Gly Asp Gly Tyr Tyr Pro Tyr Ala Met Asp Asn Trp Gly Gln Gly Ser
            100                 105                 110

Leu Val Thr Val Ser Ser
            115
```

<210> 20
<211> 112
<212> PRT
<213> Artificial Sequence

<220>

<223> Description of Artificial Sequence: Synthetic polypeptide

<400> 20

```
Asp Ile Val Met Thr Gln Ser Pro Asp Ser Leu Ser Val Ser Leu Gly
1                   5                   10                  15

Glu Arg Ala Thr Ile Asn Cys Arg Ala Ser Lys Ser Val Asp Ser Tyr
            20                  25                  30

Gly Asn Ser Phe Met His Trp Tyr Gln Gln Lys Pro Gly Gln Pro Pro
            35                  40                  45

Lys Leu Leu Ile Tyr Leu Ala Ser Asn Leu Glu Ser Gly Val Pro Asp
    50                  55                  60

Arg Phe Ser Gly Ser Gly Ser Gly Thr Asp Phe Thr Leu Thr Ile Ser
65                  70                  75                  80
```

```
        Ser Leu Gln Ala Glu Asp Val Ala Val Tyr Tyr Cys Gln Gln Asn Asn
                    85                  90                  95


        Glu Asp Pro Arg Thr Phe Gly Gly Gly Thr Lys Val Glu Ile Lys Arg
                    100                 105                 110
```

<210> 21
<211> 118
<212> PRT
<213> Artificial Sequence

<220>
<223> Description of Artificial Sequence: Synthetic polypeptide

<400> 21

```
        Glu Val Thr Leu Arg Glu Ser Gly Pro Ala Leu Val Lys Pro Thr Gln
        1               5                   10                  15


        Thr Leu Thr Leu Thr Cys Thr Val Ser Gly Phe Ser Leu Ser Ala Tyr
                    20                  25                  30


        Ser Val Asn Trp Ile Arg Gln Pro Pro Gly Lys Ala Leu Glu Trp Leu
                    35                  40                  45


        Ala Met Ile Trp Gly Asp Gly Lys Ile Val Tyr Asn Ser Ala Leu Lys
                    50                  55                  60


        Ser Arg Leu Thr Ile Ser Lys Asp Thr Ser Lys Asn Gln Val Val Leu
        65                  70                  75                  80


        Thr Met Thr Asn Met Asp Pro Val Asp Thr Ala Thr Tyr Tyr Cys Ala
                    85                  90                  95


        Gly Asp Gly Tyr Tyr Pro Tyr Ala Met Asp Asn Trp Gly Gln Gly Ser
                    100                 105                 110


        Leu Val Thr Val Ser Ser
                    115
```

<210> 22
<211> 112
<212> PRT
<213> Artificial Sequence

<220>
<223> Description of Artificial Sequence: Synthetic polypeptide

<400> 22

79

```
Asp Ile Val Leu Thr Gln Ser Pro Asp Ser Leu Ser Val Ser Leu Gly

1               5               10              15


Glu Arg Ala Thr Ile Asn Cys Arg Ala Ser Lys Ser Val Asp Ser Tyr
            20              25              30


Gly Asn Ser Phe Met His Trp Tyr Gln Gln Lys Pro Gly Gln Pro Pro
            35              40              45


Lys Leu Leu Ile Tyr Leu Ala Ser Asn Leu Glu Ser Gly Val Pro Asp
        50              55              60


Arg Phe Ser Gly Ser Gly Ser Gly Thr Asp Phe Thr Leu Thr Ile Ser
65              70              75              80


Ser Leu Gln Ala Glu Asp Val Ala Val Tyr Tyr Cys Gln Gln Asn Asn
            85              90              95


Glu Asp Pro Arg Thr Phe Gly Gly Gly Thr Lys Val Glu Ile Lys Arg
            100             105             110
```

<210> 23
<211> 25
<212> DNA
<213> Artificial Sequence

<220>
<223> Description of Artificial Sequence: Synthetic oligonucleotide

<400> 23
aagaacgcca atggtgtcct ctccc          25

<210> 24
<211> 25
<212> DNA
<213> Artificial Sequence

<220>
<223> Description of Artificial Sequence: Synthetic oligonucleotide

<400> 24
cacaccatta gtgttggaca agtgg          25

<210> 25
<211> 25
<212> DNA
<213> Artificial Sequence

<220>
<223> Description of Artificial Sequence: Synthetic oligonucleotide

<400> 25

cctatcttca agcttataat tcccc          25


<210> 26
<211> 25
<212> DNA
<213> Artificial Sequence


<220>
<223> Description of Artificial Sequence: Synthetic oligonucleotide


<400> 26

ttagccaagt acggtgctga catca          25


<210> 27
<211> 25
<212> DNA
<213> Artificial Sequence


<220>
<223> Description of Artificial Sequence: Synthetic oligonucleotide


<400> 27

cacttgccaa gccatcaagt tctct          25


<210> 28
<211> 25
<212> DNA
<213> Artificial Sequence


<220>
<223> Description of Artificial Sequence: Synthetic oligonucleotide


<400> 28

ggagctgccc ggagaagcaa actgc          25


<210> 29
<211> 25
<212> DNA
<213> Artificial Sequence


<220>
<223> Description of Artificial Sequence: Synthetic oligonucleotide


<400> 29

ggtgtcatct tcctcaccaa gaagg          25


<210> 30
<211> 25
<212> DNA
<213> Artificial Sequence


<220>
<223> Description of Artificial Sequence: Synthetic oligonucleotide


<400> 30

tgtgcccccg ctgtactccc tggtg          25

<210> 31
<211> 25
<212> DNA
<213> Artificial Sequence

<220>
<223> Description of Artificial Sequence: Synthetic oligonucleotide

<400> 31
tctacacagg agtctatatt ctgat        25

<210> 32
<211> 25
<212> DNA
<213> Artificial Sequence

<220>
<223> Description of Artificial Sequence: Synthetic oligonucleotide

<400> 32
cctgtttctt gaatgaacca tatct        25

<210> 33
<211> 25
<212> DNA
<213> Artificial Sequence

<220>
<223> Description of Artificial Sequence: Synthetic oligonucleotide

<400> 33
agcatgacaa ggcctgcgtc cgaac        25

<210> 34
<211> 25
<212> DNA
<213> Artificial Sequence

<220>
<223> Description of Artificial Sequence: Synthetic oligonucleotide

<400> 34
agcggcgtcg caccctcccc actca        25

<210> 35
<211> 25
<212> DNA
<213> Artificial Sequence

<220>
<223> Description of Artificial Sequence: Synthetic oligonucleotide

<400> 35
tcaacgtacc attcaccttc atttt        25

<210> 36
<211> 25
<212> DNA

<213> Artificial Sequence

<220>
<223> Description of Artificial Sequence: Synthetic oligonucleotide

<400> 36
ttagttgacc ttgctgcagt tctcc          25

<210> 37
<211> 25
<212> DNA
<213> Artificial Sequence

<220>
<223> Description of Artificial Sequence: Synthetic oligonucleotide

<400> 37
aaagaataga gccatagttc aaaag          25

<210> 38
<211> 25
<212> DNA
<213> Artificial Sequence

<220>
<223> Description of Artificial Sequence: Synthetic oligonucleotide

<400> 38
cgggaatgtc agtggacccc aaaga          25

<210> 39
<211> 25
<212> DNA
<213> Artificial Sequence

<220>
<223> Description of Artificial Sequence: Synthetic oligonucleotide

<400> 39
tctgtgccca gagccccacg atgtc          25

<210> 40
<211> 25
<212> DNA
<213> Artificial Sequence

<220>
<223> Description of Artificial Sequence: Synthetic oligonucleotide

<400> 40
acttctttgt gggtgtgatc tccat          25

<210> 41
<211> 25
<212> DNA
<213> Artificial Sequence

<220>

<223> Description of Artificial Sequence: Synthetic oligonucleotide

<400> 41
aggagctgaa gacagggcct gtgag      25

<210> 42
<211> 25
<212> DNA
<213> Artificial Sequence

<220>
<223> Description of Artificial Sequence: Synthetic oligonucleotide

<400> 42
ttctgcaatc aaagtaattc ctact      25

<210> 43
<211> 25
<212> DNA
<213> Artificial Sequence

<220>
<223> Description of Artificial Sequence: Synthetic oligonucleotide

<400> 43
attgtcagaa gtcccacatt caata      25

<210> 44
<211> 25
<212> DNA
<213> Artificial Sequence

<220>
<223> Description of Artificial Sequence: Synthetic oligonucleotide

<400> 44
aaggaatgga tatttgcaga tagaa      25

<210> 45
<211> 25
<212> DNA
<213> Artificial Sequence

<220>
<223> Description of Artificial Sequence: Synthetic oligonucleotide

<400> 45
cgcggcgagg aacaagagtg tggtg      25

<210> 46
<211> 25
<212> DNA
<213> Artificial Sequence

<220>
<223> Description of Artificial Sequence: Synthetic oligonucleotide

<400> 46

tctaaagagg cagacaggca gtacg          25

<210> 47
<211> 25
<212> DNA
<213> Artificial Sequence

<220>
<223> Description of Artificial Sequence: Synthetic oligonucleotide

<400> 47
gaatgaggaa gtgaaggtga gtgtg          25

<210> 48
<211> 25
<212> DNA
<213> Artificial Sequence

<220>
<223> Description of Artificial Sequence: Synthetic oligonucleotide

<400> 48
caagttcagc ctagggactc cacgt          25

<210> 49
<211> 25
<212> DNA
<213> Artificial Sequence

<220>
<223> Description of Artificial Sequence: Synthetic oligonucleotide

<400> 49
aaatctcgct gaccataacc cttct          25

<210> 50
<211> 25
<212> DNA
<213> Artificial Sequence

<220>
<223> Description of Artificial Sequence: Synthetic oligonucleotide

<400> 50
ccgtgtggga gaccggcctg ccagg          25

<210> 51
<211> 25
<212> DNA
<213> Artificial Sequence

<220>
<223> Description of Artificial Sequence: Synthetic oligonucleotide

<400> 51
aagaccccgg cacagcatat atagc          25

<210> 52

<211> 25
<212> DNA
<213> Artificial Sequence

<220>
<223> Description of Artificial Sequence: Synthetic oligonucleotide

<400> 52
cccgcacctg gtcccggcca gcctg         25

<210> 53
<211> 25
<212> DNA
<213> Artificial Sequence

<220>
<223> Description of Artificial Sequence: Synthetic oligonucleotide

<400> 53
tcaaatcgca tccagatttt cgggt         25

<210> 54
<211> 25
<212> DNA
<213> Artificial Sequence

<220>
<223> Description of Artificial Sequence: Synthetic oligonucleotide

<400> 54
aaaaatctta aaaggcctct gcctt         25

<210> 55
<211> 25
<212> DNA
<213> Artificial Sequence

<220>
<223> Description of Artificial Sequence: Synthetic oligonucleotide

<400> 55
catcaccaaa cgaatggctg cagtc         25

<210> 56
<211> 25
<212> DNA
<213> Artificial Sequence

<220>
<223> Description of Artificial Sequence: Synthetic oligonucleotide

<400> 56
cttccccagg agggcactgc cagct         25

<210> 57
<211> 25
<212> DNA
<213> Artificial Sequence

<220>

<223> Description of Artificial Sequence: Synthetic oligonucleotide

<400> 57

ggggctcatc ctggaggagg tgtga          25

<210> 58
<211> 25
<212> DNA
<213> Artificial Sequence

<220>

<223> Description of Artificial Sequence: Synthetic oligonucleotide

<400> 58

cagtggaagc caggtgcctt taatc          25

<210> 59
<211> 25
<212> DNA
<213> Artificial Sequence

<220>

<223> Description of Artificial Sequence: Synthetic oligonucleotide

<400> 59

tgagggcaca ggcacctcaa gacct          25

<210> 60
<211> 25
<212> DNA
<213> Artificial Sequence

<220>

<223> Description of Artificial Sequence: Synthetic oligonucleotide

<400> 60

accacctttt gcagggtgga tctat          25

<210> 61
<211> 25
<212> DNA
<213> Artificial Sequence

<220>

<223> Description of Artificial Sequence: Synthetic oligonucleotide

<400> 61

cagcctcagg acagatacaa agctg          25

<210> 62
<211> 25
<212> DNA
<213> Artificial Sequence

<220>

<223> Description of Artificial Sequence: Synthetic oligonucleotide

<400> 62
gctggccccg cgttcttggt tcagc        25


<210> 63
<211> 25
<212> DNA
<213> Artificial Sequence


<220>
<223> Description of Artificial Sequence: Synthetic oligonucleotide


<400> 63
caacctgcag aaggtcctgg agagt        25


<210> 64
<211> 25
<212> DNA
<213> Artificial Sequence


<220>
<223> Description of Artificial Sequence: Synthetic oligonucleotide


<400> 64
tcaagtagtg gtgactgatc tgtct        25


<210> 65
<211> 25
<212> DNA
<213> Artificial Sequence


<220>
<223> Description of Artificial Sequence: Synthetic oligonucleotide


<400> 65
ggcagcctgt cacagggacc ccaga        25


<210> 66
<211> 25
<212> DNA
<213> Artificial Sequence


<220>
<223> Description of Artificial Sequence: Synthetic oligonucleotide


<400> 66
tgacatgtgt gtgtggagtc ggttg        25


<210> 67
<211> 25
<212> DNA
<213> Artificial Sequence


<220>
<223> Description of Artificial Sequence: Synthetic oligonucleotide


<400> 67
agctctggga gttgtggaaa ggtgc        25